(19) Europäisches Patentamt
European Patent Office
Office européen des brevets

(11) Publication number: **0 434 365 A2**

(12) **EUROPEAN PATENT APPLICATION**

(21) Application number: **90313848.5**

(22) Date of filing: **18.12.90**

(51) Int. Cl.$^5$: **C07D 295/088**, A61K 31/33, C07C 237/22, A61K 31/16, C07C 271/22, C07D 521/00

(30) Priority: **18.12.89 US 452912**
**15.10.90 US 597286**
**04.12.90 US 619654**

(43) Date of publication of application: **26.06.91 Bulletin 91/26**

(84) Designated Contracting States: **AT BE CH DE DK ES FR GB GR IT LI LU NL SE**

(71) Applicant: **MERCK & CO. INC.**
**126, East Lincoln Avenue P.O. Box 2000**
**Rahway New Jersey 07065-0900 (US)**

(72) Inventor: **Thompson, Wayne J.**
**2291 Locust Drive**
**Lansdale, PA 19446 (US)**
Inventor: **Vacca, Joseph P.**
**766 Eisenhauer Drive**
**Telford, PA 18969 (US)**
Inventor: **Huff, Joel R.**
**825 Surrey Drive**
**Gwynedd Valley, PA 19437 (US)**
Inventor: **Lyle, Terry A.**
**570 Camp WaWa Road**
**Lederach, PA 19450 (US)**
Inventor: **Young, Steven D.**
**1280 Mark Drive**
**Lansdale, PA 19446 (US)**
Inventor: **Hungate, Randall W.**
**1925 Rampart Lane**
**Lansdale, PA 19446 (US)**
Inventor: **Britcher, Susan F.**
**735 Port Indian Road**
**Norristown, PA 19403 (US)**
Inventor: **Ghosh, Arun K.**
**853 Jackson Street**
**Lansdale, PA 19446 (US)**

(74) Representative: **Hesketh, Alan, Dr. et al**
**European Patent Department Merck & Co., Inc. Terlings Park Eastwick Road**
**Harlow Essex, CM20 2QR (GB)**

(54) **HIV protease inhibitors useful for the treatment of aids.**

(57) Compounds of the form,
A-G-B-B-J
wherein A is an amine protecting group or urethane, G a dipeptide isostere substituted with a basic amine nitrogen, B an amino acid or analog thereof, and J a small terminal group are described. These compounds are useful in the inhibition of HIV protease, the prevention or treatment of infection by HIV and the treatment of AIDS, either as compounds, pharmaceutically acceptable salts, pharmaceutical composition ingredients, whether or not in combination with other antivirals, immunomodulators, antibiotics or vaccines. Methods of treating AIDS and methods of preventing or treating infection by HIV are also described.

EP 0 434 365 A2

# HIV PROTEASE INHIBITORS USEFUL FOR THE TREATMENT OF AIDS

The present invention is concerned with compounds which inhibit the protease encoded by human immunodeficiency virus (HIV) or pharmaceutically acceptable salts thereof and are of value in the prevention of infection by HIV, the treatment of infection by HIV and the treatment of the resulting acquired immune deficiency syndrome (AIDS). It also relates to pharmaceutical compositions containing the compounds and to a method of use of the present compounds and other agents for the treatment of AIDS & viral infection by HIV.

## BACKGROUND OF THE INVENTION

A retrovirus designated human immunodeficiency virus (HIV) is the etiological agent of the complex disease that includes progressive destruction of the immune system (acquired immune deficiency syndrome ; AIDS) and degeneration of the central and peripheral nervous system. This virus was previously known as LAV, HTLV-III, or ARV. A common feature of retrovirus replication is the extensive post-translational processing of precursor polyproteins by a virally encoded protease to generate mature viral proteins required for virus assembly and function. Interruption of this processing appears to prevent the production of normally infectious virus. For example, Crawford, S. et al., J. Virol., 53, 899, 1985, demonstrated that genetic deletion mutations of the protease in murine leukemia virus which prevent processing of precursor structural proteins results in non-infectious viral particles. Unprocessed structural proteins also have been observed in clones of non-infectious HIV strains isolated from human patients. These results suggest that inhibition of the HIV protease represents a viable method for the treatment of AIDS and the prevention or treatment of infection by HIV.

Nucleotide sequencing of HIV shows the presence of a pol gene in one open reading frame [Ratner, L. et al., Nature, 313, 277(1985)]. Amino acid sequence homology provides evidence that the pol sequence encodes reverse transcriptase, an endonuclease and an HIV protease [Toh, H. et al., EMBO J. 4, 1267 (1985) ; Power, M.D. et al., Science, 231, 1567 (1986) ; Pearl, L.H. et al., Nature 329, 351 (1987)]. Applicants demonstrate that the compounds of this invention are inhibitors of HIV protease.

## BRIEF DESCRIPTION OF THE INVENTION

Compounds of formula I, as herein defined, are disclosed. These compounds are useful in the inhibition of HIV protease, the prevention of infection by HIV, the treatment of infection by HIV and in the treatment of AIDS, either as compounds, pharmaceutically acceptable salts, pharmaceutical composition ingredients, whether or not in combination with other antivirals, immunomodulators, antibiotics or vaccines. Methods of treating AIDS, methods of preventing infection by HIV, and methods of treating infection by HIV are also disclosed.

## ABBREVIATIONS

| Designation | Amino Acid/Residue |
|---|---|
| Ala | D- or L-alanine |
| Allo-Ile | allo-isoleucine |
| Arg | D- of L-arginine |
| Cal (Cha) | β-cyclohexylalanine |
| Cys | D- or L-cysteine |
| Gly | glycine |
| His | D- or L-histidine |
| Ile | L-isoleucine |
| Leu | D- or L-leucine |
| Lys | D- or L-lysine |
| Met | D- or L-methionine |
| Nle | L-norleucine |
| Nva | L-norvaline |
| Orn | D- of L-ornithine |
| Ph | phenyl |
| Phe | D- or L-phenylalanine |
| Pro | D- or L-proline |
| Sar | sarcosine (N-methylglycine) |
| Ser | D- or L-serine |

| | |
|---|---|
| Sta | statine, (3S,4S)-4-amino-3-hydroxy-6-methylheptanoic acid |
| Thr | D- or L-threonine |
| Trp | D- or L-tryptophan |
| Tyr | D- or L-tyrosine |
| Val | L-valine |
| | Protecting Group |
| BOC (Boc) | t-butyloxycarbonyl |
| BOM | benzyloxymethyl |
| CBZ (Cbz) | benzyloxycarbonyl(carbobenzoxy) |
| DNP | 2,4-dinitrophenyl |
| INOC | isonicotinoyloxycarbonyl |
| IPOC | isopropoxycarbonyl |
| OMe | methyl ether (methoxy), except when it immediately follows an amino acid residue abbreviation and it represents methyl ester. |
| OEt | ethoxy, except when it immediately follows an amino acid residue abbreviation and it represents ethyl ester |
| Designation | Activating Group |
| HBT(HOBT or HOBt) | 1-hydroxybenzotriazole hydrate |
| OMs | methane sulfonyloxy |
| | Condensing Agent |
| DCCI (DCC) | dicyclohexylcarbodiimide |
| DPPA | diphenylphosphorylazide |
| | Reagent |
| $(BOC)_2O$ | di-t-butyl dicarbonate |
| $BF_3 \cdot OET_2$ | boron trifluoride etherate |
| DEAD | diethyl azodicarboxylate |
| $nBu_4NF$ | tetrabutyl ammonium fluoride |
| TEA | triethylamine |
| TFA | trifluoroacetic acid |
| | Coupling Reagents |
| BOP reagent | benzotriazol-1-yloxytris-(dimethyl-amino)phosphonium hexafluorophosphate |
| BOP-C1 | bis(2-oxo-3-oxazolidinyl) phosphinic chloride |
| DSO | N,N'-disuccinimidyl oxalate |
| EDC | 1-ethyl-3-(3-dimethyl-aminopropyl) carbodiimide hydrochloride |
| MCPBA | m-Chloroperbenzoic acid |
| TBDMS | T-Butyl-dimethylsilyl |

## DETAILED DESCRIPTION OF THE INVENTION AND PREFERRED EMBODIMENTS

This invention is concerned with the use of compounds of formula I, combinations thereof, or pharmaceutically acceptable salts thereof, in the inhibition of HIV protease, the prevention or treatment of infection by HIV and in the treatment of the resulting acquired immune deficiency syndrome (AIDS). Compounds of formula I are defined as follows :

A-G-B-B-J I.

wherein A is :

1) hydrogen ;

2) $R^1-\overset{O}{\overset{\|}{C}}-$ wherein $R^1$ is hydrogen, or

a) $C_{1-6}$alkyl or $C_{1-6}$alkenyl, either unsubstituted or substituted with one or more of

i) $C_{1-4}$alkyl ;

ii) hydroxy ;

iii) carboxy ;

iv) halo wherein halo is F, Cl, Br, or I :

v) amino ;

vi) $C_{1-3}$alkoxycarbonyl ;

vii) $C_{1-3}$alkoxy unsubstituted or substituted with tetrahydropyran ;

viii) —CONR$^2$R$^3$ wherein R$^2$ and R$^3$ are the same or different and are hydrogen, $C_{1-5}$alkyl or $C_{1-5}$alkoxyalkyl or joined together either directly to form a 5-7 membered heterocycle such as pyrrolidinyl or piperidyl, or through a heteroatom selected from N, O, and S, to form a 6-membered heterocycle with the nitrogen to which they are attached such as morpholinyl, piperazinyl, or N-$C_{1-3}$ alkylpiperazinyl ;

ix) —NR$^2$R$^3$ ;

x) 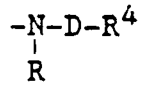 wherein,

R is hydrogen or $C_{1-4}$alkyl,

D is 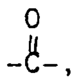, 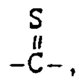, 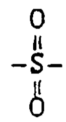, 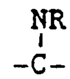, and

R$^4$ is N, $C_{1-3}$alkyl, $C_{1-4}$alkoxy, or NR$^2$R$^3$ ;

xi) $C_{3-7}$cycloalkyl or $C_{6-10}$aryl ;

xii) 5 or 6 membered heterocycle, saturated or unsaturated, such as furyl, morpholino, or a 7-10 membered bicyclic hetercyclic ring, saturated or unsaturated, the ring or rings being unsubstituted or substituted with

- i) oxo,
- ii) —OH,
- iii) NH, or
- iv) $C_{1-4}$alkyl ; or

xiii) aryl or carbocyclic of 6-10 carbon atoms, either unsubstituted or substituted with one or more of

- (a) halo,
- (b) hydroxy,
- (c) $C_{1-3}$alkoxy,
- (d) $C_{1-3}$alkyl,
- (e) —NR$_2$, wherein R is defined above,
- (f) 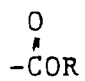,
- (g) 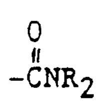,
- (h) —SO$_2$NR$_2$,
- (i) —CH$_2$NR$_2$,
- (j) 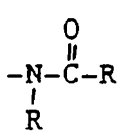, or

(k) 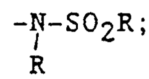

xiv) —O-[$(CH_2)_mO]_n$-R, where m is 2-5 and n is 0-5 ;

b) aryl of 6-10 carbon atoms, either unsubstituted or substituted with one or more of

i) $C_{1-4}$alkyl,

ii) $C_{1-3}$alkoxy,

iii) hydroxy, or

iv) halo ;

v) —$NR_2$,

vi) 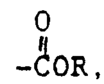

vii) —$CNR_2$,

viii) —$SO_2NR_2$,

ix) —$CH_2NR_2$,

x) —NRCOR, or

xi) —$NRSO_2R$ ;

c) 5 or 6 membered heterocycle ;

3) $R^1$-$SO_2$—,

4) 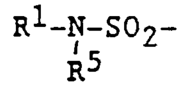 wherein

$R^5$ is H or $C_{1-5}$alkyl or joined together with $R^1$ either directly to form 5-7 membered heterocycle such as pyrrolidinyl or piperidinyl, or through a heteroatom selected from N, O, and S, to form a 6-membered heterocycle with the nitrogen to which they are attached such as morpholinyl, piperazinyl, or N-$C_{1-3}$alkyl-piperazinyl ;

5) 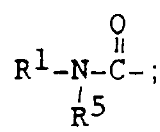

6) 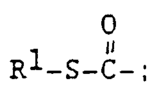

7) 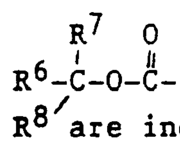 wherein $R^6$, $R^7$, and $R^8$ are independently

a) H,

b) $C_{1-6}$alkyl unsubstituted or substituted with one or more of

i) halo,
ii) OH
iii) aryl $SO_2$—
iv) —$O-[(CH_2)_mO]_n-R$,

c) Aryl unsubstituted or substituted with one or more of
i) $C_{1-4}$alkyl,
ii) $C_{1-3}$alkoxy,
iii) halo,
iv) nitro,
v) acetoxy,
vi) dimethylaminocarbonyl,
vii) phenyl,
viii) $C_{1-3}$alkoxycarbonyl ;

d) fluorenyl,

e) $R^6$, $R^7$, and $R^8$ may be independently joined to form a monocyclic, bicyclic, or tricyclic ring system which is $C_{3-10}$ cycloalkyl e.g. adamantyl, cyclopentyl, or cyclohexyl, and may be substituted with $C_{1-4}$ alkyl,

f) a 5-7 membered heterocyclic ring, saturated or unsaturated, such as pyridyl, furyl, 1,3-dioxolane, or tetrahydrofuryl ; or a 7-10 membered bicyclic heterocyclic ring, saturated or unsaturated, such as benzisoxazolyl, 4H-1,3-benzodioxin, the ring or rings being unsubstituted or substituted with $C_{1-4}$alkyl ;

g) $R^6$, $R^7$ and $R^8$ may be independently joined to form a 5-7 membered heterocyclic ring, or 7-10 membered bicyclic heterocyclic ring, saturated or unsaturated, unsubstituted or substituted with one or more of
i) acetyloxy,
i) $C_{1-4}$alkyl,
iii) hydroxy,
iv) hydroxy $C_{1-4}$alkyl ;
v) alkyloxy alkyl,
vi) phenyl alkoxy, or
vii) alkoxy ;

8) 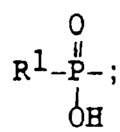

G is

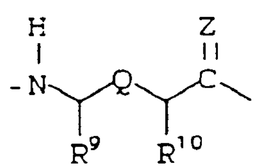 or 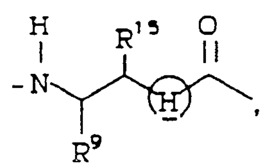,

wherein Z is O, S, or HH and
$R^9$ and $R^{10}$ are independently,
a) —$(CH_2)_n-R^{11}$,
b) $C_{1-4}$alkenyl $-R^{11}$ ;
wherein n is 0-5 ; and either or both $R^9$ and $R^{10}$ must be substituted with $R^{12}$ ;

$R^{11}$ is
a) hydrogen ;
b) $C_{1-4}$alkyl ;
c) $C_5$-$C_{10}$cycloalkyl, optionally substituted with hydroxy ;

d) $C_6$-$C_{10}$aryl, unsubstituted or substituted with $R^{12}$, and optionally substituted with one or more of

i) halo,

ii) $C_{1-4}$alkyl,

iii) $C_{1-3}$alkoxy

iv) hydroxy ;

e) monocyclic or bicyclic heterocyle containing from 1 to 3 heteroatoms chosen from N, O, S and which is unsubstituted or substituted with $R^{12}$ and optionally with one or more of

i) halo,

ii) $C_{1-4}$alkyl,

iii) $C_{1-3}$alkoxy ;

$R^{12}$ is

a) —X-$(CH_2)_m$–$NR^{13}R^{14}$ where X is —O—, —S—, or —NH—, m is 2-5, and $R^{13}$ and $R^{14}$ are independently hydrogen or

i) $C_{1-6}$alkyl,

ii) $C_{1-6}$alkyl substituted with one or more of

(a) $C_{1-3}$alkoxy,

(b) —OH,

(c) —$NR_2$ wherein R is hydrogen $C_{1-4}$alkyl ;

iii) the same or different and joined together to form a 5-7 member heterocycle, such as morpholino, containing up to two additional heteroatoms selected from -NR,

$$-O-,\ -S-,\ -\overset{O}{\overset{\|}{S}}-,\ \text{or}\ -SO_2-,\ \text{said}$$

heterocycle optionally substituted with $C_{1-4}$alkyl ;

iv) aromatic heterocycle unsubstituted or substituted with one or more of

(a) $C_{1-4}$alkyl,

(b) —$NR_2$,

b) —$(CH_2)_q$–$NR^{13}R^{14}$ wherein q is 1-5, and $R^{13}$ and $R^{14}$ are defined above, except that $R^{13}$ or $R^{14}$ are not H or $C_{1-6}$alkyl ;

c) benzofuryl, indolyl, azacycloalkyl, azabicyclo $C_{7-11}$cycloalkyl, or benzopiperidinyl, substituted or unsubstituted with $C_{1-4}$alkyl ;

$R^{15}$ is -OH or -$NHR^{16}$, wherein $R^{16}$ is -H, $-\overset{O}{\overset{\|}{C}}H$, -$C_{1-4}$-alkyl, or -COOR; and

Ⓗ is

1) $C_{3-7}$cycloalkyl either unsubstituted or substituted with one or more of

a) $C_{1-4}$alkyl,

b) hydroxy,

c) —$NR_2$,

d) —COOR,

e) —CONHR,

f) —$NHSO_2R$,

g) $-NH\overset{O}{\overset{\|}{C}}R$,

h) aryl,

i) aryl substituted with $C_{1-4}$alkyl,

j) heterocycle, or

k) heterocycle substituted with $C_{1-4}$alkyl ;

Q is

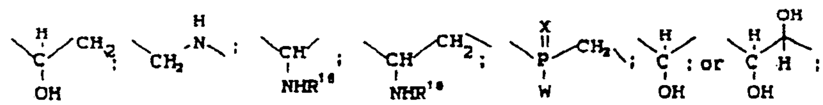

wherein $R^{16}$ is defined above ;

X is O, S or NH ; and

W is

1) OH,
2) $NH_2$,
3) OR, or
4) NHR ;

B is, independently, absent, or 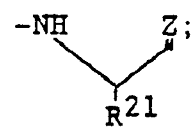

where $R^{21}$ is

a) $—CH(CH_3)_2$
b) $—CH(CH_3)(CH_2CH_3)$
c) —Phenyl ;

J is

1) $YR^{17}$ wherein :

Y is O or NH, and

$R^{17}$ is

a) H ;
b) $C_{1-6}$alkyl, unsubstituted or substituted with one or more of
   i) $—NR_2$,
   ii) —OR,
   iii) $—NHSO_2C_{1-4}$alkyl,
   iv) $—NHSO_2$aryl, or $—NHSO_2$(dialkylaminoaryl),
   v) $—CH_2OR$,
   vi) $—C_{1-4}$alkyl,
   vii) 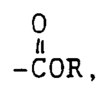,
   viii) 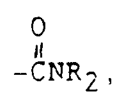,
   ix) 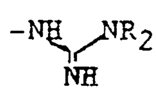 or 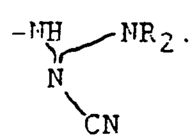.
   x) 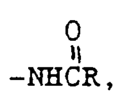,

xi) 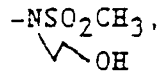

xii) 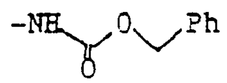

xiii) $-NR_3^{\oplus} A^{\ominus}$ wherein $A^{\ominus}$ is a counterion,

xiv) $-NR^{18}R^{19}$ wherein $R^{18}$ and $R^{19}$ are the same or different and are $C_{1-5}$ alkyl joined together directly to form a 5-7 membered heterocycle containing up to one additional heteroatom selected from —O—, —S—, or —NR—,

xv) aryl,

xvi) —CHO,

xvii) $-OP(O)(OR_x)_2$ wherein $R_x$ is H or aryl, or

xvii) 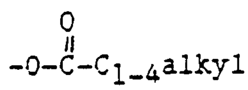 substituted with one or more of amine or quaternary amine, or $-O-[(CH_2)_mO]_n-R$, or $-OP(O)(OR_x)_2$ ;

c) $-[(CH_2)_mO]_nCH_3$ or $-[(CH_2)_mO]_n H$ ;

2) $N(R^{17})_2$ ; or

3) $-NR^{18}R^{19}$ wherein $R^{18}$ and $R^{19}$ are defined above ;

4) 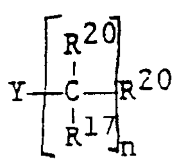

wherein :

Y, $R^{17}$ and n are defined above, and

$R^{20}$ is

a) hydrogen ;

b) aryl unsubstituted or substituted with one or more of

i) halo,

ii) —OR, wherein R is H or $C_{1-4}$alkyl,

iii) 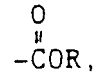

iv) 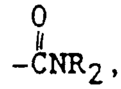

v) $-CH_2NR_2$,

vi) $-SO_2NR_2$,

vii) $-NR_2$,

viii) $-NH\overset{O}{\overset{\|}{C}}R$,

xi) $C_{1-4}$alkyl,
x) phenyl
xi) $—CF_3$,

xii) $-\overset{R}{\overset{|}{N}}-SO_2R$,

xiii) —$C_{1-4}$alkyl —$NR_2$,
xiv) —$OP(O)(OR_x)_2$ wherein $R_x$ is H or aryl, or

xv) $-O-\overset{O}{\overset{\|}{C}}-C_{1-4}$alkyl substituted with

one or more of amine or quaternary amine or —$OP(O)(OR_x)_2$ ;

c) A 5-7 membered heterocyclic ring or, 7-10 membered bicyclic heterocyclic ring which is saturated or unsaturated, such as isochroman, chroman, isothiochroman, thiochroman, benzimidazole, benzothiopyran, oxobenzothiopyran, benzopyran, benzothiopyranylsulfone, benzothiopyranylsulfoxide, the ring or rings being unsubstituted or substituted with one or more of

i) halo,
ii) —OR, wherein R is H, $C_{1-4}$alkyl, or $C_{1-4}$alkenyl,

iii) $-\overset{O}{\overset{\|}{C}}OR$,

iv) $-\overset{O}{\overset{\|}{C}}NR_2$,

v) —$CH_2NR_2$,
vi) —$SO_2NR_2$,
vii) —$NR_2$,

viii) $-NH\overset{O}{\overset{\|}{C}}R$,

xi) $C_{1-4}$alkyl,
x) phenyl
xi) —$CF_3$,

xii) $-\overset{R}{\overset{|}{N}}-SO_2R$,

xiii) phenyl $C_{1-4}$alkyl,
xiv) —$OP(O)(OR_x)_2$ wherein $R_x$ is H or aryl,

xv) $-O-\overset{O}{\overset{\|}{C}}-C_{1-4}$alkyl substituted with

one or more of amine or quaternary amine, or —$OP(O)(OR_x)_2$, or —$O[(CH_2)_mO]_n$—R, or

xvi) $-O-\overset{O}{\overset{\|}{C}}-O-[(CH_2)_mO]_n-R$;

d) A 5 to 7 membered carbocyclic or 7-10 membered bicyclic carbocyclic ring which is either saturated or unsaturated, such as cyclopentane, cyclohexane, indane, norbornane, or naphthalene, the carbocyclic ring being unsubstituted or substituted with one or more of

i) halo,

ii) —OR, or —$CH_2OR$, wherein R is H or $C_{1-4}$alkyl,

iii) $-\overset{O}{\overset{\|}{C}}OR^{21}$, wherein $R^{21}$ is H,

—$(CH_2)_n$-$NR_2$, $C_{1-16}$alkyl, pyridine,

$-(CH_2)_nNR-(CH_2)_n-NR_2$, $-(CH_2)_n-\overset{O}{\overset{\|}{C}}-OR$,

—$[(CH_2)_mO]_n$-R, quinuclidiniumyl substituted with R, piperazine— $C_{1-4}$alkyl-benzyl substituted once or more with R, or morpholinoalkyl- benzyl,

iv) $-\underset{O}{\underset{\|}{C}}NR_2$,

v) —$CH_2NR_2$,

vi) —$SO_2NR_2$,

vii) —$NR_2$,

viii) $-NH\overset{O}{\overset{\|}{C}}R$,

xi) $C_{1-4}$alkyl,

x) phenyl,

xi) —$CF_3$,

xii) $-\overset{R}{N}-SO_2R$,

xiii) —$OP(O)(OR_x)_2$ wherein $R_x$ is H or aryl,

xiv) $-O-\overset{O}{\overset{\|}{C}}-C_{1-4}$alkyl substituted with

one or more of amine or quaternary amine, —$OP(O)(OR_x)_2$, or —$O$-$[(CH_2)_mO]_n$-R, or

xv) $-O-C(=O)-O-[(CH_2)_mO]_n-R$;

or pharmaceutically acceptable salts thereof.

In a preferred embodiment of this invention,

A is $R^6-C(R^7)(R^8)-O-C(=O)-$; and G is 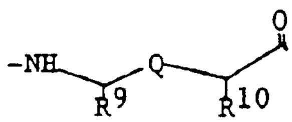 .

In a more preferred embodiment of this invention,

B is absent or present once and Q is

$$-CH(OH)-CH_2-$$

It is most preferred that B is absent and J is NH-$R^{20}$ wherein $R^{20}$ is a substituted 7 to 10 membered bicyclic carbocyclic or heterocyclic ring which is either saturated or unsaturated.

Novel compounds of the present invention include, but are not limited to, the following :

N-(2-(R)-hydroxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,

N-(2-(R)-hydroxy-1(S)-indanyl)-5(S)-((2-tetrahydrofuranyl)carbonylamino)-4(S)-hydroxy-6-phenyl-2(R)-((4-(2-(4-morpholinyl)ethoxy)phenyl)-methyl)hexanamide,

N-(2-(R)-hydroxy-1(S)-indanyl)-5(S)-(2-hydroxyethoxycarbonylamino)-4(S)-hydroxy-6-phenyl-2(R)-((4-(2-(4-morpholinyl)ethoxy)phenyl)methyl)hexanamide,

N-(2-(R)-hydroxy-1(S)-indanyl)-5(S)-(1-methylethoxycarbonylamino)-4(S)-hydroxy-6-phenyl-2(R)-((4-(2-(4-morpholinyl)ethoxy)phenyl)methyl)hexanamide,

N-(2-(R)-hydroxy-1(S)-indanyl)-5(S)-(ethoxycarbonylamino)-4(S)-hydroxy-6-phenyl-2(R)-((4-(2-(4-morpholinyl)ethoxy)phenyl)methyl)hexanamide.

N-(2-(R)-hydroxy-1(S)-indanyl)-5(S)-(1,1-dimethylethoxycarbonylamino)-4(S)-hydroxy-6-(4-(2-(4-morpholinyl)ethoxy)phenyl)-2(R)-(3′-phenylprop-2-′en-1-yl)-hexanamide,

N-(2-benzamidolylmethyl)-N-5(S)-(1,1-dimethylethoxycarbonylamino)-4(S)-hydroxy-6-(4-(2-(4-morpholinyl)ethoxy)phenyl)-2(R)-(3′-phenylprop-2-′en-1-yl)-hexanoyl isoleucyl amide,

N-(2-benzamidolylmethyl)-N′-5(S)-(1,1-dimethylethoxycarbonylamino)-4(S)-hydroxy-6-phenyl-2(R)-(4-(2-(4-morpholinyl)ethoxy)phenyl)-hexanoyl isoleucyl amide,

N-((pyrrolo[3,2-b]pyridin-2-yl)methyl)-N′-5(S)-(1,1-dimethylethoxycarbonylamino)-4(S)-hydroxy-6-phenyl-2(R)-(4-(2-(4-morpholinyl)ethoxy)phenyl)-hexanoyl isoleucyl amide,

N-((5-(2-morpholinylethyl)pyrrolo[3,2-b]pyridin-2-yl)methyl)-N′-5(S)-(1,1-dimethylethoxycarbonylamino)-4(S)-hydroxy-6-phenyl-2(R)-(4-(2-(4-morpholinyl) ethoxy)phenyl)-hexanoyl isoleucyl amide,

N-(2(R)-hydroxy-1(S)-indanyl)-5(S)-(1,1-dimethylethoxycarbonylamino)-4(S)-hydroxy-6-phenyl-2(R)-((3-(2-(4-morpholinyl)ethoxy)phenyl)methyl)hexanamide,

N-(2(R)-hydroxy-1(S)-indanyl)-5(S)-(1,1-dimethylethoxycarbonylamino)-4(S)-hydroxy-6-(4-(2-(4-morpholinyl)ethoxy)phenyl)-2(R)-phenylmethylhexanamide,

N-(2(R)-hydroxy-1(S)-indanyl)-5(S)-(1,1-dimethylethoxycarbonylamino)-4(S)-hydroxy-6-(4-(2-(4-morpholino)ethoxy)phenyl)-2(R)-((4-(2-(4-morpholinyl)ethoxy)phenyl)methyl)hexanamide,

N-(2(S)-hydroxy-1(S)-benzopyranyl)-5(S)-[1,1-dimethylethoxycarbonylamino)-4(S)-hydroxy-6-(4-(2-(4-morpholino)ethoxy)phenyl)-2(R)-((4-(2-(4-morpholinyl)ethoxy)phenyl)methyl)hexanamide,

N-(2(S)-hydroxy-1(S)-benzopyranyl)-5(S)-[1,1-dimethylethoxycarbonylamino)-4(S)-hydroxy-6-(4-(2-(4-morpholinyl)ethoxy)phenyl)-2(R)-((4-hydroxyphenyl)-methyl)hexanamide,

N-(2(S),3-(R)-dihydroxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,

N-(2(S),3(R)-dihydroxy-1(S)-indanyl)-5(S)-(1,1-dimethylethoxycarbonylamino)-4(S)-hydroxy-6-phenyl-2(R)-((3-(2-(4-morpholinyl)ethoxy)phenyl)methyl)hexanamide,

N-(2(R)-hydroxy-1(S)-indanyl)-5(S)-(1,1-dimethylethoxycarbonylamino)-4(S)-hydroxy-6-(4-(2-(4-morpholinyl)ethoxy)phenyl)-2(R)-((4-(2-(4-morpholinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(4-morpholinyl)propyloxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-dimethylaminoethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(1-piperidinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(1-pyrrolidinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(N,N-bis(2-methoxyethyl)amino)ethoxy)phenyl)-methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(thiamorpholin-4-yl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-(4-(2-(4-(1-oxothiamorpholin-4-yl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-(4-(2-(4-(1,1-dioxythiamorpholin-4-yl)ethoxy)-phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-(4-(2-(1-(4-methylpiperazin-1-yl)ethoxy)phenyl)methyl)hexanamide,
N-(3(S)-hydroxy-4(S)-benzopyranyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(S)-hydroxy-1(S)-benzothiopyranyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(R)-dimethylaminoacetoxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(R)-acetoxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(S),3(R)-diacetoxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(4)-(2-methoxyethylamino)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-(3-(3-(2-(4-morpholinyl)ethoxy)phenyl)prop-2-en-1-yl)hexanamide,
N-(2(R)-hydroxy-1(S)-indanyl-5(S)-(1,1-dimethylethoxycarbonylamino)-4(S)-hydroxy-6-phenyl-2(R)-(3-(4-(2-(4-morpholinyl)ethoxy)phenyl)prop-2-en-1-yl)hexanamide,
N-(5-(2-(4-morpholinyl)ethoxy)-2-benzimidazolylmethyl))-5(S)-(1,1-dimethylethoxycarbonylamino)-4(S)-hydroxy-6-phenyl-2(R)-((4-(2-(4-morpholinyl)ethoxy)-phenyl)methyl)hexanoyl isoleucylamide,
N-((5-(2-(4-morpholinyl)ethoxy)benzimidazolylmethyl))-5(S)-(1,1-dimethylethoxycarbonylamino)-4(S)-hydroxy-6-phenyl-2(R)-((4-(2-(4-morpholinyl)ethoxy)phenyl)-methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(2-hydroxyethylamino)ethoxy)phenyl)-methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(2-methylpropylamino)ethoxy)phenyl)methyl)hexanamide,
N-(3(R)-amino-2(R)-hydroxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(R)-((3-pyridinecarbonyloxy)-1(S)-indanyl))-5(S)-(1,1-dimethylethoxycarbonylamino)-4(S)-hydroxy-6-phenyl-2(R)-((4-(2-(4-morpholinyl)ethoxy)phenyl)-methyl)hexanamide,
N-(2(R)-n-hexadecanoyloxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2-(R)-hydroxy-1(S)-indanyl)-5(S)-(1,1-dimethylethoxycarbonylamino)-4(S)-hydroxy-6-phenyl-2(R)-((4-(2-(3,5-dimethylmorpholin-4-yl)ethoxy)phenyl)-methyl)-hexanamide,
N-(2-(R)-hydroxy-1(S)-indanyl)-5(S)-(1,1-dimethylethoxycarbonylamino)-4(S)-hydroxy-6-phenyl-2(R)-((4-(2-(2,6-dimethylmorpholin-4-yl)ethoxy)phenyl)methyl)hexanamide,
N-(2-(R)-hydroxy-1(S)-indanyl)-5(S)-(1,1-dimethylethoxycarbonylamino)-4(S)-hydroxy-6-phenyl-2(R)-((4-(2-(2,2,6,6-tetramethylpiperidin-4-yl)-amino-ethoxy)phenyl)methyl)hexanamide,
N-(2-(R)-hydroxy-1(S)-indanyl)-5(S)-(1,1-dimethylethoxycarbonylamino)-4(S)-hydroxy-6-phenyl-2(R)-((4-(3-(4-morpholinyl)propyl)phenyl)methyl)hexanamide,
N-(2-(R)-hydroxy-1(S)-indanyl)-5(S)-(1,1-dimethylethoxycarbonylamino)-4(S)-hydroxy-6-phenyl-2(R)-(4-(4-morpholinyl)methylphenyl)methyl hexanamide,
N-(2-(R)-hydroxy-1(S)-indanyl)-5(S)-(1,1-dimethylethoxycarbonylamino)-4(S)-hydroxy-6-phenyl-2(R)-((4-(3-(bis-(2-methoxyethyl)amino)propyl)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(3-(1-thiamorpholin-4-yl)propyl)phenyl)-methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(1-imidazolyl)ethoxy)phenyl)methyl)hexanamide,
N-(3(R)-hydroxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-((1,2-dimethyl-2-hydroxyethyl)phosphoroamidato-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(4-morpholinyl)-2-oxoethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(3-dimethylaminopropyl)phenyl)methyl)hexanamide,
N-(2(R)-hydroxy-1(S)-indanyl)-5(S)-(1-adamantyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(3-oxomorpholin-4-yl)ethoxy)phenyl)methyl)hexanamide,
N-(2(R)-hydroxy-1(S)-indanyl)-5(S)-((1,1-dimethylethoxycarbonyl)-Ala)-amino-4(S)-hydroxy-6-phenyl-2(R)-((4-(2-(4-morpholinyl)ethoxy)phenyl)methyl)hexanamide,
N-(2-(R)-hydroxy-1(S)-indanyl)-5(S)-(Ala-amino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(4-pyridinylamino)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(1-triazolyl)ethoxy)phenyl)methyl)hexanamide,
N-(2-(R)-(8-((2-dimethylaminoethyl)ethylamino)-8-oxooctanoyloxy)-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2-(R)-hydroxy-1(S)-indanyl)-5(S)-(2,2-dimethyl-1-propyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1.1.2-trimethyl-1-propyloxycarbonylamino)-4(S)-hydroxy-6-phenyl-2(R)-((4-(2-(4-morpholinyl)ethoxy)phenyl)methyl)hexanamide,
N-(2-(R)-hydroxy-1(S)-indanyl)-5(S)-(1,1-dimethyl-1-propyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(cyclohexyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(cycloheptyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(cycloheptyloxycarbonylamino)-4(S)-hydroxy-6-phenyl-2(R)-((4-(2-(4-morpholinyl)ethoxy)phenyl)methyl)hexanamide,
N-(2-(R)-hydroxy-1(S)-indanyl)-5(S)-(1-methyl-1-propyloxycarbonylamino)-4(S)-hydroxy-6-phenyl-2(R)-((4-(2-(4-morpholinyl)ethoxy)phenyl)methyl)hexanamide,
N-(2-(R)-hydroxy-1(S)-indanyl)-5(S)-(1,1-dimethyl-2-hydroxyethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethyl-2-methoxyethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethyl-2-(2-methoxyethoxy)ethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethyl-2-(2-(2-methoxyethoxy)ethoxy)ethoxycarbonylamino)-4(S)-hydroxy-6-phenyl-2(R)-((4-(2-(4-morpholinyl)ethoxy)-phenyl)methyl)hexanamide,
N-(2(R)-hydroxy-1(S)-indanyl)-5(S)-(3-methyl-3-pentyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(3-ethyl-3-pentyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1-bicyclo[2,2,2]octyloxycarbonylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-(1-bicyclo[2,2,1]heptyloxycarbonylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-(methoxycarbonylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-((2-tetrahydrofuranyl)methoxycarbonylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-((3-tetrahydrofuranyl)methoxycarbonylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-(1-methyl-1-(2-tetrahydrofuranyl)ethoxycarbonylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-(1-methyl-1-(3-tetrahydrofuranyl)methoxycarbonylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-(3-tetrahydrofuranyloxycarbonylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-(3-tetrahydropyranyloxycarbonylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-(4-tetrahydropyranyloxycarbonylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-((2-tetrahydropyranyl)methoxycarbonylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-(2-methoxyacetylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-(2-(2-methoxyethoxy)acetylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-(2-(2-(2-methoxyethoxy)ethoxy)acetylamino)-4(S)-hydroxy-6-phenyl-2(R)-((4-(2-(4-morpholinyl)ethoxy)phenyl)-methyl)hexanamide,

N-(2(R)-hydroxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-((2(R)-tetrahydrofuranyl)carbonylamino)-4(S)-hydroxy-6-phenyl-2(R)-((4-(2-(4-morpholinyl)ethoxy)phenyl)-methyl)hexanamide,

N-(2(R)-hydroxy-1(S)-indanyl)-5(S)-((2(S)-tetrahydrofuranyl)carbonylamino)-4(S)-hydroxy-6-phenyl-2(R)-((4-(2-(4-morpholinyl)ethoxy)phenyl)-methyl)hexanamide,

N-(2(R)-hydroxy-1(S)-indanyl)-5(S)-((3-tetrahydrofuranyl)carbonylamino)-4(S)-hydroxy-6-phenyl-2(R)-((4-(2-(4-morpholinyl)ethoxy)phenyl)-methyl)hexanamide,

N-(2(R)-hydroxy-1(S)-indanyl)-5(S)-(2-methylpropyloxycarbonylamino)-4(S)-hydroxy-6-phenyl-2(R)-((4-(2-(4-morpholinyl)ethoxy)phenyl)-methyl)hexanamide,

N-(2(R)-hydroxy-1(S)-indanyl)-5(S)-(2-methoxyethoxycarbonylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-(2-(2-methoxyethoxy)ethoxycarbonylamino)-4(S)-hydroxy-6-phenyl-2(R)-((4-(2-(4-morpholinyl)ethoxy)phenyl)-methyl)hexanamide,

N-(2(R)-hydroxy-1(S)-indanyl)-5(S)-(2-(2-(2-methoxyethoxy)ethoxy)ethoxycarbonylamino)-4(S)-hydroxy-6-phenyl-2(R)-((4-(2-(4-morpholinyl)ethoxy)-phenyl)methyl)hexanamide,

N-(2(R)-hydroxy-1(S)-indanyl)-5(S)-(1,1-dimethylethoxycarbonylamino)-4(S)-hydroxy-6-phenyl-2(R)-((4-(2-(2-oxomorpholin-4-yl)ethoxy)phenyl)methyl)hexanamide,

N-(2(R)-hydroxy-1(S)-indanyl)-5(S)-(1,1-dimethylethoxycarbonyl-N-methylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-(1,1-dimethylethoxycarbonylamino)-4(S)-hydroxy-6-phenyl-2(R)-((4-(2-(4-morpholinyl)ethylthio)phenyl)methyl)hexanamide,

N-(2(R)-hydroxy-1(S)-indanyl)-5(S)-((carbobenzyloxy)amino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-(4-pyridylmethy-oxycarbonylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-(n-propyloxycarbonylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-(n-butyloxycarbonylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-(n-pentyloxycarbonylamino)-4(S)-hydroxy-6-phenyl-2(R)-((4-(2-(4-morpholinyl)ethoxy)phenyl)methyl)hexanamide,

N-(2(R)-hydroxy-1(S)-indanyl)-5(S)-(1,1-dimethylethoxycarbonylamino)-3(S),4(R)-dihydroxy-6-phenyl-2(R)-((4-(2-(4-morpholinyl)ethoxy)phenyl)methyl)hexanamide,
N-(4(R)-hydroxy-2(R)-methyl-1(R)-cyclopentyl)-5(S)-(1,1-dimethylethoxycarbonylamino)-4(S)-hydroxy-6-phenyl-2(R)-((4-(2-(4-morpholinyl)ethoxy)phenyl)-methyl)hexanamide,
N-(4(R)-hydroxy-2(S)-methyl-1(R)-cyclopentyl)-5(S)-(1,1-dimethylethoxycarbonylamino)-4(S)-hydroxy-6-phenyl-2(R)-((4-(2-(4-morpholinyl)ethoxy)phenyl)-methyl)hexanamide,
N-(2(R)-hydroxy-5(R)-methyl-1(S)-cyclopentyl)-5(S)-(1,1-dimethylethoxycarbonylamino)-4(S)-hydroxy-6-phenyl-2(R)-((4-(2-(4-morpholinyl)ethoxy)phenyl)-methyl)hexanamide,
N-(2(R)-hydroxy-5(S)-methyl-1(S)-cyclopentyl)-5(S)-(1,1-dimethylethoxycarbonylamino)-4(S)-hydroxy-6-phenyl-2(R)-((4-(2-(4-morpholinyl)ethoxy)phenyl)-methyl)hexanamide,
N-(2(S),3(R)-dihydroxy-5(R)-methyl-1(S)-cyclopentyl)-5(S)-(1,1-dimethylethoxycarbonylamino)-4(S)-hydroxy-6-phenyl-2(R)-((4-(2-(4-morpholinyl)ethoxy)phenyl)-methyl)hexanamide,
N-(2(S),3(R)-dihydroxy-5(S)-methyl-1(S)-cyclopentyl)-5 (S)-(1,1-dimethylethoxycarbonylamino)-4(S)-hydroxy-6-phenyl-2(R)-((4-(2-(4-morpholinyl)ethoxy)phenyl)-methyl)hexanamide,
N-(3(S)-hydroxy-4(S)-thiochromanyl)-5(S)-(1,1-dimethylethoxycarbonylamino)-4(S)-hydroxy-6-phenyl-2(R)-((4-(2-(4-morpholinyl)ethoxy)phenyl)methyl)-hexanamide,
N-(1(S)-oxo-4(R)-thiochromanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-bicyclo[4,3,0]nonyl)-5(S)-(1,1-dimethylethoxycarbonylamino)-4(S)-hydroxy-6-phenyl-2(R)-((4-(2-(4-morpholinyl)ethoxy)phenyl)-methyl)hexanamide,
N-(1(R,S)-oxo-4(S)-isothiochromanyl)-5(S)-(1,1-dimethylethoxycarbonylamino)-4(S)-hydroxy-6-phenyl-2(R)-((4-(2-(4-morpholinyl)ethoxy)phenyl)-methyl)hexanamide,
N-(2(R)-hydroxy-1(S)-indanyl)-5(S)-(1,1-dimethylethoxycarbonyl)amino-4(S)-hydroxy-6-phenyl-2(R)-[4-(2-(4-azabicyclo[2.2.2]octanyl-1-aminiumyl)ethoxy)-phenylmethylhexanamide,
N-[(1-hydroxycyclohexyl)methyl]-5(S)-t-butoxycarbonylamino-4(S)-hydroxy-6-phenyl-2(R)-(4-[2-morpholinoethoxy]-phenyl)methylhexanamide,
N-(3(S)-amino-2(R)-hydroxy-1(S)-indanyl)-5(S)-t-butoxycarbonylamino-4(S)-hydroxy-6-phenyl-2(R)-(4-[2-morpholinoethoxy]phenyl)methylhexanamide,
N-(2(R)-hydroxy-3A,4,5,6,7,7A-hexahydro-indan-1(S)-yl)-5(S)-(1,1-dimethylethoxycarbonylamino)-4(S)-hydroxy-6-phenyl-2(R)-(4-(2-(4-morpholinyl)ethoxy)-phenyl)methylhexanamide,
N-(2(R)-((3-carboxypropanoyl)oxy)-1(S)-indanyl)-5(S)-(1,1-dimethylethoxycarbonylamino)-4(S)-hydroxy-6-phenyl-2(R)-((4-(2-(4-morpholinyl)ethoxy)phenyl)-methylhexanamide,
N-(2(R)-hydroxy-1(S)-indanyl)-5(S)-succinoylamino-4(S)-hydroxy-6-phenyl-2(R)-[(4-(2-morpholinoethoxy)phenyl)methyl]hexanamide,
N-(2(R)-[(2-(2-(2-methoxyethoxy)ethoxy)ethoxy)-carbonyl]oxy)-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(4-tetrahydropyranylcarbonylamino)-4(S)-hydroxy-6-phenyl-2(R)-((4-(2-(4-morpholinyl)ethoxy)phenyl)methylhexanamide,
N-(2(R)-hydroxy-1(S)-indanyl)-5(S)-((4-tetrahydropyranyl)acetamido)-4(S)-hydroxy-6-phenyl-2(R)-((4-(2-(4-morpholinyl)ethoxy)phenyl)methylhexanamide,
N-[(cis-2(R)-(1′-methyl-3′-(S)-quinuclidiniumylcarbonyl)oxy-(1(S)-indanyl)-5(S)-(1.1-dimethyletoxycarbonylamino)-4(S)-hydroxy-6-phenyl-2(R)-[4-(2-morpholinoethoxy)phenyl)methyl]hexanamide,
N-(2(R)-hydroxy-1(S)-indanyl)-5(S)-acetylamino-4(S)-hydroxy-6-phenyl-2(R)-(4-(2-(4-morpholinyl)-ethoxy)phenyl)hexanamide,
N-(cis-2(R)-[4-(4-methyl-1-piperazinylmethyl)-benzoyl]oxy-1(S)-indanyl]-5(S)-(1,1-dimethylethoxycarbonylamino)-4(S)-hydroxy-6-phenyl-2(R)-[4-(2-morpholinoethoxy)phenyl]methylhexanamide,
N-(cis-2(R)-hydroxy-1(S)-indanyl)-5(S)-[2(S)-(1,1-dimethylethoxycarbonylamino)-4-hydroxybutyrylamino]-4(S)-hydroxy-6-phenyl-2(R)-[(4-(2-morpholinoethoxy)-phenyl)methyl]hexanamide,
N-(cis-(2(R)-hydroxy-1(S)-indanyl)-5(S)-[(5-oxo-2(S)-tetrahydrofuranyl)carbonylamino]-4(S)-hydroxy-6-phenyl-2(R)-[4-(2-morpholinoethoxy)phenyl]methyl-hexanamide,
N-(cis-2(R)-hydroxy-1(S)-indanyl)-5(S)-[2(S)-(amino-4-hydroxybutyryl)amino]-4(S)-hydroxy-6-phenyl-2(R)-[(4-(2-morpholinoethoxy)phenyl)methyl]hexanamide,
N-(2(R)-[(4-(morpholinomethyl)benzoyl)oxy]-1(S)-indanyl)-5(S)-(1,1-dimethylethoxycarbonylamino)-4(S)-hydroxy-6-phenyl-2(R)-[(4-(2-morpholinoethoxy)phenyl)-methyl]hexanamide,
N-(cis-2(R)-hydroxy-1(S)-indanyl)-5(S)-[1-trans-(2-hydroxyindanylidenyl)carbonyl]amino-4(S)-hydroxy-6-phenyl-2(R)-[(4-(2-morpholinoethoxy)phenyl)methyl]-hexanamide,
N-(cis-2(R)-hydroxy-1(S)-indanyl)-5(S)-[1-cis-(2-hydroxyindanylidenyl)carbonyl]amino-4(S)-hydroxy-6-

phenyl-2(R)-[(4-(2-morpholinoethoxy)phenyl)methyl]-hexanamide,
N-(cis-2(R)-hydroxy-1(S)-indanyl)-5(S)-[(2-hydroxy-1-indanylmethyl)carbonyl]amino-4(S)-hydroxy-6-phenyl-2(R)-[(4-(2-morpholinoethoxy)phenyl)methyl]hexanamide,
N-(cis-2(R)-hydroxy-1(S)-indanyl)-5(S)-[(4-morpholinyl)carbonyl]amino-4(S)-hydroxy-6-phenyl-2(R)-[(4-(2-morpholinoethoxy)phenyl)methyl]hexanamide,
N-(2(R)-hydroxy-1(S)-indanyl)-5(S)-(3(R)-tetrahydrofuranyloxycarbonylamino)-4(S)-hydroxy-6-phenyl-2(R)-(4-(2-(4-morpholinyl)ethoxy)phenyl)methylhexanamide,
N-(cis-2(S)-hydroxy-1(S)-benzopyranyl)-5(S)-(2-methoxyethoxycarbonylamino-4(S)-hydroxy-6-phenyl-2(R)-[(4-(2-morpholinoethoxy)phenyl)methyl]hexanamide,
N-(cis-2(S)-hydroxy-1(S)-benzopyranyl)-5(S)-(3,6,9,12-tetraoxatridecyloxycarbonylamino)-4(S)-hydroxy-6-phenyl-2(R)-[(4-(2-morpholinoethoxy)phenyl)methyl]-hexanamide,
N-(2(R)-hydroxy-1(S)-indanyl)-5(S)-(hexahydrofuro[2,3-b]furanyl-3A-oxycarbonylamino)-4(S)-hydroxy-6-phenyl-2(R)-(4-(2-(4-morpholinyl)ethoxy)phenyl)-methylhexanamide,
N-(cis-2(R)-hydroxy-1(S)-indanyl)-5(S)-[(2,2-dimethyl-1,3-dioxolane-4-methyloxy)carbonyl]amino-4(S)-hydroxy-6-phenyl-2(R)-[(4-(2-morpholinoethoxy)-phenyl)methyl]hexanamide,
N-(cis-2(R)-hydroxy-1(S)-indanyl)-5(S)-[(2-furanyl-methyloxy)carbonyl]amino-4(S)-hydroxy-6-phenyl-2(R)-[(4-(2-morpholinoethoxy)phenyl)methyl]hexanamide,
N-[2(R)-hydroxy-1(S)-indanyl]-5(S)-(N′-acetylneuraminoyl]amino-4(S)-hydroxy-6-phenyl-2(R)-(4-(2-morpholino)ethoxy)phenyl]methylhexanamide,
N-(2(R)-hydroxy-1(S)-indanyl)-5(S)-[2-(1,3-bis-(methoxyethoxy))propoxycarbonyl]amino-4(S)-hydroxy-6-phenyl-2(R)-[4-(2-(4-morpholino)ethoxy)phenyl]-methylhexanamide,
N-(2(R)-hydroxy-1(S)-indanyl)-5(S)-[5(R)-hydroxymethyl-3(S)-methoxytetrahydrofuranyloxyamino-4(S)-hydroxy-6-phenyl-2(R)-(4-(2-(4-morpholinyl)ethoxy)-phenyl)methyl]hexanamide,
N-(cis-2(R)-hydroxy-1(S)-indanyl)-5(S)-[(3-methoxy-1-butyloxy)carbonyl]amino-4(S)-hydroxy-6-phenyl-2(R)-[(4-(2-morpholinoethoxy)phenyl)methyl]hexanamide,
N-(cis-2(R)-hydroxy-1(S)-indanyl)-5(S)-[(1-methoxy-2-propyloxy)carbonyl]amino-4(S)-hydroxy-6-phenyl-2(R)-[(4-(2-morpholinoethoxy)phenyl)methyl]hexanamide,
N-(2(R)-hydroxy-1(S)-indanyl]-5(S)-[2-(1,3-bis-(methoxy)propoxycarbonyl]amino-4(S)-hydroxy-6-phenyl-2(R)-[4-(2-(4-morpholino)ethoxy)phenyl]methylhexanamide,
N-(cis-2(R)-hydroxy-1(S)-indanyl)-5(S)-[(2-(1,4-benzodioxanyl)methyl)oxycarbonyl]amino-4(S)-hydroxy-6-phenyl-2(R)-[(4-(2-morpholinoethoxy)phenyl)methyl]-hexanamide,
N-(2,2-dioxo-4(S)-isothiochromanyl)-5(S)-(1,1-dimethylethoxycarbonylamino)-4(S)-hydroxy-6-phenyl-2(R)-((4-(2-morpholinoethoxy)phenyl)methyl]-hexanamide,
N-(2(R,S)-oxo-4(S)-isothiochromanyl)-5(S)-(1,1-dimethylethoxycarbonylamino)-4(S)-hydroxy-6-phenyl-2(R)-((4-(2-morpholinoethoxy)phenyl)methyl]-hexanamide,
N-(cis-2(R)-hydroxy-1(S)-indanyl)-5(S)-[(2,3-bis-methoxyethyloxy)propyloxycarbonyl]amino-4(S)-hydroxy-6-phenyl-2(R)-[4-(2-morpholinoethoxy)phenyl)methyl]-hexanamide,
N-(cis-2(R)-hydroxy-1(S)-indanyl)-5(S)-[4H-tetrahydro-4-pyranyloxymethylcarbonyl]amino-4(S)-hydroxy-6-phenyl-2(R)-[4-(2-morpholinoethoxy)phenyl)methyl]-hexanamide,
N-(2(R)-hydroxy-1(S)-indanyl)-5(S)-(1,1-dimethylethoxycarbonylamino)-4(S)-hydroxy-6-cyclohexyl-2(R)-[4-(2-(4-morpholino)ethoxy)phenylmethyl]hexanamide,
N-(cis-2(R)-hydroxy-1(S)-indanyl)-5(S)-[1,1-dimethylethoxycarbonyl-L-asparaginyl]amino-4(S)-hydroxy-6-phenyl-2(R)-[(4-(2-morpholinoethoxy)phenyl)methyl]-hexanamide,
N-(4(S)-isothiochromanyl)-5(S)-(1,1-dimethylethoxycarbonylamino)-4(S)-hydroxy-6-phenyl-2(R)-((4-(2-morpholinoethoxy)phenyl)methyl)hexanamide,
N-(cis-2(R)-hydroxy-1(S)-indanyl)-5(S)-[[N-2-quinolylcarbonyl-L-asparaginyl]amino-4(S)-hydroxy-6-phenyl-2(R)-[(4-(2-morpholinoethoxy)phenyl)methyl]hexanamide,
N-(2(R)-hydroxy-1(S)-indanyl)-5(S)-(2-furanylmethoxycarbonylamino)-4(S)-hydroxy-2(R)-(4-(2-(4-morpholino)-ethoxy)phenyl)methyl-6-cyclohexylhexanamide,
N-(2(R)-hydroxy-1(S)-indanyl)-5(S)-((3(R,S)-tetrahydrofuranyl)acetamido)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-((2(R,S)-tetrahydrofuranyl)acetamido)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-((4,5-dihydro-3(2H)-furylidene)acetamido)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-(1,1-dimethylethoxycarbonylamino)-4(S)-hydroxy-6-cyclohexyl-2(R)-(4-(2-(4-methylpiperazinyl)ethoxy)phenyl)methylhexanamide,
N-(2(R)-hydroxy-1(S)-indanyl)-5(S)-[(2-furanylmethyloxycarbonyl]amino-4(S)-hydroxy-6-phenyl-2(R)-[3-(4-(2-morpholinoethoxy)phenylprop-2-en-1-yl)hexanamide, N-(2(R)-hydroxy-1(S)-indanyl)-5(S)-[(2-methoxyethoxy)carbonyl]amino-4(S)-hydroxy-6-phenyl-2(R)-[3-(4-(2-morpholinoethoxy)phenylprop-2-en-1-yl)hexanamide, N-(2(R)-hydroxy-1(S)-indanyl)-5(S)-(1,1-dimethylethoxycarbonyl)amino-4(S)-hydroxy-6-cyclohexyl-2(R)-(3-(4-(2-(4-morpholinyl)ethoxy)phenyl)prop-2-en-1-yl)hexanamide, N-(2(R)-hydroxy-1(S)-indanyl)-5(S)-(1,1-dimethylethoxycarbonyl)amino-4(S)-hydroxy-6-cyclohexyl-2(R)-(4-(2-(diethylamino)ethoxy)phenyl)methylhexanamide, N-(2(R)-hydroxy-1(S)-indanyl)-5(S)-(1,1-dimethylethoxycarbonylamino)-4(S)-hydroxy-6-cyclohexyl-2(R)-(4-(2-(diisopropylamino)ethoxy)phenyl)methylhexanamide, N-(2(R)-hydroxy-1(S)-indanyl)-5(S)-(1,1-dimethylethoxycarbonylamino)-4(S)-hydroxy-6-(4-(2-dimethylaminoethoxy)phenyl)-2(R)-(3-phenyl-2-propen-1-yl)-hexanamide, N-(2(R)-hydroxy-1(S)-indanyl)-5(S)-(3(R.S)-tetrahydropyranyloxycarbonylamino)-4(S)-hydroxy-6-phenyl-2(R)-(4-(2-(4-morpholinyl)ethoxy)phenyl)methylhexanamide, N-(2(R)-hydroxy-1(S)-indanyl)-5(S)-(5(R)-methoxymethyl-3(S)-tetrahydrofuranyloxycarbonylamino)-4(S)-hydroxy-6-phenyl-2(R)-(4-(2-(4-morpholinyl)ethoxy)-phenyl)methylhexanamide, N-(2(R)-hydroxy-1(S)-indanyl)-5(S)-(5-(2-acetoxy-1,4 :3,6-dianhydro-D-glucityl)carbonylamino)-4(S)-hydroxy-6-phenyl-2(R)-(4-(2-(4-morpholinyl)ethoxy)-phenyl)methylhexanamide, N-(2(R)-hydroxy-1(S)-indanyl)-5(S)-(3(S)-methyl-3-tetrahydrofuranyloxycarbonylamino)-4(S)-hydroxy-6-phenyl-2(R)-(4-(2-(4-morpholinyl)ethoxy)phenyl)methylhexanamide, N-(2(R)-hydroxy-1(S)-indanyl)-5(S)-(4(S)-hydroxy-5(S)-(hydroxymethyl)-3(R)-tetrahydrofuranyloxycarbonylamino)-4(S)-hydroxy-6-phenyl-2(R)-(4-(2-(4-morpholinyl)ethoxy)phenyl)methylhexanamide, N-(2(R)-hydroxy-1(S)-indanyl)-5(S)-(4(S)-benzyloxy-3(R)-tetrahydrofuranyloxycarbonylamino)-4(S)-hydroxy-6-phenyl-2(R)-(4-(2-(4-morpholinyl)ethoxy)phenyl)-methylhexanamide, N-(2(R)-hydroxy-1(S)-indanyl)-5(S)-(4(R)-benzyloxy-3(R)-tetrahydrofuranyloxycarbonylamino)-4(S)-hydroxy-6-phenyl-2(R)-(4-(2-(4-morpholinyl)ethoxy)phenyl)-methylhexanamide, N-(2(R)-hydroxy-1(S)-indanyl)-5(S)-(4(S)-methoxy-3(R)-tetrahydrofuranyloxycarbonylamino)-4(S)-hydroxy-6-phenyl-2(R)-(4-(2-(4-morpholinyl)ethoxy)phenyl)-methylhexanamide, N-(2(R)-hydroxy-1(S)-indanyl)-5(S)-(4(R)-methoxy-3(R)-tetrahydrofuranyloxycarbonylamino)-4(S)-hydroxy-6-phenyl-2(R)-(4-(2-(4-morpholinyl)ethoxy)phenyl)-methylhexanamide, N-(2(R)-hydroxy-1(S)-indanyl)-5(S)-(5(S)-methyl-3(S)-tetrahydrofuranyloxycarbonylamino)-4(S)-hydroxy-6-phenyl-2(R)-(4-(2-(4-morpholinyl)ethoxy)phenyl)-methylhexanamide, N-(2(R)-hydroxy-1(S)-indanyl)-5(S)-(5(R)-methyl-3(S)-tetrahydrofuranyloxycarbonylamino)-4(S)-hydroxy-6-phenyl-2(R)-(4-(2-(4-morpholinyl)ethoxy)phenyl)-methylhexanamide, N-(2(R)-hydroxy-1(S)-indanyl)-5(S)-(5(R)-methoxymethyl-3(S)-tetrahydrofuranyloxycarbonylamino)-4(S)-hydroxy-6-phenyl-2(R)-(4-(2-(4-morpholinyl)ethoxy)-phenyl)methylhexanamide, N-(2(R)-hydroxy-1(S)-indanyl)-5(S)-(5(S)-methoxymethyl-3(S)-tetrahydrofuranyloxycarbonylamino)-4(S)-hydroxy-6-phenyl-2(R)-(4-(2-(4-morpholinyl)ethoxy)-phenyl)methylhexanamide, N-(2(R)-hydroxy-1(S)-indanyl)-5(S)-(4(S)-methoxy-5(S)-methoxymethyl-3(R)-tetrahydrofuranyloxycarbonylamino)-4(S)-hydroxy-6-phenyl-2(R)-(4-(2-(4-morpholinyl)ethoxy)phenyl)methylhexanamide, N-(2(R)-hydroxy-1(S)-indanyl)-5(S)-(3(S)-tetrahydrofuranyloxycarbonylamino)-4(S)-hydroxy-6-phenyl-2(R)-(trans-3-(4-(2-(4-morpholinyl)ethoxy)phenyl)prop-2-en-1-yl)hexanamide, N-(2(R)-hydroxy-1(S)-indanyl)-5(S)-(3(S)-tetrahydropyranyloxycarbonylamino)-4(S)-hydroxy-6-phenyl-2(R)-(trans-3-(4-(2-(4-morpholinyl)ethoxy)phenyl)prop-2-en-1-yl)hexanamide, N-(2(R)-hydroxy-1(S)-indanyl)-5(S)-(3(S)-tetrahydrofuranyloxycarbonylamino)-4(S)-hydroxy-6-cyclohexyl-2(R)-(trans-3-(4-(2-(4-morpholinyl)ethoxy)phenyl)-prop-2-en-1-yl)hexanamide, N-(2(R)-hydroxy-1(S)-indanyl)-5(S)-(3(S)-tetrahydropyranyloxycarbonylamino)-4(S)-hydroxy-6-cyclohexyl-2(R)-(trans-3-(4-(2-(4-morpholinyl)ethoxy)phenyl)-prop-2-en-1-yl)hexanamide, N-(2(R)-hydroxy-1(S)-indanyl)-5(S)-(3(S)-tetrahydrofuranyloxycarbonylamino)-4(S)-hydroxy-6-cyclohexyl-2(R)-(4-(2-(4-morpholinyl)ethoxy)phenyl)methylhexanamide, N-(2(R)-hydroxy-1(S)-indanyl)-5(S)-(3(S)-tetrahydrofuranyloxycarbonylamino)-4(S)-hydroxy-6-cyclohexyl-2(R)-(4-(2-diethylaminoethoxy)phenyl)methyl)-hexanamide, N-(2(R)-hydroxy-1(S)-indanyl)-5(S)-(3(S)-tetrahydrofuranyloxycarbonylamino)-4(S)-hydroxy-6-cyclohexyl-2(R)-(4-(2-(N,N-di-2-propyl)aminoethoxy)phenyl)-methylhexanamide, N-(2(R)-hydroxy-1(S)-indanyl)-5(S)-(3(S)-tetrahydrofuranyloxycarbonylamino)-4(S)-hydroxy-6-cyclohexyl-2(R)-(4-(2-(4-methyl-1-piperazinyl)ethoxy)phenyl)-methylhexanamide, N-(2(R)-hydroxy-1(S)-indanyl)-5(S)-(3(S)-tetrahydropyranyloxycarbonylamino)-4(S)-hydroxy-6-cyclohexyl-2(R)-(4-(2-(4-methyl-1-piperazinyl)ethoxy)phenyl)-methylhexanamide, N-(2(R)-hydroxy-1(S)-indanyl)-5(S)-(3(S)-1,1-dioxotetrahydrothiofuranyloxycarbonylamino)-4(S)-hydroxy-6-cyclohexyl-2(R)-(4-(2-(4-morpholinyl)ethoxy)phenyl)-methylhexanamide,
N-(2(R)-hydroxy-1(S)-indanyl)-5(S)-(3(S)-1,1-dioxotetrahydrothiofuranyloxycarbonylamino)-4(S)-hydroxy-6-phenyl-2(R)-(trans-3-(4-(2-(4-morpholinyl)ethoxy)-phenyl)prop-2-en-1-yl)hexanamide,
N-(2(R)-hydroxy-1(S)-indanyl)-5(S)-(3(S)-1,1-dioxotetrahydrothiofuranyloxycarbonylamino)-4(S)-hydroxy-6-cyclohexyl-2(R)-(trans-3-(4-(2-(4-morpholinyl)-ethoxy)phenyl)prop-2-en-1-yl)hexanamide,
N-(2(R)-hydroxy-1(S)-indanyl)-5(S)-(3(S)-1,1-dioxotetrahydrothiofuranyloxycarbonylamino)-4(S)-hydroxy-6-cyclohexyl-2(R)-(4-(2-(4-methyl-1-piperazinyl)-ethoxy)phenyl)methylhexanamide,
N-(2(R)-hydroxy-1(S)-indanyl)-5(S)-(3(S)-1,1-dioxotetrahydrothiopyranyloxycarbonylamino)-4(S)-hydroxy-6-cyclohexyl-2(R)-(4-(2-(4-morpholinyl)ethoxy)phenyl)-methylhexanamide,
N-(2(R)-hydroxy-1(S)-indanyl)-5(S)-(3(S)-1,1-dioxotetrahydrothiopyranyloxycarbonylamino)-4(S)-hydroxy-6-phenyl-2(R)-(trans-3-(4-(2-(4-morpholinyl)ethoxy)-phenyl)prop-2-en-1-yl)hexanamide,
N-(2(R)-hydroxy-1(S)-indanyl)-5(S)-(3(S)-1,1-dioxotetrahydrothiopyranyloxycarbonylamino)-4(S)-hydroxy-6-cyclohexyl-2(R)-(trans-3-(4-(2-(4-morpholinyl)-ethoxy)phenyl)prop-2-en-1-yl)hexanamide,
N-(2(R)-hydroxy-1(S)-indanyl)-5(S)-(3(S)-1,1-dioxotetrahydrothiopyranyloxycarbonylamino)-4(S)-hydroxy-6-cyclohexyl-2(R)-(4-(2-(4-methyl-1-piperazinyl)-ethoxy)phenyl)methylhexanamide,
N-(2(R)-hydroxy-1(S)-indanyl)-5(S)-(4(S)-hydroxy-3(R)-tetrahydrofuranyloxycarbonylamino)-4(S)-hydroxy-6-phenyl-2(R)-(4-(2-(4-morpholinyl)ethoxy)phenyl)-methylhexanamide,
N-(2(R)-hydroxy-1(S)-indanyl)-5(S)-(4(R)-hydroxy-3(R)-tetrahydrofuranyloxycarbonylamino)-4(S)-hydroxy-6-phenyl-2(R)-(4-(2-(4-morpholinyl)ethoxy)phenyl)-methylhexanamide,
N-(2(R)-hydroxy-1(S)-indanyl)-5(S)-(4(S)-methyl-3(S)-tetrahydrofuranyloxycarbonylamino)-4(S)-hydroxy-6-phenyl-2(R)-(4-(2-(4-morpholinyl)ethoxy)phenyl)-methylhexanamide,
N-(2(R)-hydroxy-1(S)-indanyl)-5(S)-(4(R)-methyl-3(S)-tetrahydrofuranyloxycarbonylamino)-4(S)-hydroxy-6-phenyl-2(R)-(4-(2-(4-morpholinyl)ethoxy)phenyl)-methylhexanamide,
N-(2(R)-hydroxy-1(S)-indanyl)-5(S)-(2-methoxyethoxycarbonylamino)-4(S)-hydroxy-6-cyclohexyl-2(R)-(4-(2-(4-morpholinyl)ethoxy)phenyl)methylhexanamide,or
N-(2(R)-hydroxy-1(S)-indanyl)-5(S)-(3(S)-tetrahydrofuranyloxycarbonylamino)-4(S)-hydroxy-6-phenyl-2(R)-(4-(2-(4-morpholinyl)ethoxy)phenyl)methylhexanamide,
or pharmaceutically acceptable salts thereof.

Another embodiment of the present invention is the following compounds :
N-(2(R)-hydroxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)-methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(N,N-bis(2-methoxyethyl)amino)-ethoxy)phenyl)methyl)hexanamide,
N-(2(R)-hydroxy-1(S)-indanyl)-5(S)-(1-methylethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-ethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-cyclohexyl-2(R)-(4-(2-(4-morpholinyl)ethoxy)phenyl)methyl hexanamide,
N-(2(R)-hydroxy-1(S)-indanyl)-5(S)-(3(S)-tetrahydrofuranyloxycarbonylamino)-4(S)-hydroxy-6-cyclohexyl-2(R)-(4-(2-(4-morpholinyl)ethoxy)phenyl)methyl hexanamide,
N-(2(R)-hydroxy-1(S)-indanyl)-5(S)-(1,1-dimethylethoxycarbonylamino)-4(S)-hydroxy-6-cyclohexyl-2(R)-(4-(2-(4-methyl-1-piperazinyl)ethoxy)phenyl)methyl hexanamide,
N-(2(R)-hydroxy-1(S)-indanyl)-5(S)-(3(S)-tetrahydrofuranyloxycarbonylamino)-4(S)-hydroxy-6-cyclohexyl-2(R)-(4-(2-(4-methyl-1-piperazinyl)ethoxy)phenyl)methyl hexanamide,
N-(2(R)-hydroxy-1(S)-indanyl)-5(S)-(1,1-dimethylethoxycarbonylamino)-4(S)-hydroxy-6-cyclohexyl-2(R)-(4-(2-(N,N-di-2-propyl)aminoethoxy)phenyl)methyl hexanamide,
N-(2(R)-hydroxy-1(S)-indanyl)-5(S)-(3(S)-tetrahydrofuranyloxycarbonylamino)-4(S)-hydroxy-6-cyclohexyl-2(R)-(4-(2-(N,N-di-2-propyl)aminoethoxy)phenyl)methyl hexanamide,
N-(2(R)-hydroxy-1(S)-indanyl)-5(S)-(1,1-dimethylethoxycarbonylamino)-4(S)-hydroxy-6-cyclohexyl-2(R)-(trans-3-(4-(2-(4-morpholinyl)ethoxy)phenyl)prop-2-en-1-yl)hexanamide,
N-(2(R)-hydroxy-1(S)-indanyl)-5(S)-(3(S)-tetrahydrofuranyloxycarbonylamino)-4(S)-hydroxy-6-cyclohexyl-2(R)-(trans-3-(4-(2-(4-morpholinyl)ethoxy)phenyl)prop-2-en-1-yl)hexanamide,
N-(2(R)-hydroxy-1(S)-indanyl)-5(S)-(1,1-dimethylethoxycarbonylamino)-4(S)-hydroxy-6-phenyl-2(R)-(trans-3-(4-(2-(4-morpholinyl)ethoxy)phenyl)prop-2-en-1-yl)hexanamide,
N-(2(R)-hydroxy-1(S)-indanyl)-5(S)-(3(S)-tetrahydrofuranyloxycarbonylamino)-4(S)-hydroxy-6-phenyl-2(R)-(trans-3-(4-(2-(4-morpholinyl)ethoxy)phenyl)prop-2-en-1-yl)hexanamide, or
N-(2(R)-hydroxy-1(S)-indanyl)-5(S)-(2-methoxyethyloxycarbonylamino)-4(S)-hydroxy-6-phenyl-2(R)-

(trans-3-(4-(2-(4-morpholinyl)ethoxy)phenyl)prop-2-en-1-yl)hexanamide,
or pharmaceutically acceptable salts thereof.

In the compounds of the present invention, the A, G, B and J components and the like may have asymmetric centers and occur as racemates, racemic mixtures and as individual diastereomers, with all isomeric forms being included in the present invention.

When any variable (e.g., aryl, heterocycle, R, $R^1$, $R^2$, $R^3$, $R^4$, $R^7$, $R^9$, $R^{10}$, $R^{11}$, $R^{12}$, $R^{14}$, $R^{15}$, $R^{16}$, $R^{17}$, $A^{\ominus}$, n, Z, etc.) occurs more than one time in any constituent or in formula I, its definition on each occurrence is independent of its definition at every other occurrence. Also, combinations of substituents and/or variables are permissible only if such combinations result in stable compounds.

As used herein except where noted, "alkyl" is intended to include both branched— and straight-chain saturated aliphatic hydrocarbon groups having the specified number of carbon atoms (Me is methyl, Et is ethyl, Pr is propyl, Bu is butyl) ; "alkoxy" represents an alkyl group of indicated number of carbon atoms attached through an oxygen bridge ; and "cycloalkyl" is intended to include saturated ring groups, such as cyclopropyl, cyclobutyl, cyclopentyl, cyclohexyl (Cyh) and cycloheptyl. "Alkenyl" is intended to include hydrocarbon claims of either a straight or branched configuration and one or more unsaturated carbon-carbon bonds which may occur in any stable point along the chain, such as ethenyl, propenyl, butenyl, pentenyl, and the like. "Halo", as used herein, means fluoro, chloro, bromo and iodo ; and "counterion" is used to represent a small, single negatively-charged species, such as chloride, bromide, hydroxide, acetate, trifluroacetate, perchlorate, nitrate, benzoate, maleate, tartrate, hemitartrate, benzene sulfonate, and the like.

As used herein, with exceptions as noted, "aryl" is intended to mean phenyl (Ph) or naphthyl. "Carbocyclic" is intended to mean any stable 5- to 7-membered carbon ring or 7- to 10-membered bicyclic carbon ring, any of which may be saturated or partially unsaturated.

The term heterocycle or heterocyclic, as used herein except where noted, represents a stable 5- to 7-membered mono— or bicyclic or stable 7- to 10-membered bicyclic heterocyclic ring which is either saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. The heterocyclic ring may be attached at any heteroatom or carbon atom which results in the creation of a stable structure. Examples of such heterocyclic elements include piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazoyl, benzopyranyl, benzothiazolyl, benzoxazolyl, furyl, tetrahydrofuryl, tetrahydropyranyl, thienyl, benzothienyl, thiamorpholinyl, thiamorpholinyl sulfoxide, thiamorpholinyl sulfone, and oxadiazolyl. Morpholino is the same as morpholinyl.

The pharmaceutically-acceptable salts of the compounds of Formula I (in the form of water— or oil— soluble or dispersible products) include the conventional non-toxic salts or the quaternary ammonium salts of these peptides, which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quaternized with such agents as lower alkyl halides, such as methyl, ethyl, propyl, and butyl chloride, bromides and iodides ; dialkyl sulfates like dimethyl, diethyl, dibutyl ; and diamyl sulfates, long chain halides such as decyl, lauryl, myristyl and stearyl chlorides, bromides and iodides, aralkyl halides like benzyl and phenethyl bromides and others.

HIV protease inhibitors of Formula I may be prepared in accordance with well-known procedures for preparing peptide analogs from their constituent amino acids or analogs thereof. In general, once the G substituent is made, the rest of the synthesis follows the principle of amide bond formation by the coupling methods of either solution-phase or solid-phase peptide synthesis. The addition and removal of one or more protecting groups is also typical practice.

Amide couplings used to form the compounds of this invention are typically performed by the carbodiimide

method with reagents such as dicyclohexylcarbodiimide, or N-ethyl, N'-(3-dimethylaminopropyl) carbodiimide. Other methods of forming the amide or peptide bond include, but are not limited to synthetic routes via an acid chloride, azide, mixed anhydride or activated ester. Typically, solution phase amide couplings are performed, but solid-phase synthesis by classical Merrifield techniques may be employed instead.

The selection of protecting groups is, in part, dictated by particular coupling conditions, in part by the amino acid and peptide components involved in the reaction. Such amino-protecting groups ordinarily employed include those which are well known in the art, for example, urethane protecting substituents such as benzyloxycarbonyl (carbobenzoxy), p-methoxycarbobenzoxy, p-nitrocarbobenzoxy, t-butyloxycarbonyl, and the like. It is preferred to utilize t-butyloxycarbonyl (BOC) for protecting the a-amino group in the amino acids undergoing reaction at the carboxyl end of said amino acid, in part because the BOC protecting group is readily removed by relatively mild acids such as trifluoroacteic acid (TFA), or hydrogen chloride in ethyl acetate.

The OH group of Thr, Tyr or Ser and analogs thereof may be protected by the Bzl (benzyl) group and the epsilon-amino group of Lys may be protected by the IPOC group or the 2-chlorobenzyloxycarbonyl (2-C1-CBZ) group. Treatment with HF or catalytic hydrogenation are typically employed for removal of IPOC or 2-C1-CBZ.

Two schemes for preparing compounds of Formula I are presented below.

Scheme I (Y=O,N,S)

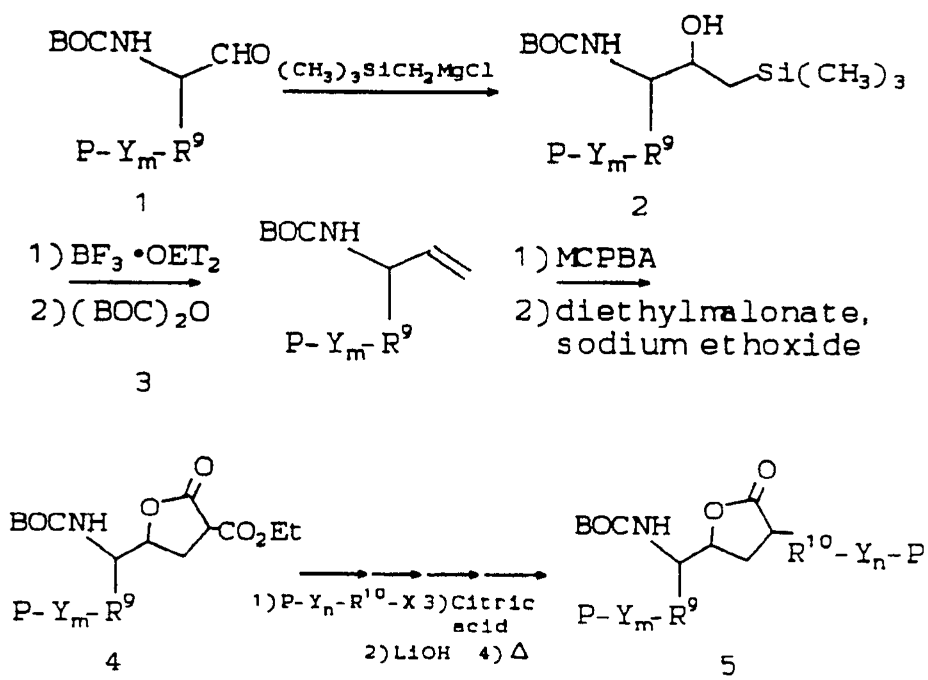


1 2 3 4 5

where P=protecting group

$Y_m$ and/or $Y_n$=O, N, S

X=C1, Br, I

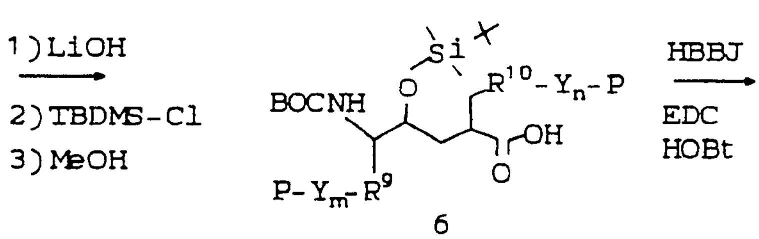


6

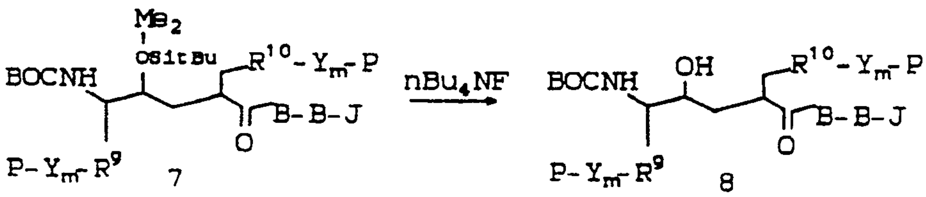


7 8

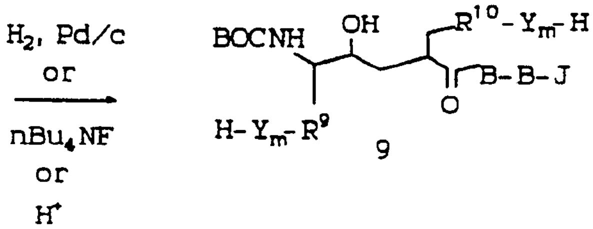


9

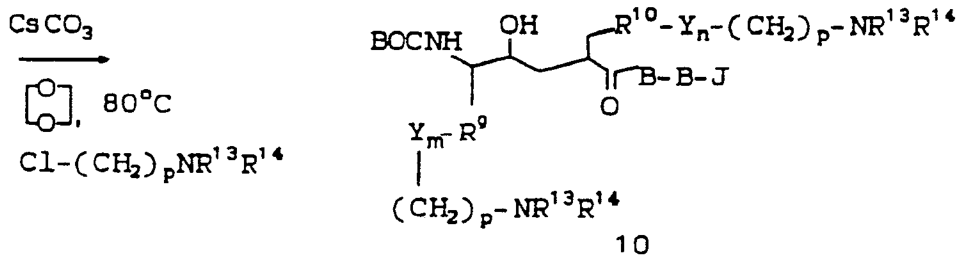


10

Where p=0-4
n=0-5
Y=O, N, S.

## Scheme II (Y=C)

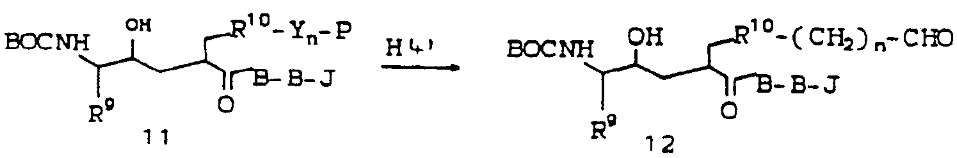


11 12

Where $Y_n$-P=-$(CH_2)$n-$CH(OMe)_2$

n=0 to 5

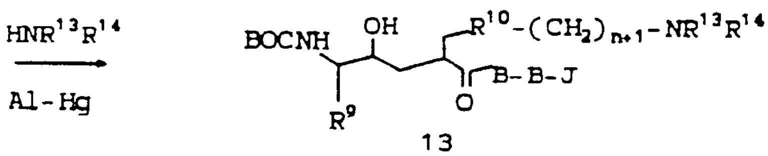


13

In Scheme I, wherein Y is O, N or S, the aldehyde 1 is converted to the alcohol 2 by conventional reaction with chloromethyltrimethylsilane. Subsequent Peterson olefin synthesis by reaction with borontrifluoride etherate results in dehydroxysilation and removal of the Boc group. The Boc group is added back with excess di-tert-butyldicarbonate to yield 3. Epoxidation of 3 by reaction with diethylmalonate and MCPBA and sodium ethoxide results in 4. Subsequent coupling with a halogenated $R^{10}$ eide chain by treatment with aqueous acid followed by aqueous base results in an intermediate carboxylated at C2. Heat removes the carboxyl group to give 5. The lactone is opened with base, then the hydroxyl group protected by silylation. Further conventional steps of coupling and removal of silyl or other protecting groups yields 9. Subsequent alkylation with cesium

carbonate at high temperature (at least about 80°C) yields 10.

When Y = C as in Scheme II, a different alkylating reagent is necessary. After removal of the protecting group with acid, 12 is subjected to conventional reductive amination to yield 13.

Additional schemes and synthetic methods are found in EPO 0 337 714, Application No. 89303539.4, published in October of 1989, which published application is herein incorporated by reference for these purposes.

The present invention is specifically illustrated by Tables I through IX.

TABLE I

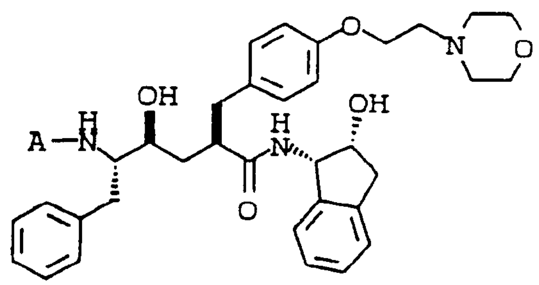

TABLE I (CONT'D)

| A | mp |
|---|---|
| 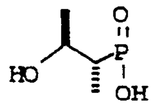 | amorphous |
| BOC-Ala | 132-3°C |
| $H_2N$-Ala | 118-20°C |
| 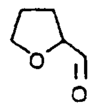 | 174-9°C |
| 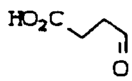 | 172-3°C |
| 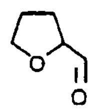 | 207-9°C |
| 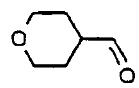 | 234-6°C |
| 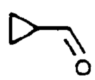 | 200-2°C |
| 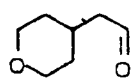 | 210-12°C |
| 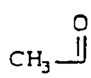 | 217-9°C |
| BOC-(homo)-Ser- | 161-4°C |
| 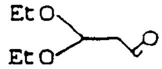 | 179-83°C |
| $H_2N$-(homo)-Ser | 139-42°C |

TABLE I (CONT'D)

| A | mp |
|---|---|
| 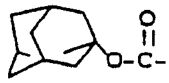 | 95-7°C |
| 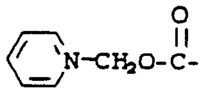 | 179-81°C |
| 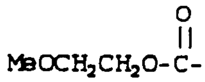 | 158-60°C |
| 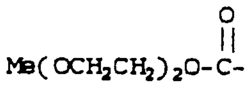 | 127-32°C |
| 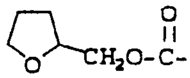 | 154-6°C |
| 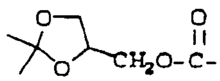 | 107-10°C |
| 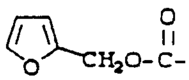 | 181-3°C |
| 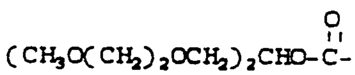 | 120-22°C |
| 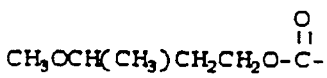 | 149-50°C |
| 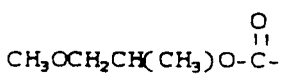 | 169-71°C |
| 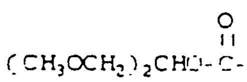 | 174-5°C |
| 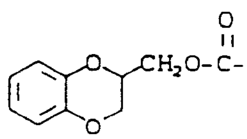 | 155-58°C |
| 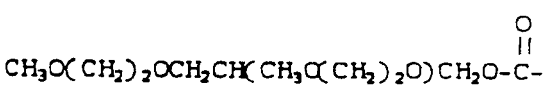 | amorphous |

TABLE I (CONT'D)

| A | mp |
|---|---|
| 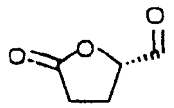 | 168-2°C |
| 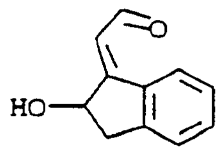 | 209-12°C |
| 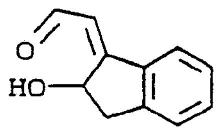 | 106-9°C |
| 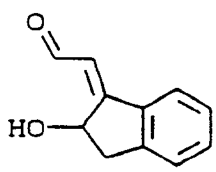 | 121-4°C |
| 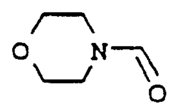 | 92-5°C |
| N' -acetylneuraminoyl | 206-9°C |

TABLE I (CONT'D)

| A | mp |
|---|---|
| 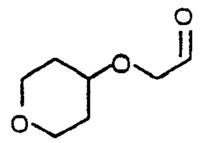 | 122-6°C |
| BOC-Asp- | 213-5°C |
| 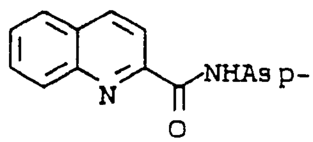 | 221-5°C |
| 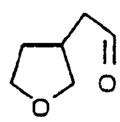 | 214-6°C |
| 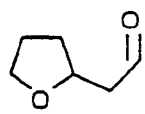 | 200-2°C |
| 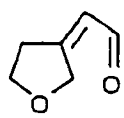 | 185-7°C |
| 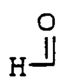 | |

TABLE I (CONT'D)

| A | mp |
| --- | --- |
| 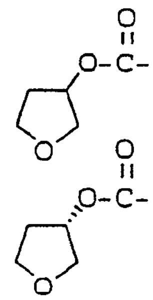 | 204-8°C |
| | 201-2°C |
| 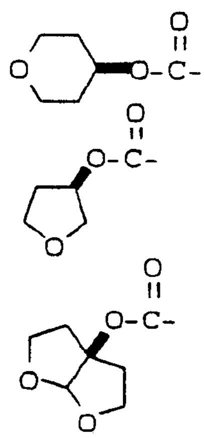 | 205-6°C |
| | 199-201°C |
| | 194-6°C |

TABLE I (CONT'D)

| A | mp |
| --- | --- |
| 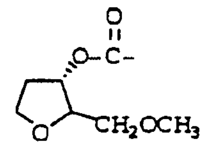 | |
| 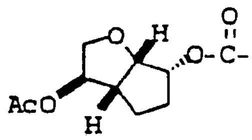 | 204-205°C |
| 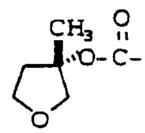 | |
| 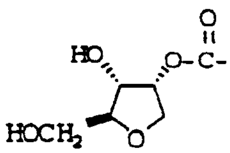 | |
| 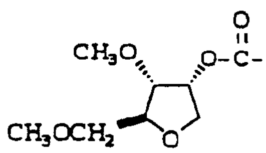 | |

TABLE I (CONT'D)

| A | mp |
|---|---|
| 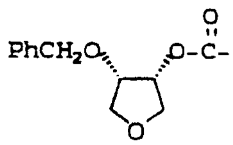 | 175-6°C |
| 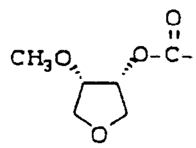 | 170-2°C |
| 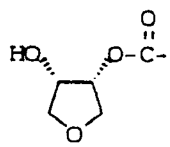 | |
| 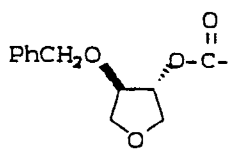 | 202-3°C |
| 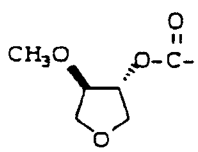 | 205-6°C |

TABLE I (CONT'D)

| A | mp |
|---|---|
| 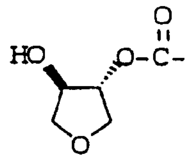 | |
| 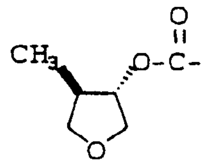 | |
| 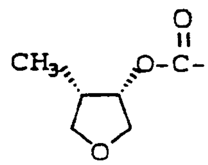 | |
| 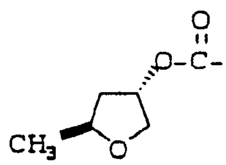 | |
| 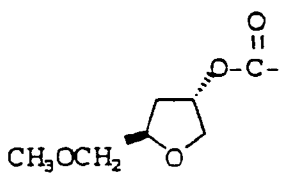 | |

TABLE II

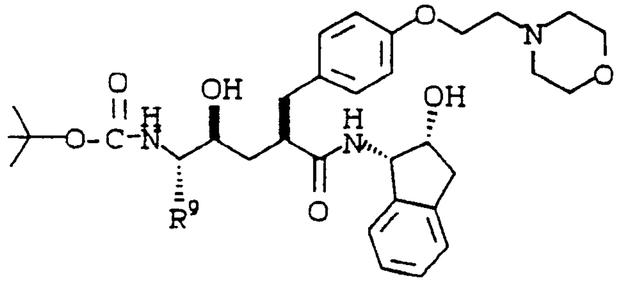

| $R^9$ |
| --- |
| $CH_3-$ |
| $CH_3CH_2-$ |
| $CH_3CH_2CH_2-$ |
| $CH_3CH_2CH_2CH_2-$ |
| $CH_3CH_2CH_2CH_2CH_2-$ |
| 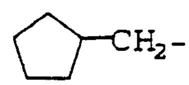 |
| $CH_2=CHCH_2-$ |
| $(CH_3)_2C=CHCH_2$ |
| $(CH_3)_2CH-$ |
| $CH_3CH_2CH(CH_3)-$ |
| 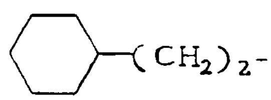 |
| 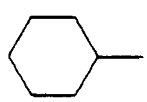 |
| 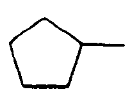 |

## TABLE III

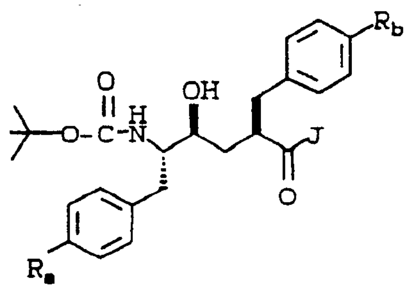

| $R_a$ | $R_b$ | J | mp |
|---|---|---|---|
| H | 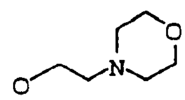 | 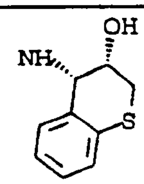 | 162-5°C |
| H | 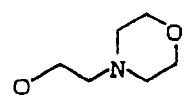 | 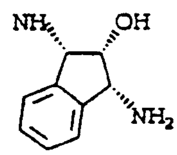 | 191-4°C |
| 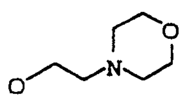 | 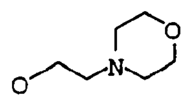 | 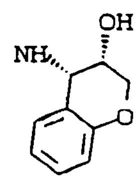 | 189-90°C |

TABLE III (CONT'D)

| $R_a$ | $R_b$ | J | mp |
|---|---|---|---|
| 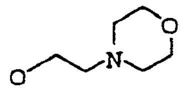 | OH | 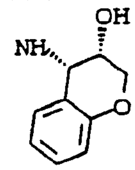 | 130-2°C |
| H | 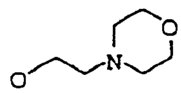 | 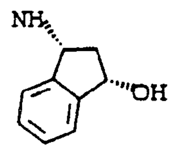 | 176-8°C |
| H | 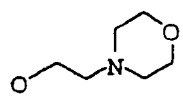 | 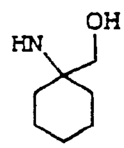 | 225-7°C |
| H | 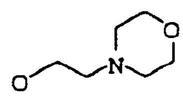 | 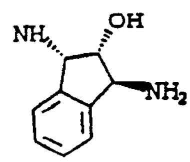 | amorphous |

TABLE IV

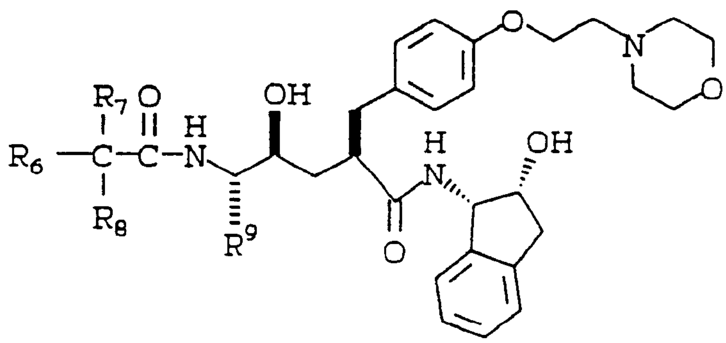

| 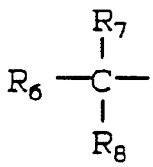 | $R^9$ | mp |
|---|---|---|
| tert-butyl | 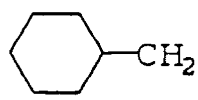 | 150-1°C |
| 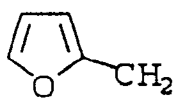 | 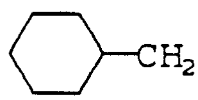 | 164-5°C |
| $CH_3OCH_2CH_2$ | 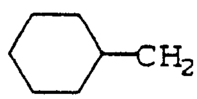 | 140-2°C |
| $CH_3OCH_2C(CH_3)_2$- | 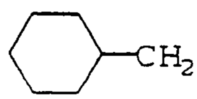 | |
| $(CH_3)_2CH_2$ | 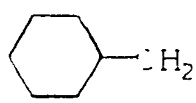 | |
| cyclopentyl | 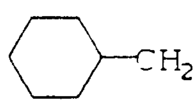 | |

TABLE IV (CONT'D)

| 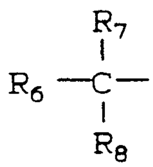 | $R^9$ | mp |
|---|---|---|
| cyclohexyl | 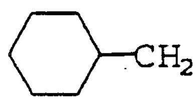 | |
| tert-butyl | 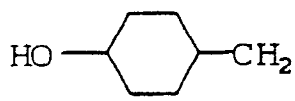 | |
| tert-butyl | $PhCH_2CH_2$ | |
| tert-butyl | 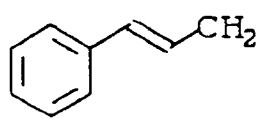 | |
| tert-butyl | 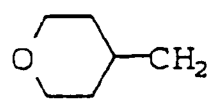 | |
| tert-butyl | 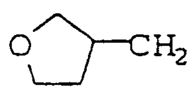 | |
| tert-butyl | isobutyl | |
| tert-butyl | 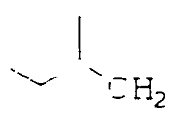 | |

TABLE V

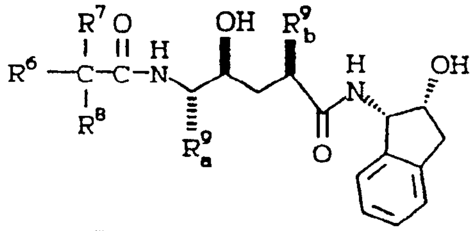

| 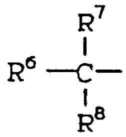 | $R^9_a$ | $R^9_b$ | mp |
|---|---|---|---|
| tert-butyl | $PhCH_2$ | 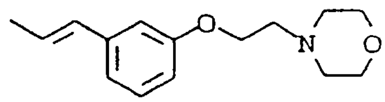 | 175-6°C |
| tert-butyl | $PhCH_2$ | $CH_2$ 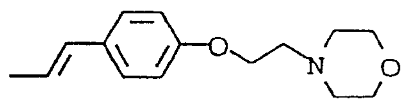 | 178-9°C |
| tert-butyl | $PhCH_2$ | $CH_2$ 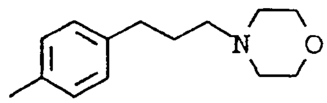 | 171-3°C |
| tert-butyl | $PhCH_2$ | $CH_2$ 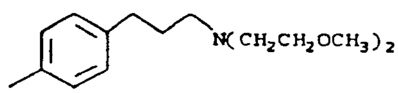 | 157-9°C |
| tert-butyl | $PhCH_2$ | $CH_2$ 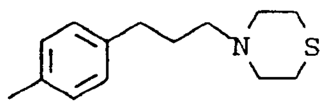 | 169-71°C |
| tert-butyl | $PhCH_2$ | $CH_2$ 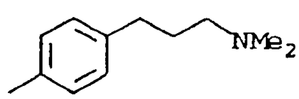 | 204-6°C |
| tert-butyl | 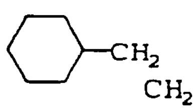 | $CH_2$ 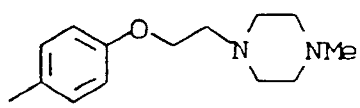 | 144-5°C |
| tert-butyl | 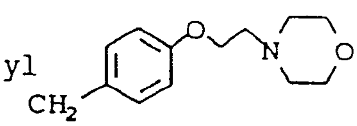 | 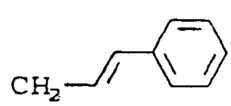 | 177-9°C |

TABLE V (CONT'D)

| 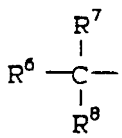 | $R^9_a$ | $R^9_a$ | mp |
|---|---|---|---|
| 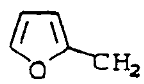 | $PhCH_2$ | 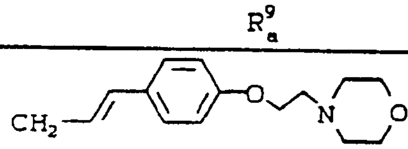 | 166-7°C |
| $CH_3OCH_2CH_2$ | $PhCH_2$ | 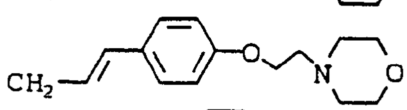 | 153-4°C |
| tert-butyl | 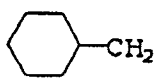 | 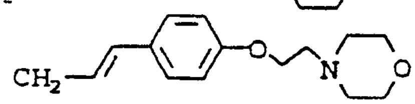 | 148-9°C |
| tert-butyl | 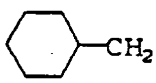 | 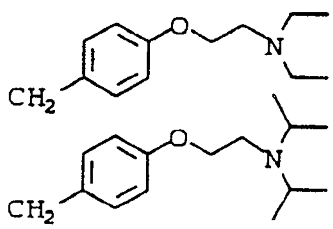 | 140-2°C |
| tert-butyl | 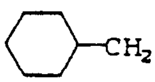 | | 92-4°C |
| tert-butyl | 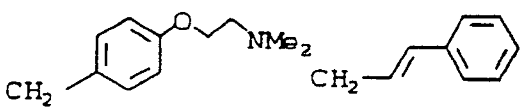 | | 194°C(dec) |

TABLE VI

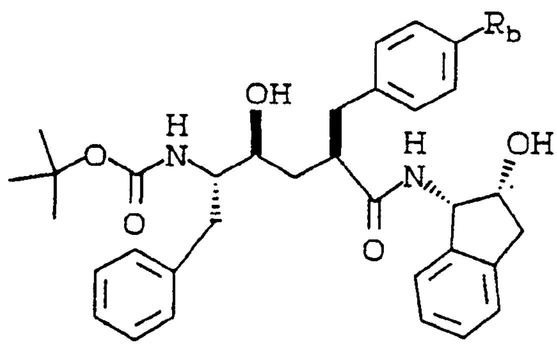

| $R_b$ | mp |
|---|---|
| 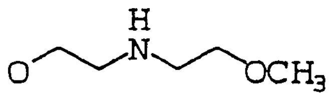 | 184-6°C |
| 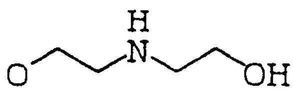 | 189-90°C |
| 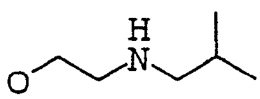 | 192-3°C |
| 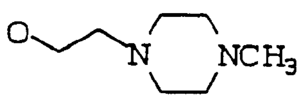 | 181-3°C |
| 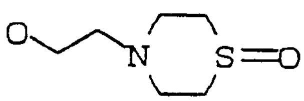 | 164-6°C |
| 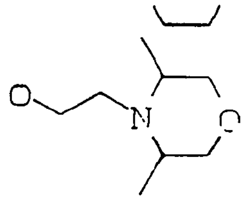 | 184-6°C |
| 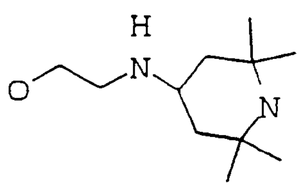 | 190-2°C |

TABLE VI (CONT'D)

| $R_b$ | mp |
|---|---|
| 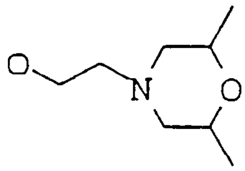 | 184-6°C |
| 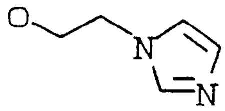 | 190-2°C |
| 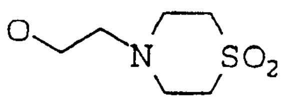 | 157-8°C |
| 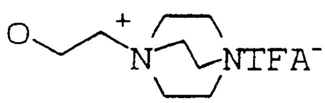 | 112-8°C |
| 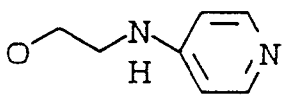 | amorphous |
| 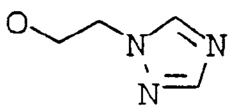 | 228-30°C |
| 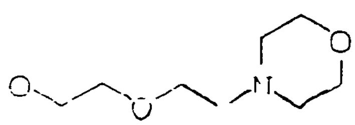 | |
| $OCH_2CH_2N(CH_2CH_2OH)_2$ | |

## TABLE VII

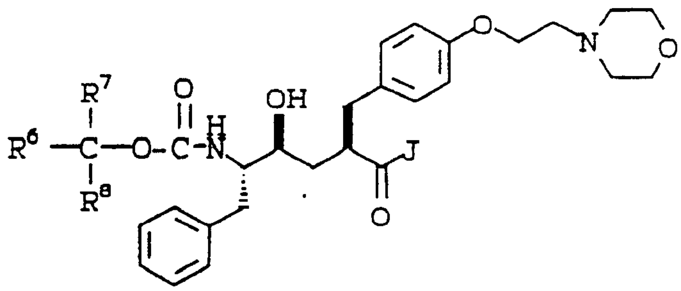

| 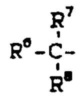 | J | | mp |
|---|---|---|---|
| tert-butyl | 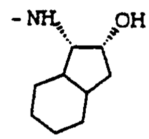 | | 148-51°C |
| tert-butyl | 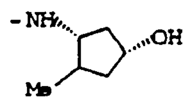 | α-Me<br>β-Me | 133-4°C<br>110-2°C |
| tert-butyl | 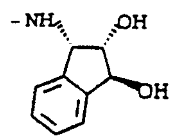 | | amorphous |
| $MeO(CH_2)_2$ | 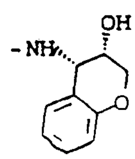 | | 198-201°C |
| $Me[O(CH_2)_2]_4$ | 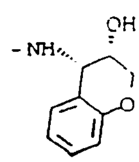 | | 142-4°C |

## TABLE VII (CONT'D)

| $R^7$<br>$R^6$-C-<br>$R^8$ | J | mp |
|---|---|---|
| tert-butyl | 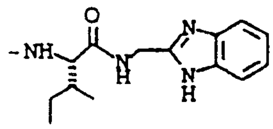 | 90-5°C |
| tert-butyl | 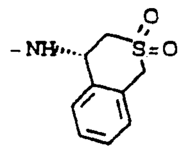 | 201-3°C |
| tert-butyl | 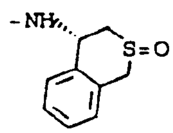 | 185-7°C |
| tert-butyl | 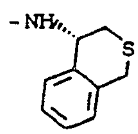 | 173-5°C |
| tert-butyl | 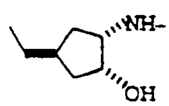 | |
| tert-butyl | 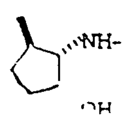 | |

TABLE VIII

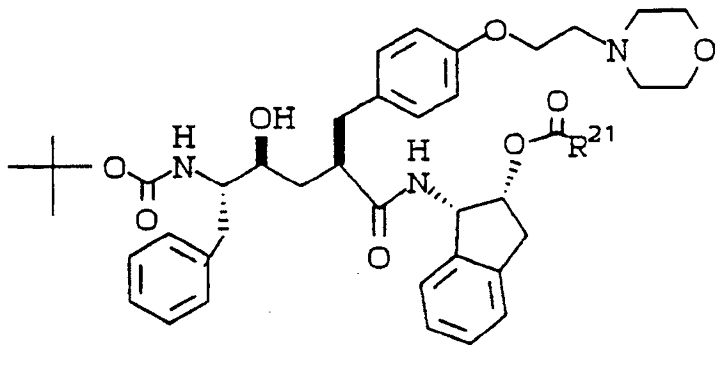

| $R^{21}$ | mp |
|---|---|
| 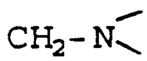 | 165-7°C |
| $CH_3$ | 194-5°C |
| 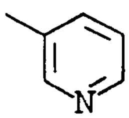 | 170-2°C |
| $(CH_2)_{14}CH_3$ | amorphous |
| 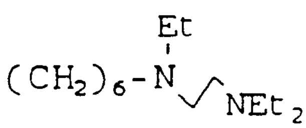 | amorphous |
| $CH_2CH_2CCO_2H$ | 149-50°C |

TABLE VIII (CONT'D)

| $R^{21}$ | mp |
|---|---|
| $[OCH_2CH_2]_3OCH_3$ | 101-4°C |
| 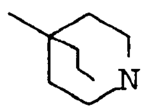 | 94-5°C |
| 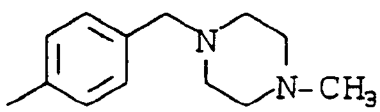 | 123°C |
| 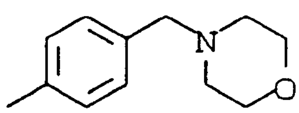 | 201-4°C |
| 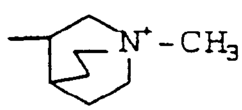 | 223-5°C |

## TABLE IX

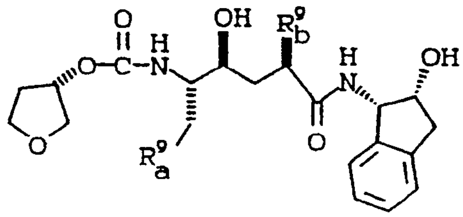

| $R^9_a$ | $R^9_b$ | mp |
|---|---|---|
| cyclo-$C_6H_{11}$ | 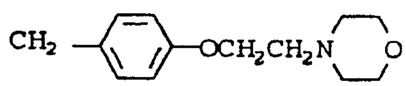 | 132-4°C |
| cyclo-$C_6H_{11}$ | 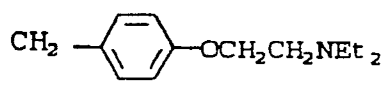 | 128-30°C |
| cyclo-$C_6H_{11}$ | 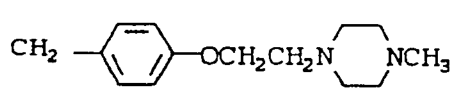 | 163-5°C |
| cyclo-$C_6H_{11}$ | 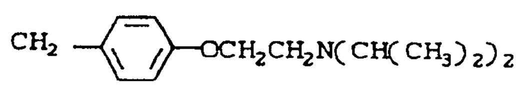 | 123-5°C |
| cyclo-$C_6H_{11}$ | 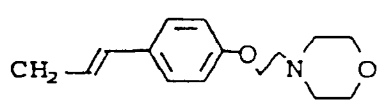 | 155-6°C |
| $C_6H_5$ | 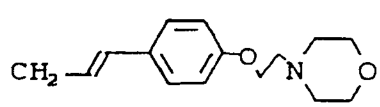 | 184-6°C |

The compounds of the present invention are useful in the inhibition of HIV protease, the prevention or treatment of infection by the human immunodeficiency virus (HIV) and the treatment of consequent pathological conditions such as AIDS. Treating AIDS or preventing or treating infection by HIV is defined as including, but not limited to, treating a wide range of states of HIV infection : AIDS, ARC (AIDS related complex), both symptomatic and asymtomatic, and actual or potential erposure to HIV. For example, the compounds of this invention are useful in treating infection by HIV after suspected past exposure to HIV by e.g., blood transfusion, accidental needle stick, or exposure to patient blood during surgery.

In the present invention, compounds with asymmetric centers may occur as racemates, racemic mixtures and as individual diastereomers, with all isomeric forms of the compounds being included in the present invention.

For the purposes, the compounds of the present invention may be administered orally, parenterally (including subcutaneous injections, intravenous, intramuscular, intrastemal injection or infusion techniques), by inhalation spray, or rectally, in dosage unit formulations containing conventional non-toxic pharmaceutically-acceptable carriers, adjuvants and vehicles.

Thus, in accordance with the present invention there is further provided a method of treating and a pharmaceutical composition for treating HIV infection and AIDS. The treatment involves administering to a patient in need of such treatment a pharmaceutical composition comprising a pharmaceutical carrier and a therapeutically-effective amount of a compound of the present invention, or a pharmaceutically-acceptable salt thereof.

These pharmaceutical compositions may be in the form of orally-administrable suspensions or tablets ; nasal sprays ; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweeteners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, flourocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquidify and/or dissolve in the rectal cavity to release the drug.

Dosage levels of the order of 0.02 to 5.0 or 10.0 grams-per-day are useful in the treatment or prevention of the above-indicated conditions, with oral doses two-to-five times higher. For example, infection by HIV is effectively treated by the administration of from 10 to 50 milligrams of the compound per kilogram of body weight from one to three times per day. It will be understood, however, that the specific dose level and frequency of dosage for any particular patient may be varied and will depend upon a variety of factors including the activity of the specific compound employed the metabolic stability and length of action of that compound, the age, body weight, general health sex, diet, mode and time of administration, rate of excretion, drug combination, the severity of the particular condition, and the host undergoing therapy.

The present invention is also directed to combinations of the DIV protease-inhibitory compounds with one or more agents useful in the treatment of AIDS.

For example, the compounds of this invention can be given in combination with the antivirals, immunomodulaters, antibiotics or vaccines or other derivative forms thereof as listed in the following Table [source : Marketletter, Nov. 30. 1987, pp. 26-27 ; Genetic Engineering News, Jan. 1988, Vol. 8, 23] :

TABLE[1]

A. Antivirals

| Drug Name | Manufacturer | Indication |
|---|---|---|
| AL-721 | Ethigen | ARC, PGL |
| BETASERON (interferon beta) | Triton Biosciences | AIDS, ARC, KS |
| CARRISYN (polymannoacetate) | Carrington Labs | ARC |
| CYTOVENE (ganciclovir) | Syntex | CMV |
| DDC (dideoxycytidine) | Hoffmann-La Roche | AIDS, ARC |

[1]Abbreviations: AIDS (Acquired Immune Deficiency Syndrome); ARC (AIDS related complex); CMV (Cytomegalovirus, which causes an opportunistic infection resulting in blindness or death in AIDS patients); HIV (Human Immunodeficiency Virus, previously known as LAV, HTLV-III or ARV); KS (Kaposi's sarcoma); PCP (Pneumonocystis carinii pneumonia, an opportunistic infection); PGL (persistent generalized lymphadenopathy).

| Drug Name | Manufacturer | Indication |
|---|---|---|
| FOSCARNET (trisodium phosphonoformate) | Astra AB | HIV inf, CMV retinitis |
| HPA-23 | Rhone-Poulenc Sante | HIV infection |
| ORNIDYL (eflornithine) | Merrell Dow | PCP |
| PEPTIDE T (octapeptide sequence) | Peninsula Labs | AIDS |
| RETICULOSE (nucleophospho-protein) | Advanced Viral Research | AIDS, ARC |
| RETROVIR advanced (zidovudine; AZT) | Burroughs Wellcome | AIDS, ARC pediatric AIDS, KS, asympt HIV, less severe HIV, neurological involvement. |

| Drug Name | Manufacturer | Indication |
|---|---|---|
| RIFABUTIN (ansamycin LM 427) | Adria Labs | ARC |
| (trimetrexate) | Warner-Lambert | PCP |
| UA001 | Ueno Fine Chem Industry | AIDS, ARC |
| VIRAZOLE (ribavirin) | Viratek/ICN | AIDS, ARC, KS |
| WELLFERON (alfa interferon) | Burroughs Wellcome | KS, HIV, in comb with RETROVIR |
| ZOVIRAX (acyclovir) | Burroughs Wellcome | AIDS, ARC, in comb with RETROVIR |

B. Immunomodulators

| Drug Name | Manufacturer | Indication |
|---|---|---|
| ABPP KS (bropirimine) | Upjohn | Advanced AIDS, |
| AMPLIGEN (mismatched RNA) | DuPont HEM Research | ARC, PGL |
| (Anti-human alpha interferon antibody) | Advanced Biotherapy Concepts | AIDS, ARC, KS |

| | | |
|---|---|---|
| Colony Stimulating Factor (GM-CSF) | Sandoz Genetics Institute | AIDS, ARC, HIV, KS |
| CL246,738 (CL246,738) | American Cynamid | AIDS |
| IMREG-1 | Imreg | AIDS, ARC, PGL, KS |
| IMREG-2 | Imreg | AIDS, ARC, PGL, KS |
| IMUTHIOL (diethyl dithio carbamate) | Merieux Institute | AIDS, ARC |
| Drug Name | Manufacturer | Indication |
| IL-2 (interleukin-2) | Cetus | AIDS, KS |
| IL-2 (interleukin-2) | Hoffmann-La Roche Immunex | AIDS, KS |
| INTRON-A (interferon alfa) | Schering-Plough | KS |
| ISOPRINOSINE (inosine pranobex) | Newport Pharmaceuticals | ARC, PGL, HIV seropositive patients |
| (methionine enkephalin) | TNI Pharmaceuticals | AIDS, ARC |

| | | |
|---|---|---|
| MTP-PE (muramyl-tripeptide) | Ciba-Geigy | KS |
| THYMOPENTIN (TP-5) (thymic compound) | Ortho Pharmaceuticals | HIV infection |
| ROFERON (interferon alfa) | Hoffmann-La Roche | KS |
| (recombinant erythropoietin) | Ortho Pharmaceuticals | severe anemia assoc with AIDS & RETROVIR therapy |
| TREXAN (naltrexone) | DuPont | AIDS, ARC |
| TNF (tumor necrosis factor) | Genentech | ARC, in combination interferon gamma |

C. Antibiotics

| Drug Name | Manufacturer | Indication |
|---|---|---|
| PENTAM 300 (pentamidine isethionate) | LyphoMed | PCP |

D. Vaccines

Any one of a variety of AIDS or HIV vaccines presently under study and development can be used in combination with the compounds of this invention or salt or derivative forms thereof, in the treatment or prevention of AIDS and diseases of similar character caused by HIV.

It will be understood that the scope of combinations of the compounds of this invention with AIDS antivirals, imunomodulators, antibiotics or vaccines is not limited to the list in the above Table, but includes in principle any combination with any pharmaceutical composition useful for the treatment of AIDS.

SYNTHESIS

The preparation and synthesis follows, in general, U.S. Patent 4,661,473 ; Evans, B.E. et al, J. Org. Chem., 50, 4615, (1985) and Evans, B.E. et al., "A Stereocontrolled Synthesis of Hydroxyethylene Dipeptide Isosteres," Proc. Am. pept. Symp., 9, 743-6(1985), and Luly, J.R. et al 1. J. Org. Chem, 52, 1487 (1987), all herein incorporated by reference. All temperatures are in degrees centigrade, unless indicated otherwise.

EXAMPLE 1

Preparation of N-(cis-2(R)-hydroxy-1(S)-indanyl)-5(S)-(1,1-dimethylethoxycarbonylamino)-4(S)-hydroxy-6-phenyl-2(R)-[(4-(2-(4-morpholinyl)ethoxy)phenyl) ethyl]-hexanamide.

Step A : Preparation of N-3(S)-[(1,1-Dimethylethoxycarbonyl)amino]-2(RS)-hydroxy-4-phenyl-1-trimethylsilyl butane :

To a stirred suspension of magnesium turnings (9.79 g, 403 mmol) in dry diethyl ether (200 mL) under nitrogen was added chloromethyltrimethylsilane (50 mL, 358 mmol). The reaction was initiated by gentle warming and then was cooled in an ice bath to maintain gentle reflux. After exotherm was complete the reaction was stirred at room temperature for 1 hour then cooled to -78°C in a dry ice/acetone bath. To the solution of the Grignard was added dropwise with stirring a solution of N-2(S)-[(1,1-dimethylethoxycarbonyl)amino]-3-phenyl propionaldehyde (19.3 g, 77.4 mmol) in dry diethyl ether (250 mL) dropwise such that the temperature of the reaction remained below -55°C. The resultant gray suspension was allowed to warm to room temperature where it was stirred for 30 minutes then was quenched by pouring into a mixture of ice (500 g) and 10% citric acid (500 mL). The organic phase was collected and the aqueous phase was extracted with diethyl ether (3 × 300 mL). The combined organics were washed with 10% citric acid (1 × 300 mL) and brine (1 × 200 mL), dried over anhydrous magnesium sulfate, filtered, and concentrated to give crude N-3(S)-[(1,1-dimethylethoxycarbonyl)amino]-2(RS)-hydroxy-4-phenyl-1-trimethylsilyl butane (26.6 g, quantitative crude yield) as a yellow oil. An analytical sample was obtained by low pressure chromatography (silica gel, 230-400 mesh ; diethyl ether: hexanes, 30% : 70%) followed by recrystallization from heptane. mp = 91-95°C ; elemental analysis. Calcd. for $C_{18}H_{31}NO_3Si$ (337.53) :

C = 64.05, H = 9.26, N = 4.15 ;
Found : C = 64.05, H = 9.13, N = 4.22 ; $[a]_D20$ = -40.0°.

Step B : Preparation of 3(S)-Amino-4-phenyl-1-butene.

To a stirred solution of the product of Step A (22.8 g, 67.5 mmol) in dry methylene chloride (400 mL) cooled in an ice bath and under nitrogen was added in a fine stream boron trifluoride etherate (43 mL, 345 mmol). The solution was allowed to warm to room temperature where it was stirred for 4 days. Reaction was cooled in an ice bath and quenched by the dropwise addition of 10% sodium hydroxide (400 mL). The organic phase was collected and the aqueous phase was extracted with methylene chloride (2 × 250 mL). The combined organics were washed with brine (1 × 200 mL), dried over anhydrous magnesium sulfate, filtered, and concentrated to give crude 3(S)-amino-4-phenyl-1-butene (14.2 g) as a yellow oil.

Step C : Preparation of N-3(S)-[(1,1-Dimethylethoxycarbonyl)amino]-4-phenyl-1-butene :

A solution of the product of Step B (14.2 g) and di-tert-butyl dicarbonate (31.0 g, 142 mmoL) in dry methylene chloride (200 mL) was stirred at room temperature for 18 hours, washed with 10% citric acid (3 × 100 mL), water (1 × 100 mL), sat'd. sodium bicarbonate (3 × 125 mL), and brine (1 × 250 mL), dried over anhydrous magnesium sulfate, filtered and concentrated to yield crude N-3(S)-1[(1,1-dimethylethoxycarbonyl)amino]-4-phenylbutene (34.6 g) as a yellow oil. Crude product was purified by low pressure chromatography (silica gel, 230-400 mesh, 10 × 20 cm column ; diethylether : hexanes, 20% : 80%) to yield N-3(S)-[(1,1-dimethylethoxylcarbonyl)amino]-4-phenyl-1-butene (16.3 g, 97.6% yield) as a white solid. An analytical sample was obtained by recrystallization from heptane. mp = 67.5 - 68.5°C ;
elemental analysis, Calcd. for $C_{15}H_{21}NO_2$ (247.34) :

C = 72.84, H = 8.56, N = 5.66.
Found : C = 72.78, H = 8.76, N = 5.64.

Step D : Preparation of 1(R)-[1'(S)-(1,1-Dimethylethoxycarbonyl)amino-2-phenylethyl]oxirane :

To a solution of the product of Step C (9.4 g, 38 mmol) in dry methylene chloride (100 mL) cooled in an ice bath and under nitrogen was added 3-chloroperoxybenzoic acid (technical grade, 80-85% ; 41 g, 200 mmol). The mixture was stirred at 0°C for 18 hours and 25°C for 23 hours, then diluted with diethyl ether (300 mL), and poured in ice cold aqeous 10% sodium sulfite (1 L). The organic layer was collected and the aqueous layer was extracted with diethyl ether (3 × 100 mL). The combined organics were washed with 10% sodium sulfite

(3 × 100 mL), satd. sodium bicarbonate (3 × 100 mL), and brine (1 × 100 mL), dried over anhydrous sodium sulfate, filtered and concentrated to give a white solid. Crude product was purified by low pressure chromatography (silica gel 230 - 400 mesh, 8 × 15 cm column ; ethyl acetate : hexanes, 25% : 75%) to yield 1(R)-[1'(S)-(1,1-dimethylethoxycarbonyl)amino-2-phenylethyl]oxirane (7.0 g, 70% yield) as a clear oil which crystallized upon standing. An analytical sample was obtained by recrystallization from heptane. mp = 51.5 - 52°C ; elemental analysis, Calcd. for $C_{15}H_{21}NO_2$ (263.34) :

C = 68.42, H = 8.04, N = 5.32.
Found : C = 68.22, H = 8.26, N = 5.29 ; $[a]_D^{20}$ = 1.34°.

Step E : Preparation of(5S,1'S)-3-carboethoxy-5-(1-((1',1'-dimethylethoxycarbonyl)amino)-2-phenylethyl)-dihydrofuran-2-(3H)-one.

The product from Step D, 9.93 g, was dissolved in 100 mL of absolute ethanol and added to a solution of 2.6 g of sodium and 20.1 mL of diethyl malonate in 170 mL of absolute ethanol. After stirring ovemite, the reaction was acidified to pH 4 with 10% citric acid and extracted with 2 × 500 mL of ether. The combined organic extracts were washed 1 × 500 mL $H_2O$, 1 × 500 mL sat'd $NaHCO_3$, 1 × 500 mL sat'd brine and dried over $MgSO_4$. The solvents were removed and the crude product purified by low pressure chromatography on silica gel eluting with 50% ether/hexanes (or EtOAc/hexanes). The yield of semi-solid product was 10.6 g. The later fractions contained 2.5 g of the undesired 5 R isomer as a white solid.

Step F : Preparation of (5S,1'S)-3-carboethoxy-3-(4-benzyloxyphenylmethyl)-5-[1-(1,1-dimethylethoxycarbonyl)amino)-2-phenylethyl] dihydrofuran-2-(3H)-one

To a stirred solution of (5S,1'S)-3-carboethoxy-5-[1-((1',1'-dimethylethoxycarbonyl)amino)-7-phenylethyl)-dihydrofuran-2-(3S)-one (product of Step E), 2 g (5.3 mmol) in 25 mL of absolute ethanol was added a solution of 0.13 g of sodium in 2.2 mL of absolute ethanol followed by 1.30 g (5.5 mmol) of 4-benzyloxybenzyl chloride. The solution was heated to 50°C under nitrogen for 1 hour, then cooled in an ice bath and acidified with 20 mL of 10% citric acid and diluted with 200 mL of water. The mixture was extracted with 3 × 100 mL of ether and the combined ether extracts washed with 50 mL of water, 200 mL of sat'd NaHCO3 and dried over $MgSO_4$. Removal of solvents under reduced pressure and purification by low pressure chromatography on silica gel, eluting with 40% ether in hexanes gave 1.56 g (51% yield) of a clear colorless glass essentially homogeneous by TLC (50% ether/hexanes).

Step G : Preparation of (3R,5S,1'S)-3-(4-benzyloxyphenylmethyl)-5-(1((1,1-dimethylethoxycarbonyl)amino)-2-phenylethyl)-dihydrofuran-2-(3H)-one.

The product of Step F, 13.6 g, was dissolved in 250 mL of 1,2-dimethoxyethane, and to it was added 117 mL of 1 M lithium hydroxide at room temperature. After stirring for 12 hours, the solvents were removed under reduced pressure, the residue suspended in 200 mL of 10% citric acid and extracted 3 × 500 mL of diethyl ether. The combined ether extracts were washed with 500 mL of brine, dried ($MgSO_4$) and the concentrated to dryness. The residue was dissolved in 250 mL of toluene, heated to reflux for 12 hours, then concentrated to dryness under reduced pressure. Purification by medium pressure chromatography over silica gel eluting with 15% ethyl acetate/hexanes gave 3.2 g of the 3R-lactone as a clear foam. Further elution with the same solvents gave 6.15 g of the 3S-lactone as a white solid.

Step H : Preparation of N'-(1,1-dimethylethoxycarbonyl)-5(S)-amino-4(S)-(1',1'-dimethylethyl-1,1-dimethylsilyloxy)-6-phenyl-2(R)-(4-benzyloxyphenylmethyl-hexanoic acid.

The product of Step G, 0.6 g, was dissolved in 30 mL of a 2 : 1 mixture of ethylene glycol dimethyl ether/water, and to it was added 5 mL of 1 M lithium hydroxide at room temperature. After stirring for 1 hour, the mixture was partitioned between 200 mL chloroform and 20 mL 10% citric acid. The layers were separated and the aqueous phase extracted with 3 × 20 mL chloroform. The combined organic layers were dried ($Na_2SO_4$) and the solvent removed to yield 0.56 g of the crude hydroxy acid. This residue was dissolved in 5 mL of dry

DMF and 0.845 g tert-butyl dimethylsilyl chloride and 0.725 g of imidazole were added. After stirring for 18 hours, the reaction was poured into 50 mL of water and extracted with 3 x 20 mL of ethyl acetate. The combined organic extracts were washed with 3 x 20 mL of 10% citric acid, 1 x 20 mL of water, 3 x 10 mL of saturated aqueous solution of $Na_2CO_3$, and 20 mL of brine. After drying ($Na_2SO_4$), the solvent was removed and the resulting residue dissolved in a mixture of 5 mL of THF, 5 mL of glacial acetic acid, and 2 mL of water. The mixture was stirred for 4 hours, then poured into 50 mL of water and extracted with 3 x 20 mL of ether. The combined ether extracts were washed with 2 x 20 mL of water, brine, dried ($Na_2SO_4$), and the solvent removed. Purification by medium pressure chromatography over silica gel, eluting with MeOH/$CHCl_3$ gave 0.60 g of the product as a white glassy solid.

Step I : Resolution of 1-Amino-2-hydroxyindan

From the known racemic 1-amino-2hydroxyindan, the resolution was carried out as described for the 3-amino-1,2-dihydroxyindan in Example 7 below (Steps D and E). The (1S,2R)-1-amino-2-hydroxyindan resulting from saponification of the higher $R_f$ diastereomer was shown to have an $a_D$ of -58° (c = 1.0, $CHCl_3$). The (1R, 2S)-1-amino-2-hydroxyindan resulting from saponification of the lower $R_f$ diastereomer was found to have an $a_D$ of +62° (c = 1.0, $CHCl_3$).

Step J : Preparation of N-(2(R)-hydroxy-1(S)-indanyl)-5(S)-(1,1-dimethylethoxycarbonylamino)-4(S)-hydroxy-6-phenyl-2(R)-(4-benzyl oxyphenylmethyl) hexanamide

The product from Step H, 0.12 g, was dissolved in 2 ml dry DMF and to it was added 40 mg of 1(S)-amino-2(R)-hydroxyindane, (Step I) 25 mg of 1-hydroxybenzotriazole hydrate and 70 mg of dimethyl-3-(3-dimethyl aminopropyl)carbodiimide hydrochloride. Triethylamine was added to the stirred solution until the pH was 8.5 (32 mL). After stirring for concentrated to dryness under reduced pressure, the residue was dissolved in 100 mL of chloroform and worked with 1 x 50 mL of 10% citric acid, 1 x 50 mL $H_2O$, 1 x 50 mL sat'd $NaHCO_3$, dried over $MgSO_4$ and concentrated to dryness. The residue was dissolved in 1 mL of tetrahydrofuran and added to 2 mL of 1 M tetrabutylammonium fluoride in THF. After stirring overnight at room temperature the reaction mixture was diluted with 10 mL of 10% citric acid and the white precipitate collected by filtration. The product was purified by low pressure chromatography on silica gel eluting with 2% methanol/$CH_2Cl_2$ to give 85 mg of product which was essentially homogeneous by TLC (3% methanol/$CH_2Cl_2$).

Step K : Preparation of N-(2(R)-hydroxy-1(S)-indanyl)-5(S)-(1,1-dimethylethoxycarbonylamino)-4(S)-hydroxy-6-phenyl-2(R)-(4-hydrox yphenylmethyl)hexanamide

The product of Step J, 85 mg was dissolved in 10 mL of methanol and 10 mL of THF, and to it was added 0.10 g of 10% palladium on carbon. The mixture was stirred under an atmosphere of hydrogen for 48 hours at room temperature, then filtered and concentrated to dryness. The residue was dissolved in 10 mL of hot ethanol and 20 mL water was added. On cooling the white solid precipitate was collected and dried under vacuum over $P_2O_5$. The yield was 72 mg (98% yield) of pure product : mp 218 - 219°C (effervesces, sinters at 215) elemental analysis, Calc'd for $C_{33}H_{40}N_2O_6$ : (560.696) :

| | |
|---|---|
| | C, 70.69 ; H, 7.19 ; N. 5.00. |
| found : | C, 70.62 ; H, 7.39 ; N. 4.79. |

Step L : Preparation of N-(cis-2(R)-hydroxy-1(S)-indanyl)-5(S)-[1,1-dimethylethoxycarbonylamino)-4(S)-hydroxy-6-phenyl-2(R)-[(4-(2-(4-morpholinyl)ethoxy)phenyl]methyl]hexanamide

A stirred mixture of Step K product, N-(2(R)-hydroxy-1(S)-indanyl)-5(S)-[1,1-dimethylethoxycarbonyl)amino]-4(S)-hydroxy-6-phenyl-2(R)-(4-hydroxyphenylmethyl) hexanamide (0.50 g, 0.9 mmol), anhydrous cesium carbonate (1.0 g, 3 mmol) and N-(2-chloroethyl) morpholine, free base (2.35 g, 17 mmole) in 100 mL of anhydrous dioxane was heated to 80°C (internal temperature) for 3 hrs. After cooling to room temperature the mixture was diluted with chloroform (50 mL) filtered, concentrated to dryness under reduced pressure, and the residue triturated with 50 ml of anhydrous ether and 10 mL of ethyl acetate. The white solid product was collected and dried under vacuum over $P_2O_5$. The yield was 0.54 g (89%) of pure product : mp 195 - 7°C. elemental analysis,

Calc'd. for $C_3H_{51}N_3O$ : $C_3H_{51}N_3O$ : (673.856) :

C, 69.52 H, 7.63 ; N, 6.23 ;
Found : C, 69.19 H, 7.45 ; N, 6.15.

maleate hydrate :
mp 112-113°C dec. elemental analysis.
Calc'd. for $C_{39}H_{51}N_3O_7$. $C_4H_4O_4.H_2O$ : (807.946) :

C, 63.92 H, 7.11 ; N, 5.20 ;
Found : C, 64.23 H, 6.94 ; N, 5.10.

EXAMPLE 2

Preparation of N-((cis)-2(R)-hydroxy-1(S)-indanyl) -5(S)-[2-hydroxyethoxycarbonyl)amino]-4(S)-hydroxy-6-phenyl-2(R)-[(4-(2-(4–morpholinyl)ethoxy)phenyl) methyl]hexanamide.

Step A : Preparation of [2-((1',1'-dimethylethyl-1,1-diphenylsilyloxy)-ethoxy]-p-nitrophenoxycarbamate.

To a solution of [2-((1',1'-dimethylethyl-1, 1-diphenylsilyloxy)-ethanol (544 mg) and N-methyl morpholine (202 mg) in 30 ml of $CH_2Cl_2$ was added 403 mg of p-nitrophenyl] chloroformate. After,18 hours at room temperature, the reaction was shaken with 30 ml of 10% citric acid and the organic layer was dried, filtered and concentrated to an oil. Hexane was added to precipitate p-nitrophenol and the filtrate was evaporated to give the product which was contaminated with about 30% of starting alcohol. This was used as is.

Step B : Preparation of N-((cis)-2(R)-hydroxy-1(S)indanyl)-5(S)-[2-((1',1'-dimethylethyl-1,1-diphenylsilyloxy)-ethoxycarbonyl)amino]-4 (S)-hydroxy-6-phenyl-2-(R)-[(4-(2-(4-morpholinyl)ethoxy)phenyl)methyl]-hexanamide

To a solution of 95 mg of N-((cis)-2(R)-hydroxy-1(S)-indanyl)-5(S)-[((1,1-dimethylethoxy)-carbonyl)amino]-4(S)-hydroxy-6-phenyl-2(R)-[(4-(2-(4-morpholiylethoxy)phenyl)methyl]-hexanamide in 6 ml of methylene chloride at 0°C was added 2 ml of trifluoroacetic acid. After 3 hours the reaction was evaporated to dryness and the crude residue dissolved in 3 ml of DMF and 0.2 ml of triethylamine. A solution of 150 mg of the compound from step A in 2 ml of DMF was added and the reaction stirred at room temperature for 18 hr after which time it was concentrated to dryness and purified (2 mm $SiO_2$ prep plate, 7% MeOH/$CHCl_3$) to afford 73 mg of pure product.

Step C : Preparation of N-((cis)-2(R)-hydroxy-1(S)-indanyl)-5(S)-[2-hydroxy)-ethoxycarbonyl) amino]-4(S)-hydroxy-6-phenyl-2(R)-[(4-(2-(4-morpholinyl)ethoxy)phenyl)methyl]-hexanamide.

The product from Step B above was dissolved in 4 ml of THF and 1 ml of a 1 M solution of tetrabutylammonium flouride was added. After 2 hours at room temp., the reaction was diluted with 15 ml of saturated $NaHCO_3$, extracted with ethyl acetate followed by methylene chloride and the organics were combined, dried filtered and concentrated to a yellow solid. The residue was chromatographed (2 mm $SiO_2$ prep plate, 5% MeOH/ammonia satd. $CHCl_3$) to afford 42 mg of product.
Analysis : Calcd for $C_{37}H_{47}N_3O_8 \cdot 0.5\ H_2O$

C, 66.24, H, 7.21, N, 6.26.
Found C, 66.17, H, 6.93, N, 6.18. mp = 195-197°C.

EXAMPLE 3

Preparation of N-((cis)-3(S)-hydroxy-4(S)-benzopyranyl)-N'-5(S)-[(1,1-dimethylethoxycarbonyl) amino]-4(S)-hydroxy-6-(4-(2-(4-morpholinyl)ethoxy) phenyl)2(R)-(3'-phenylprop-2'-en-1-yl)-hexanamide.

Step A : Preparation of (5S,1'S)-3-carboethoxy-5-(1((1',1'-dimethylethoxycarbonyl)amino-2-(4-acetoxyphenyl)-ethyl)dihydrofuran-2-(3

H)-one

To 4 g of (5S,1'S)-3-carboethoxy-5-(1-((1',1'-dimethylethoxycarbonyl)amino-2-(4-benzylo xyphenyl)-ethyl)dihydrofuran-2-(3H)-one dissolved in 30 ml of methanol and 70 ml of ethyl acetate was added 3.5 g of 10% Pd/C. The reaction was first flushed with N2 followed by $H_2$ (balloon, 1 atm) and stirred at room temperature. After 18 hours the reaction was flushed with $N_2$, filtered through celite and the filtrate evaporated to dryness to give a foam. The crude product was dissolved in 75 ml of methylene chloride and 1.25 g of triethylamine and 1.26 g of acetic anhydride were added. After 3 days at room temperature, the reaction was washed with 50 ml of 10% citric acid solution, water, dried, filtered and concentrated to give an oil. The crude compound was chromatographed ($SiO_2$, 30% EtOAc/Hexane) to give 2.4 g of acetate.

Step B : Preparation of (3S. 5S. 1'S)-3-(3'phenylprop-2'-en-1-yl)-5-(1-((1',1'-dimethylethoxycarbonyl)amino-2-(4-hydroxyphenyl)-ethyl-dihydrofuran-2-(3H)-one

To 132 mg of oil free sodium hydride in 100 ml of THF at 0°C was added 2.4 g of (5S,1'S)-3-carboethoxy-5-(1-((1',1'-dimethylethoxycarbonyl)amino-2-(4-acetoxyphenyl)-ethyl)dihydrofuran-2-(3H)-one in 25 ml of THF. After stirring for 1 hour, solid cinnamoyl bromide (1.09 g) was added in one portion and the reaction was warmed to room temperature. After 2 hr, the reaction was diluted with 20 ml of a 1M lithium hydroxide solution and stirred overnight. After 18 hr the reaction was concentrated to dryness, diluted with 150 ml of water, made acidic with an excess of 10% citric acid solution, washed with ether which was dried, filtered and concentrated to a foam. The foam was dissolved in 200 ml of toluene and heated at 120°C. After 18 hour, the reaction was evaporated to dryness and chromatographed on $SiO_2$ (40% EtOAc/Hexane) to afford 0.45 g of pure product along with 0.65 g of the opposite 3(R) diastereomer.

Step C : Preparation of (3S,5S,1'S)-3-(3'-phenylprop-2'-en-1-yl)-5-(1-((1',1'-dimethylethoxycarbonyl)amino-2-(4-(2-(4-morpholinyl)ethoxy)phenyl)-ethyl)dihydrofuran-2-(3H)-one.

To 370 mg of (3S,5S,1'S)-3-(3'-phenylprop-2'-en-1-yl)-5-(1',1'-dimethylethoxycarbonyl) amino-2-(4-hydroxyphenyl)-ethyl)dihydrofuran-2-(3H)-one in 100 ml of acetone was added 442 mg of potassium carbonate, 300 mg of 2-chloroethyl morpholine and 100 mg of sodium iodide. The reaction was heated at 60°C for 4 days after which time it was cooled, filtered and the filtrate concentrated to dryness. The residue was dissolved in 100 ml of water and washed with ethyl acetate which was dried, filtered and concentrated to give a foam. The residue was chromatographed on $SiO_2$ (10%NelG/$CHCl_3$) to afford 250 mg of pure product.

Step D : Preparation of N'-(1,1-dimethylethoxycarbonyl)-5(S)-amino-4(S)-(1',1'-dimethylethyl-1,1-dimethylsilyloxy)-6-(4-(2-(4-morpholinyl)ethoxy)phenyl)-2(R)-(3'phenylprop-2-en-1-yl)-hexanoiciacid.

To a solution of 250 mg of (3S,5S,1'S)-3-(3'-phenylprop-2'-en-1-yl)-5-(1-((1',1'-dimethyl ethoxycarbonyl)amino-2-(4-(2-(4-morpholinyl)ethoxy)phenyl)ethyl)dihydrofuran-2-(3H)-one in 3 ml of DME was added 0.7 ml of a 1M solution of lithium hydroxide. After 2 hours at room temperature, the reaction was concentrated to dryness and diluted with saturated ammonium chloride solution. This was washed with ethyl acetate which was dried, filtered and concentrated to afford an oil.

The above oil was dissolved in 15 ml of DMF and 306 mg of imidazole and 340 mg of t-butyldimethylsilyl chloride were added. After 2 days at room temperature, the reaction was concentrated to dryness and diluted with satd. $NH_4Cl$ solution. The aqueous layer was washed with ethyl acetate, and the organic layer was dried, filtered and concentrated. The residue was chromatographed ($SiO_2$, 10% MeOH/$CHCl_3$) to give 250 mg of product as a foam.

Step E : Preparation of N-((cis)-3(S)-hydroxy-4(S)-benzopyranyl)-N'-5(S)-[(1,1-dimethylethoxycarbonyl)amino')-4(S)-(1',1'-dimethylethyl-1,1-dimethylsilyloxy)-6-(4-(2-(4-morpholinyl)ethoxy)phenyl)2(R)-(3-phenylprop-2-en-1-yl)-hexanamidexy)phenyl)2(R)-(3-phenylprog-2-en-1-yl)-hexanamide.

To a solution of 95 mg of N'-(1,1-dimethylethoxycarbonyl)-5(S)-amino-4(S)-(1',1'-dimethylethyl-1,1-dimethylsilyloxy)-6-(4-(2-(4-morpholinylethoxy)phenyl)-2(R)-(3'-phenylprop-2' en-1-yl)-hexanoic acid in 5 ml of

DMF at 0°C was added 29 mg of 1-ethyl-3-(3-dimethylaminopropyl)carbodiimide hydrochloride, 20 mg of hydroxybenztriazole hydrate and 30 mg of 3(S)-hydroxy-4(S)-amino-benzopyran. The pH of the solution was adjusted to 8.5 with triethylamine and the reaction stirred at room temperature. After 36 hours, the reaction was concentrated to dryness and the residue diluted with 40 ml of water. The aqueous layer was washed with ethyl acetate which was then dried, filtered and concentrated to an oil which was chromatographed on a 2 mm prep plate (50/50 EtOAc/Hexane) to afford 74 mg of product.

Step F : Preparation of N-((cis)-3(S)-hydroxy-4(S)-benzopyranyl)-N'-5(S)-[(1,1-dimethylethoxycarbonyl)amino]-4(S)-hydroxy-6-(4-(2-(4-morpholinyl)ethoxy)phenyl)-2(R)-(3-phenylprop-2-en-yl)-hexanamide.

The product from Step E (N-((cis)-3(S)-hydroxy-4(S)-benzopyranyl)-N'-5(S)-'((1,1-dimethylethoxycarbonyl)amino')-4(S)-(1',1'-dimethylethyl-1,1-dimethylsilyloxy)-6-(4-(2-(4-morpholinyl)ethoxy)phenyl)-2(R)-(3'p henylprop-2'en-1-yl)-hexanamide, 75 mg) was dissolved in 3 ml of THF and 1 ml of a 1 M solution of tetrabutylammonium fluoride was added. After 18 hours at room temperature, the reaction was diluted with water which was washed with ethyl acetate. The organic layer was dried, filtered and concentrated to a crude solid which was chromatographed ($SiO_2$, 2 mm prep plate, 10% MeOH/$CHCl_3$) to afford 32 mg of product. A maleate salt was made in the usual manner.
mp 168 - 170°C. Analysis : calcd. for $C_{41}H_{53}N_3O_8.C_4H_4O_4$ (831.96)

| | |
|---|---|
| | C, 64.96 H, 6.91 N, 5.05 |
| Found : | C,64.99, H, 6.94, N, 4.91. |

EXAMPLE 4

The preparation of N-(2-benzimidazolylmethyl)N'-5(S)-[(1,1-dimethylethoxycarbonyl)amino]-4(S)-hydroxy-6-(4-(2-(4-morpholinyl) ethoxy)phenyl)-2(R)-(3'phenylprop-2'en-1-yl)-hexanoyl isoleucyl amide.

Step A : The preparation of N-(2-benzimidazolylmethyl)-N'-5(S)-[(1,1-dimethylethoxycarbonyl) amino]-4(S)-1',1'-dimethylethyl-1,1-dimethylsilyloxy)-6-(4-(2-(4-morpholinylethoxy)-phenyl)-2(R)-(3'phenylprop -2'en-1-yl)-hexanoyl isoleucylamide.

As in the same manner as described for Example 3, Step E, 115 mg of N'-(1,1-dimethylethoxycarbonyl)-5(S)-amino-4(S)-(1',1'-dimethylethyl-1,1-dimethylsilyloxy)-6-(4-(2-(4-morpholinyl)ethoxy)phenyl)-2(R)-(3-ph enylprop-2-en-1-yl)-hexenoic acid was coupled with N-(2-benzimadazolylmethyl)-isoleucyl amide to afford 82 mg of pure product ($SiO_2$, 10% MeOH/$CHCl_3$) as a foam.

Step B : The preparation of N-(2-benzimidazolylmethyl)-N'-5(S)-[(-1,1-dimethylethoxycarbonyl)amino]-4(S)-hydroxy-6-(4-(2-(4-morpholiny l)ethoxy)phenyl)-2(R)-(3-phenylprop-2-en-1-yl-hexanoyl isoleucyl amide.

To 82 mg of the product of Step A in 4 ml of THF was added a 1M soln of tetrabutylammonium fluoride in THF. After 18 hr at room temperature, the reaction was diluted with 20 ml of satd. $NaHCO_3$ soln. and washed with ethyl acetate. The organic layer was dried, filtered and concentrated to afford an oil which was chromatographed ($SiO_2$, 2 mm prep plate, 10% MeOH/$NH_3$ satd $CHCl_3$) to afford 55 mg of product. A maleate salt was generated in ethyl acetate/ether to give 48 mg of a white solid. mp 105-110°C. Anal. Calcd. for $C_{46}H_{62}N_6O_7.C_4H_4O_4.2H_2O$ (963.15)

| | |
|---|---|
| | C, 62.35 H, 7.33 N, 8.73 |
| Found : | C, 62.20 H, 7.09 N, 8.44 |

EXAMPLE 5

The preparation of N-(2-benzimidazolylmethyl)-N'-5(S)-[(1,1-dimethylethoxycarbonyl)amino]-4(S)-hydroxy-6-phenyl-2(R)-[(4-(2-(4 -morpholinyl)-ethoxy)-phenyl)]hexanoyl isoleucyl amide.

Step A : Preparation of (3S,5S,1'S)-3-(4-hydroxyphenyl)-5-(1-((1',1'-dimethylethoxycarbonyl)amino-2-phenyl-ethyl)dihydrofuran-2-(3H)-one

To 250 mg of (5S,1'S)-3-(4-benzyloxyphenyl)-5-(1-((1',1'-dimethylethoxycarbonyl)amino-2-phenylethyl)dihydrofuran-2-(3H)-one dissolved in 10 ml of methanol and 20 ml of ethyl acetate was added 200 mg of 10% Pd/C. The reaction was first flushed with $N_2$ followed by $H_2$ (balloon, 1 atm) and stirred at room temperature. After 2 hours the reaction was flushed with $N_2$, filtered through celite and the filtrate evaporated to dryness to give a foam.

Step B : Preparation of (3S,5S,1'S)-3-((4-(2-(4-morpholinyl)ethoxy)phenyl)methyl)-5-(1-((1',1'-dimethylethoxycarbonyl)amino-2-phenyl)-ethyl)dihydrofuran-2-(3H)-one

To 385 mg of (3S,5S,1'S)-3-((4-hydroxyphenyl) methyl)-5-(1-((1'-dimethylethoxycarbonyl)amino))-2-(phenyl)-ethyl)dihydrofuran-2-(3H)-one in 60 ml of acetone and 20 ml of DME was added 600 mg of potassium carbonate, 500 mg of 2-chloroethyl morpholine and 130 mg of sodium iodide. The reaction was heated at 60°C for 18 hour after which time it was cooled, filtered and the filtrate concentrated to dryness. The residue was dissolved in 100 ml of satd. $NH_4Cl$ soln. and washed with ethyl acetate which was dried, filtered and concentrated to give a foam. The residue was chromatographed on $SiO_2$ (4 : 1 EtOAc/hexane followed by 5%MeOH/$CHCl_3$) to afford 420 mg of pure product as a 3 : 1 ratio of 3(S) to 3(R) epimers.

Step C : Preparation of N'-(1,1-dimethylethoxycarbonyl)-5(S)-amino-4(S)-(1',1'-dimethylethyl-1,1-dimethylsilyloxy)-6-phenyl-2(R)-[(4-(2-4-morpholinyl)ethoxy)phenyl)methyl]-hexanoic acid

To a solution of 420 mg of (3S,5S,1'S)-3-((4-(2-(4-morpholinylethoxy)phenyl)methyl)-5-(1-((1',1'-dimethylethoxycarbonyl)amino-2-phenyl)ethyl)-dihydrofuran-2-(3H)-one in 10 ml of DME was added 3 ml of a 1M solution of lithium hydroxide. After 2 hours at room temperature, the reaction was concentrated to dryness and diluted with saturated reaction was concentrated to dryness and diluted with saturated ammonium chloride solution. This was washed with ethyl acetate which was dried, filtered and concentrated to afford an oil.

The above oil was dissolved in 20 ml of DMF and 836 mg of imidazole and 900 mg of t-butyldimethylsilyl chloride were added. After 4 days at room temperature, the reaction was concentrated to dryness and diluted with satd. $NH_4Cl$ solution. The aqueous layer was washed with ethyl acetate, and the organic layer was dried, filtered and concentrated. The residue was chromatographed ($SiO_2$, 10% MeOH/$CHCl_3$) to give 420 mg of product as a foam.

Step D : The preparation of N-(2-benzimidazolylmethyl)-N'-5(S)-[(1,1-dimethylethoxy carbonyl)-amino]-4(S)-(1',1'-dimethylethyl-1,1-dimethylsilyoxy)-6-phenyl-2(R)-[(4-(2-(4-morpholinyl)ethoxy)phenyl)]hexanoyl isoleucyl amide.

As in the same manner as described for Example 3, Step E, 150 mg of N'-(1,1-dimethylethoxycarbonyl)-5(S)-amino-4(S)-(1',1'-dimethylethyl-1,1-dimethylsilyloxy)-6-phenyl-2(R)-[(4-(2-(4-morpholinyl)ethoxy)phenyl)methyl]hexanoic acid was coupled with N-(2-benzimadazolylmethyl)-isoleucyl amide to afford 196 mg of pure product after chromatography ($SiO_2$, 10% MeOH/ammonia satd. $CHCl_3$) as a foam. The IH NMR indicates that the product is a 4 : 1 mixture of 2(R)/2(S) epimers.

Step E : The preparation of N-(2-benzimidazolylmethyl)-N'-5(S)-[(1,1-dimethylethoxycarbonyl) amino]-4(S)hydroxy-6-phenyl-2(R)-[(4-(2-(4-morpholinylethoxy)phenyl)]-hexanoyl isoleucyl amide.

To 190 mg of N-(2-benzimidazolylmethyl)-N'-5(S)-[(1,1-dimethylethoxycarbonyl)amino]-4(S)-(1',1'-dimethylethyl-1,1-dimethylsilyoxy)-6-phenyl-2(R)-[(4-(2-(4-morpholinylethoxy)phenyl)]-hesanoyl isoleucyl amide in 4 ml of THF was added a 1 M solution of tetrabutylammonium fluoride in THF. After 18 hr at room temperature, the reaction was diluted with 20 ml of satd. $NaHCO_3$ solution. and washed with ethyl acetate. The organic layer was dried, filtered and concentrated to afford 150 mg of an oil which was chromatographed ($SiO_2$, 2 mm prep plate, 10% MeOH/$NH_3$ satd $CHCl_3$) to afford 120 mg of a product which was still a mixture. The compound was rechromatographed on reverse phase HPLC (porasil, ubonda, 95/5 1% TFA/water : $CH_3CN$-95/5 $CH_3CN$ : 1% TFA/water, 22 min gradient, 15 ml/min) and the fractions containing the product were lyopholized to afford 44

mg of a bis-TFA salt. mp 119-122°C. Anal. Calcd. for $C_{44}H_{61}N_6O_7.2CF_3COOH$ MW = 1014.032.

| | |
|---|---|
| | C, 62.35 H, 7.33 N, 8.73 |
| Found : | C, 62.20 H, 7.09 N, 8.44 |

EXAMPLE 6

Preparation of N-(2(R)-hydroxy-1(S)-indanyl)-5(S)-[(1,1-dimethylethoxycarbonyl)amino]-4(S)-hydroxy-6-phenyl-2(R)-[3-(2-(4-morpholinyl)ethoxy)phenyl]methyl hexanamide

Step A : Preparation of (3R,5S,1')-3-(3-benzyloxyphenylmethyl)-5-[1-((1,1-dimethylethoxycarbonyl)amino)-2-phenylethyl]-dihydrofuran-2-(3H)-one

Under an argon atmosphere 0.565 g of a 60% dispersion of sodium hydride in mineral oil (14.0 mmol of NaH) was washed twice with hexane to remove the oil. The remaining grey powder was suspended in 50 mL of freshly distilled THF and to this stirred suspension was added a solution containing 3.45 g (9.1 mmol) of (5S,1'S)-3-carboethoxy-5-[1-((1,1-dimethylethoxycarbonyl)amino-2-phenylethyl)dihydrofuran-2-(3H)-one (product of Example 1, Step E) dissolved in 75 mL of dry THF. After 30 minutes at room temperature the suspension was heated to 50° and 3.2 g (11.5 mmol) of 3-benzyloxybenzyl bromide was added over 2 minutes. After 2 hours at 50° the solution was cooled in an ice bath and treated with 75 mL of a 1.0 N lithium hydroxide solution. The resulting solution was stirred at room temperature for 4 hours, after which time the THF was evaporated in vacuo. The residue was taken up in 300 mL of water and extracted with 2 × 75 mL of EtOAc. 1 × 75 mL of ether. The alkaline aqueous phase was chilled in an ice bath and acidified to pH 4 with 10% citric acid. The acidic suspension was then extracted with 3 × 100 mL of EtOAc and the combined acidic organic layers were washed with water, brine, and dried over $MgSO_4$. Removal of the solvent in vacuo left an oil which was dissolved in 400 mL of toluene. This solution was stirred at reflux under argon for 18 hours, after which the toluene was removed under reduced pressure. The oil which remained was purified by flash chromatography on silica gel, eluting with 20% EtOAc in hexane. The first fractions contained 1.3 g of the pure 3(R)-lactone. Continued elution provided 1.1 g of a mixture of 3(R)- and 3(S)-lactones, followed by 0.8 g of pure 3(S)-lactone.

Step B : Preparation of N'-(1,1-Dimethylethoxycarbonyl)5(S)-amino-4(S)-(1',1'-dimethylethyl-1,1-dimethylsilyloxy)-6-phenyl-2(R)-(3-benzyloxyphenylmethyl)hexanoic acid.

The product of Step A (1,28 g. 2.55 mmol) was dissolved in 35 mL of dioxane and stirred at room temperature under argon ; 18 mL of 1.0 N lithium hydroxide was added and the resulting solution was stirred for 4 hours. The organic solvent was removed under pressure and the residue was treated with 250 mL of water and 100mL of EtOAc. This mixture was stirred in an ice bath as 10% citric acid was added to a pH of 4.0, after which the layers were separated and the aqueous phase was extracted with 2 x 100 mL of ether. The combined organic layers were washed with brine, dried over $Na_2SO_4$, and concentrated in vacuo to a glass. This material was dissolved in 12 mL of dry DMF, the solution treated with 2.45 g (36.0 mmol) of imidazole, followed by 2.70 g (17.9 mmol) of tert-butyl dimethylsilyl chloride and the reaction mixture was stirred overnight at 25° under argon. The reaction was quenched by the addition of 14 mL of methanol ; after stirring at 25° for 3 hr the solution was concentrated to dryness in vacuo. The residue was treated with 100 mL of ice water and acidified with 10% aqueous citric acid to pH 4. The mixture was then extracted with 3 x 75 mL ether and the combined ethers were washed successively with water and brine. Upon drying ($Na_2SO_4$), the solvent was removed under reduced pressure to leave 1.5 g of a foam which was purified by flash chromatography : elution with 15% EtOAc in $CHCl_3$ until the product began to appear, then with 100% EtOAc. Thus was obtained 1.14 g of pure N'-(1,1-dimethylethoxycarbonyl)-5(S)-amino-4(S)-(1',1'-dimethylethyl-1,1-dimethylsilyloxy)-6-phenyl-2(R)-(3-benzyl oxyphenylmethyl)hexanoic acid.

Step C : Preparation of N-(2(R)-hydroxy-1(S)-indanyl)S)-(1,1-dimethylethoxycarbonylamino-4(S)-hydroxy-6-phenyl-2(R)-(3-hydroxyphenylmethyl)hexanamide.

The product from Step B, 600 mg (0.95 mmol), was dissolved in 7 mL dry DMF and to the solution were

added 213 mg of 1(S)-amino-2(R)-hydroxyindane, 135 mg of 1-hydroxybenzotriazole hydrate and 192 mg of 1-(3-dimethylaminopropyl)-3-ethylcarbodiimide hydrochloride. Triethylamine was added to the stirred solution until the pH was 8.5. After stirring under argon at 25° for 18 hours the solution was concentrated to dryness under reduced pressure and the residue was dissolved in EtOAc. The EtOAc solution was washed with 10% citric acid, water, sat'd $NaHCO_3$, brine, and dried ($Na_2SO_4$) ; evaporation of the solvent left an oily residue (690 mg). Flash chromatography of this oil on silica gel, eluting with 15% EtOAc in hexane, afforded 116 mg of a compound resulting from racemization at the carbon during the coupling reaction : the 2(S)-(3-hydroxyphenylmethyl isomer. Continuing the elution with 30% EtOAc in hexane gave 466 mg of the desired 2(R-)isomer. This latter compound was dissolved in 4 mL of dry THF and to the solution was added 2.0 mL of 1M tetrabutylammonium fluoride in THF. After stirring ovenight at room temperature the reaction mixture was concentrated to dryness and the residue was diluted with a small amount of crushed ice and 10 mL of 10% citric acid. The product, a white solid, was collected by suction filtration and washed with cold water. The yield of N-(2(R)-hydroxy-1(S)-indanyl)-5(S)-(1,1-dimethylethoxycarbonylamino-4(S)-hydroxy-6-phenyl-2(R)-(3-benzyloxyphenylmethyl)hexanamide was 450 mg.

Step D : Preparation of N-(2(R)-hydroxy-1(S)-indanyl)-(S)-1,1-dimethlethoxycarbonylamino-4(S)-hydroxy-6-phenyl-2(R)-(3-hydroxyphenylmethyl)-hexanamide

The product from Step C (450 mg) was dissolved in 50 mL each of methanol and EtOAc and to this solution was added 130 mg of 10% palladium on carbon. The mixture was stirred under an atmosphere of hydrogen for 24 hours at room temperature, filtered and concentrated to dryness in vacuo, leaving 320 mg of the product as a white solid. An analytical sample was recrystallized from acetonitrile.

Calc'd for $C_{33}H_{40}N_2O_6$ (560.70) :

| | |
|---|---|
| | C, 70.69 ; H, 7.19 ; N, 5.00 ; |
| Found : | C, 70.46 ; H, 7.16 ; N, 5.02. |

Step E : Preparation of N-(2(R)-hydroxy-1(S)-indanyl)-(S)-[(1,1-dimethylethoxycarbonyl)amino']-4(S)-hydroxy-6-phenyl-2(R)-[3-(2-(4-morpholinyl)ethoxy)phenyl]methylhexanamide

A mixture of the product from Step D (193 mg, 0.34 mmol), 1.42 g (9.5 mmol) of 4-(2-chloroethyl)-morpholine, and 1.11 g (3.4 mmol) of cesium carbonate in 60 mL of dry 1,4-dioxane was stirred under an atmosphere of argon at 80° for 90 minutes. The mixture was cooled to room temperature, filtered, and the filtrate concentrated to dryness in vacuo. The sticky solid which remained was triturated in 3 drops each of acetonitrile and ethyl ether. Filtration of the suspension afforded 116 mg of the title compound as a white solid, mp 176 - 180° dec.

Calc'd for $C_{39}H_{51}N_3O_7.O.75H_2O$ (687.37) :

| | |
|---|---|
| | C, 68.14 ; H, 7.70 ; N, 6.11 ; |
| Found : | C, 68.31 ; H, 7.58 ; N, 6.00. |

EXAMPLE 7

Preparation of N-((1R,2S,3S)-1,2-Dihydroxy-3-indanyl)-N'-(1,1-dimethylethoxycarbonyl)-5(S)-amino-4(S)-hydroxy-6-phenyl-2(R)-(3-(2-(4-morpholinyl)-ethoxy)phenylmethyl)hexanamide

Step A : (1RS,2RS,3RS)3-Amino-1-bromo-2-hydroxyindan cyclic carbamate

A mixture of 2.5 g (14.3 mmol) of the known 1-amino-2-hydroxyindan cyclic carbamate, 2.9 g (16.3 mmol) of N-bromosuccinimide, and 100 mL of $CCl_4$ was heated at reflux for 3 hours. After cooling, the mixture was diluted with 100 mL of $CHCl_3$ and washed with 10% $Na_2SO_3$. The aqueous layer was extracted with $CHCl_3$, and the combined organic layers were washed with 10% $Na_2SO_3$, dried over $Na_2SO_4$, and the solvents removed at reduced pressure to give an oil which was chromatographed on 250 g of fine $SiO_2$ using 1 : 1 EtOAc-hexanes to afford 950 mg (26%) of the Step A product as a colorless solid.

Step B : (1RS,2SR,3SR)1-Acetoxy-3-amino-2-hydroxyindancyclic carbamate

A solution of 950 mg (3.7 mmol) of 1-Bromo-2-hydroxy-3-aminoindan cyclic carbamate (Step A product) and 1.9 g (6.3 mmol) of tetra-n-butylammonium acetate in 50 mL of DMF was heated at 80°C under $N_2$ for 6 hours. The solvents were removed at reduced pressure and the residue was partitioned between $CHCl_3$ and water. The aqueous layer was extracted twice with $CHCl_3$ and the combined organic layers were washed with water, dried over $Na_2SO_4$ and the solvents removed to give an oil which was chromatographed on 125 g fine $SiO_2$ using EtOAc-hexanes to afford 372 mg (43%) of Step B product as a colorless solid : mp 131-133°C, Anal;

Calc'd for : $C_{12}H_{11}NO_4$ C, 61.80 ; H, 4.75 ; N, 6.01.

Found : C, 62.12 ; H, 4.73 ; N, 5.96.

Step C : (1RS.2SR.3SR)3-Amino-1.2-dihydroxyindan

To a stirred solution of 180 mg (0.77 mmol) of 1-acetoxy-2-hydroxy-3-aminoindan cyclic carbamate (Step B product) in 12 mL of EtOH was added a solution of 705 mg (17.6 mmol) of NaOH in 5 mL of $H_2O$. This mixture was heated at reflux for 3 hours, the solvents removed at reduced pressure, and the residue partitioned between brine and $CHCl_3$, and the combined organic layers were dried over $Na_2SO_4$. The solvents were removed to give 60 mg (47%) of Step C product as a solid.

Step D : (1R,2S,3S)-N-1,2-dihydroxy-3-indanyl)-L-phenylalanine amide

To a stirred solution of 422 mg (2.55 mmol) of 1,2-dihydroxy-3-aminoindan (Step C product), 746 mg (2.81 mmol) of Boc-L-Phenylalanine, 539 mg (2.81 mmol) of EDC, and 380 mg (2.81 mmol) of 1-hydroxybenzotriazole hydrate in 10 mL of DMF under $N_2$ was added 783 µL (5.62 mmol) of $Et_3N$. After stirring overnight at room temperature, the solvents were removed at reduced pressure, and the residue was partitioned between $CHCl_3$ and 1.0 M citric acid. The aqueous layer was extracted with $CHCl_3$ and the combined organic layers were washed with two portions of $Na_2CO_3$ and dried over $Na_2SO_4$. The solvents were removed at reduced pressure and the residue was dissolved in 14 mL of $CH_2Cl_2$ and treated with 7 mL of TFA under $N_2$ for 0.5 hours. The solvents were removed at reduced pressure, and the residue was partitioned between 2% HCl and ether. The aqueous layer was basified with 40% NaOH, extracted with three portions of $CHCl_3$, dried over $Na_2SO_4$ and chromatographed on 23 g fine $SiO_2$ using 223 mg (56%) of Step D product as a colorless solid.

Step E : (1R.2S.3S)-3-Amino-1,2-dihydroxyindan

To a solution of 215 mg (0.69 mmol) of N-(1,2-dihydroxy-3-indanyl) L-phenylalanine amide (Step D product) in 10 mL of EtOH was added a solution of 84 mg (2.1 mmol)of NaOH in 4 mL of $H_2O$. The mixture was heated at reflux for 6 hours. The solvents removed at reduced pressure, and the residue triturated with 8 mL of $CHCl_3$. The triturate was applied to a column of 4 g of fine $SiO_2$ using 88 : 12 : 1.2 $CHCl_3$-$CH_3OH$-$NH_4OH$ at the eluent to give 104 mg (91%) of Step E product as a colorless solid.

Step F : Preparation of N-(2(R),3(R)-dihydroxy-1(S)-indanyl-5(S)-(1,1-dimethylethoxycarbonylamino)-4(S)-hydroxy-6-phenyl-2(R)-(3-hydroxyphenylmethyl)hexanamide.

Employing the procedure substantially as described in Example 6, Steps C and D, but substituting for the 1(S)-amino-2(R)-hydroxyindane in Step C, (1R,2S,3S)-3-amino-1,2-dihydroxyindane there was obtained N-(2(R),3(R)-dihydroxy-1(S)-indanyl-5(S)-(1,1-dimethylethoxycarbonylamino)-4(S)-hydroxy-6-phenyl-2(R)-(3-hydroxyphenylmethyl)hexanamide as a white solid, homogeneous by TLC (10% methanol/ chloroform), mp 209°C dec, begins to sinter at 160°C.

Step G : Preparation of N-(2(S),3(R)-dihydroxy-cis-1(S)-indanyl)-5(S)-(1,1-dimethylethoxycarbonylamino)-4(S)-hydroxy-6-phenyl-2(R)-(3-(2-(4-morpholinyl)ethoxy)phenylmethyl) hexanamide hydrate

Employing the procedure substantially as descrbied in Example 1, Step L but substituting for the N-(2(R)-hydroxy-1(S)-indanyl-5(S)-(1,1-dimethylethoxycarbonylamino)-4(S)-hydroxy-6-phenyl-2(R)-(4-hydroxyphenylmethyl)hexanamide, N-(2(S),3(R)-dihydroxy-1(S)-indanyl)-5(S)-(1,1-dimethylethoxycarbonylamino)-4(S)-hydroxy-6-phenyl-2(R)-(3-hydroxyphenylmethyl) hexanamide (product of Step H), there was obtained the product, mp 166-8°C dec. (sinters at 140°C). Elemental analysis, calc'd. for $C_{39}H_{51}N_3O_8 \cdot H_2O$ : (707.565) :

C, 66.20 ; H, 7.55 ; N, 5.94 ;
Found : C, 65.93 ; H, 7.39 ; N, 5.90.

maleate hydrate
mp 101-3°C. elemental analysis,
Calc'd. for $C_{39}H_{51}N_3O_8 \cdot C_4H_4O_4 \cdot H_2O$ : (823.945) :

C, 62.68 ; H, 6.97 ; N, 5.09 ;
Found : C, 62.42 ; H, 6.70 ; N, 4.72.

EXAMPLE 8

Step A : Preparation of N-(2(R),3(R)-dihydroxy-1(S)-indanyl-5(S)-(1,1-dimethylethoxycarbonylamino)-4(S)-hydroxy-6-phenyl-2(R)-(4-hydroxyphenylmethyl)hexanamide.

Employing the procedure substantially as described in Example 1, Steps J and K, but substituting for the 1(S)-amino-2(R)-hydroxyindane in Step J, (1R,2S,3S)-3-amino-1,2-dihydroxyindane there was obtained N-(2(R),3(R)-dihydroxy-1(S)-indanyl-5(S)-(1,1-dimethylethoxycarbonylamino)-4(S)-hydroxy-6-phenyl-2(R)-(4-hydroxyphenylmethyl)hexanamide as a white solid, homogeneous by TLC (10% methanol/chloroform), mp 183-5°C dec, begins to sinter at 180°C.

Step B : Preparation of N-(2(S),3(R)-dihydroxy-cis-1(S)-indanyl)-5(S)-(1,1-dimethylethoxycarbonylamino)-4(S)-hydroxy-6-phenyl-2(R)-(4-(2-(4-morpholinyl)ethoxy)phenylmethyl)hexanamide

Employing the procedure substantially as described in Example 1, Step L but substituting for the N-(2(R)-hydroxy-1(S)-indanyl-5(S)-(1,1-dimethylethoxycarbonylamino)-4(S)-hydroxy-6-phenyl-2(R)-(4-hydroxyphenylmethyl)hexanamide, N-(2(S),3(R)-dihydroxy-cis-1(S)-indanyl)-5(S)-(1,1-dimethylethoxycarbonylamino)-4(S)-hydroxy-6-phenyl-2(R)-(4-hydroxyphenylmethyl)hexanamide (product of Step H), there was obtained N-(2(S),3(R)-dihydroxy-1(S)-indanyl)-5(S)-(1,1-dimethylethoxycarbonylamino)-4(S)-hydroxy-6-phenyl-2(R)-(4-(2-(4-morpholinyl)ethoxy)phenylmethyl)hexanamide, mp 190°C (dec.) elemental analysis, Calc'd. for $C_{39}H_{51}N_3O_8 \cdot 0.4\ CHCl_3$ : (737.586) :

C, 64.16 ; H, 7.02 ; N, 5.70 ;
Found : C, 64.23 ; H, 7.10 ; N, 5.73.

maleate hydrate
mp 112-3°C. elemental analysis.
Calc'd. for $C_{39}H_{51}N_3O_8 \cdot C_4H_4O_4 \cdot H_4O$ : (823.945) :

C, 62,68 ; H, 6.97 ; N, 5.09 ;
Found : C, 62,53 ; H, 6.65 ; N, 4.98

EXAMPLE 9

Preparation of N-(2(R)-hydroxy-1(S)-indanyl)-5(S)-[(1,1-dimethylethoxycarbonyl)amino]-4(S)-hydroxy-6-(4-(2-(4-morpholinyl)ethoxy)phenylmethyl)-2(R)-phenylmethylhexanamide

Step A : Preparation of N-methoxy-N-methyl-N'-2(S)-[(1,1-dimethylethoxycarbonyl)amino]-3-(4-benzyloxyphenyl)propionamide

To a stirred solution of N.O-dimethylhydroxylamine hydrochloride (15.8 g, 162 mol) in 120 mL dry methylene chloride cooled to 0°C in an ice/water bath and under nitrogen was added N-methylpiperidine (19.7 mL, 162 mmol) such that the temperature of the reaction remained below 2°C. The clear solution was stored at 0°C until, needed.

To a stirred solution of N-Boc-O-benzyl-L-tyrosine (50.0 g, 135 mmol) in 800 mL dry methylene chloride and 200 mL dry tetrahydrofuran cooled to -20°C in a dry ice/isopropanol/water bath and under nitrogen was added rapidly N-methylpiperidine (19.7 mL, 162 mmol). The solution was allowed to warm to -12°C and isobutyl chloroformate (21.1 mL, 162 mmol) was added rapidly such that the temperature of the reaction remained between -12°C and -8°C. After addition was complete the reaction was stirred at 0°C for 30 minutes then the previously prepared solution of N,O-dimethylhydroxylamine hydrochloride and N-methylpiperidine in methylene chloride was added in one portion. The reaction was allowed to warm to 25°C where it was stirred for 15 hours. After cooling to 0°C in an ice/water bath the reaction was washed with 10% citric acid (2 x 200 mL) and brine (1 x 200 mL), dried over anhydrous magnesium sulfate, filtered, and concentrated to give 60.2 g tan solid. Crude product was dissolved in ethyl acetate and filtered through a pad of silica gel (230-400 mesh, 400 g in a 2 1 sintered glass funnel). The pad was rinsed with 3 1 ethyl acetate and the filtrate was concentrated to give Step A product N-methoxy-N-methyl-N'-2(S)-[(1,1-dimethylethoxycarbonyl)amino]-3-(4-benzyloxyphenyl)propionamide (51.8 g. 92.8% yield) as a white fluffy solid, mp = 107-108°C : elemental analysis, Calcd. for $C_{23}H_{30}N_2O_5$ (414.50) :

C, 66.65 ; H, 7.30 ; N, 6.76 ;
Found : C, 66.68 ; H, 7.13 ; N, 6.64 ; $[\alpha]_D^{25}$ = +5.6° (c = 1.7, methanol).

Step B : Preparation of N-2(S)-[(1,1-dimethylethoxycarbonyl)amino]-3-(4-benzyloxyphenyl)propionaldehyde

To a suspension of lithium aluminum hydride (2.22 g, 58.0 mmol) in 200 mL dry diethyl ether cooled to -40°C in a dry ice/isopropanol bath and under nitrogen was added dropwise a solution of Step A product, N-methoxy-N-methyl-N'-2(S)-[(1,1-dimethylethoxycarbonyl)amino]-3-(4-benzyloxyphenyl)propionamide (20.5 g, 49.5 mmol) such that the temperature of the reaction remained between -36°C and -38°C. After addition was complete the reaction was warmed to 7°C, then cooled to -35°C and quenched by the addition of 40 mL 2.75 M potassium bisulfate. After warming to 25°C and stirring for 1 hour the mixture was filtered through a pad of Celite® with diethyl ether rinse. The filtrate was washed with 10% citric acid (3 x 100 mL), water (1 x 100 mL), diluted $NaHCO_3$ (2 x 100 mL), and brine (1 x 100 mL), dried over anhydrous magnesium sulfate, filtered, and concentrated to give Step B product N-2(S)-[(1,1dimethylethoxycarbonyl)amino]-3-(4-benzyloxyphenyl)propionaldehyde (17.76 g, quantitative yield) as a white solid, mp = 98-99°C, $[\alpha]_D^{25}$ = 27.4° (c = 1.6, methanol).

Step C

Thru

Step J : Preparation of N'-(1.1-dimethylethoxycarbonyl)-5(S)-amino-4(S)-(1',1'-dimethylethyl-1,1-dimethylsilyloxy)-6-(4-benzyloxyphenyl)-2(R)-(phenylmethyl)-hexanoic acid

Employing the procedure substantially as described in Example 1, Steps A through H but substituting for the N-2(S)-[(1,1-dimethylethoxycarbonyl)amino]-3-phenyl propionaldehyde the product of Step B, 13.4 g, there was produced in sequence, the following :

Step C

Thru

Step E :

N-3(S)-[(1,1-Dimethylethoxycarbonyl)amino]-4-(4-benzyloxyphenyl)-1-butene (9.9 g, 74% yield) mp 87-8°C, elemental analysis,
Calcd. for $C_{22}H_{27}NO_3$ (353467) :

C, 74.76 ; H, 7.70 ; N, 3.96.
Found : C, 74.85, H, 7.69 ; N, 3.93. $[\alpha]_D^{25}$ = t6.23° (c = 1, methanol).

Step F :

1(R)-[1'(S)-(1',1'-dimethylethoxycarbonylamino-2-(4-benzyloxyphenyl)ethyl]oxirane as an oil (77% yield) homogeneous by thin layer chromatography (silica gel, 25% ethyl-acetate/hexanes).

Step G :

(5S,1'S)-3-carboethoxy-5-(1-((1',1'-dimethylethoxycarbonyl)amino)-2-(4-benzyloxyphenyl)ethyl)-dihydro furan-2-(3H)-one (79% yield) as a white solid, mp 117-119°C
analysis, Calc'd for $C_{27}H_{33}NO_7$ (483.567) :

| | |
|---|---|
| | C, 67.06 ; H, 6.88 : N. 2.90. |
| Found : | C, 66.94 ; H, 6.96 ; N, 2.91. $[\alpha]_D^{25}$ = -11.27°, (c = 1.81, methanol). |

Step H :

(5S,1'S)-3-carboethoxy-3-phenylmethyl-5-[1-((1,1-dimethylethoxycarbonyl)amino)-2-(4-benzyloxymethyl) ethyl]dihydrofuran-(3H)-one, (97% yield) as a clear glass which was essentially homogeneous by TLC (50% diethyl-ether/hexanes).

Step I :

(3R,5S,1'S)-3-benzyl-5-[1-((1,1-dimethylethoxycarbonyl)amino)-2-(4-benzyloxyphenyl)ethyl]-dihydrofur an-2-(3H)-one, (38% yield) as a clear glass which was homogeneous by TLC (50% diethyl ether/hexane). The latter fractions of the chromatography gave 40% of a mixture of the 3S and 3R lactones.

Step J :

N'-(1,1-dimethylethoxycarbonyl)-5(S)-amino-4(S)-(1',1'-dimethylethyl-1,1-dimethylsilyloxy)-6-(4-benzylo xyphenyl)-2(R)-(phenylmethyl)-hexanoic acid, 94% yield, as a colorless foam.

Step K

Thru

Step L : Preparation of
N-(2(R)-hydroxy-1(S)-indanyl)-5(S)-[(1,1-dimethylethoxycarbonyl)amino]-4(S)-hydroxy-6-(4-hydroxyphenyl)-2(R)-(phenylmethyl)hexanamide

Employing the procedure substantially as described in Example 1, Steps J through K, but substituting for the N'-(1,1-dimethylethoxycarbonyl)-5(S)-amino-4(S)-(1',1'-dimethylethyl-1,1-dimethylsilyloxy)-6-phenyl-2(R)-(4-benzyloxyphenylmethyl)hexanoic acid the product of Step J, 0.350 g, there was produced in sequence, the following :

Step K :

N-(2(R)-hydroxy-1(S)-indanyl)-5(S)-[1,1-dimethylethoxycarbonyl)amino]-4(S)-hydroxy-6-(4-benzyloxyph enyl)-2(R)-(phenylmethyl) hexanamide, 343 mg (96% yield), as a white solid which was essentially homogeneous by TLC (3% methanol/$CH_2Cl_2$).

Step L :

N-(2(R)-hydroxy-1(S)-indanyl)-5(S)-(1,1-dimethylethoxycarbonylamino)-4(S)-hydroxy-6-(4-hydroxyphen yl)-2(R)-(phenylmethyl) hexanamide, 280 mg (92% yield) of pure product : mp 210-211°C (effervescent) elemental analysis, Calc'd for $C_{33}H_{40}N_2O_6$ (560.696) :

| | |
|---|---|
| | C, 70.69 ; H, 7.19 ; N, 5.00. |
| Found : | C, 70.62 ; H, 7.39 ; N, 4.79. |

Step M : Preparation of

N-(cis-2(R)-hydroxy-1(S)-indanyl)-5(S)-(1,1-dimethylethoxycarbonylamino)-4(S)-hydroxy-6-(4-(2-(4-morpholinyl)ethoxy)phenyl)-2(R)-phenylmethyl hexanamide

Employing the procedure substantially as described in Example 1, Step L but substituting for the N-(2(R)-hydroxy-1(S)-indanyl-5(S)-(1,1-dimethylethoxycarbonylamino)-4(S)-hydroxy-6-phenyl-2(R)-(4-hydroxyphenylmethyl)hexanamide. N-(2(R)-hydroxy-1(S)-indanyl-5(S)-(1,1-dimethylethoxycarbonylamino)-4(S)-hydroxy-6-(4-hydroxyphenyl)-2(R)-phenylmethyl hexanamide (product of Step L), there was obtained N-(cis-2(R)-hydroxy-1(S)-indanyl)-5(S)-(1,1-dimethylethoxycarbonylamino)-4(S)-hydroxy-6-(4-(2-(4-morpholinyl)ethoxy)phenyl)-2(R)-phenylmethyl hexanamide, mp 160-1°C (sinters). elemental analysis.
Calc'd. for $C_{39}H_{51}H_3O_7 \cdot 0.25\ H_2O$ : (678.36) :

C, 69.05 ; H, 7.65 ; N, 6.19 ;
Found : C, 69.01 ; H, 7.59 ; N, 5.91.

EXAMPLE 10

Preparation of N-(2(R)-hydroxy-1(S)-indanyl)-5(S)-(1,1-dimethylethoxycarbonylamino)-4(S)-hydroxy-6-(4-hydroxyphenyl)-2(R)-(4-hydroxyphenylmethyl)hexanamide

Employing the procedure substantially as described in Example 1, Steps F through L, but substituting for the (5S,1S')-3-carboethoxy-5[1-((1',1'-dimethylethoxycarbonyl)amino)-2-phenylethyldihydrofuran-2-(3H)-one the product of Example 9, Step H, 1.80 g, there was obtained in sequence the following (Step A through F).

Step A :

(5S,1S')-3-carboethoxy-3-(4-benzyloxyphenylmethyl)-5-[1-((1,1-dimethylethoxycarbonyl)amino)-2-(4-benzyloxyphenyl)ethyl]-dihydrofuran-2-(3H)-one, 1.17 g (67% yield) as a clear resin essentially homogeneous by TLC (50% ether/hexanes).

Step B :
(3R,5S,1'S)-3-(4-benzyloxyphenylmethyl)-5-[1-((1,1-dimethylethoxycarbonyl)amino)-2-(4-benzyloxyphenyl)ethyl]-dihydrofuran-2-(3H)-one, 0.65 g (65% yield) as a clear resin which was essentially homogeneous by TLC (25% ethyl acetate/hexanes). The latter fractions of the chromatography gave a 30% yield of the 35 lactone.

Step C :

N'-(1,1-dimethylethoxycarbonyl)-5(S)-amino-4(S)-(1',1'-dimethylethyl-1,1-dimethylsilyloxy)-6-(4-benzyloxyphenyl)-2(R)-(4-benzyloxyphenylmethyl)-hexanoic acid, 0.80 g (quantitative yield) as a colorless foam, essentially homogeneous by TLC (5% methanol/$CHCl_3$).

Step D :

N-(2(R)-hydroxy-1(S)-indanyl)-5(S)-[1,1-dimethylethoxycarbonyl)amino]-4(S)-hydroxy-6-(4-benzyloxyphenyl)-2(R)-(4-benzyloxyphenylmethyl) hexanamide, 256 mg (95% yield) as a white solid, essentially homogeneous by TLC (2% methanol/$CH_2Cl_2$).

Step E :

N-(2(R)-hydroxy-1(S)-indanyl)-5(S)-(1,1-dimethylethoxycarbonylamino)-4(S)-hydroxy-6-(4-hydroxyphenyl)-2(R)-(4-hydroxyphenylmethyl) hexanamide hydrate, 196 mg (quantitative yield) as a white solid : mp 203-4°C (effervescents sinters at 185°C), elemental analysis, calc'd for $C_{33}H_{40}N_2O_7 \cdot 0.75\ H_2O$ (590.207) :

C, 67.15 ; H, 6.91 ; N, 4.74.
Found : C, 66.96 ; H, 6.68 ; N, 4.64.

Step F :

N-(2(R)-hydroxy-1(S)-indanyl)-5(S)-(1,1-dimethylethoxycarbonylamino)-4(S)-hdyroxy-6-[(4-(2-(4-morpholinyl)ethoxy)phenyl-2(R)-(4-(2-morpholinyl)ethoxy)phenylmethyl hexanamide hemihydrate as a white solid : mp 164-5°C. elemental analysis, calc'd for $C_{45}H_{62}H_4O_9 \cdot 0.5\ H_2O$ : (812.023) :

C, 66.56 ; H, 7.82 ; N, 6.90 ;
Found : C, 66.82 ; H, 7.95 ; N, 6.88.
maleate dihydrate
mp 80-1°C. elemental analysis,
Calc'd. for $C_{45}H_{62}H_4O_9 \cdot C_4H_4O_4 \cdot 2\ H_2O$ : (955.121) :

C, 61.62 ; H, 7.38 ; N, 5.87 ;
Found : C, 61.93 ; H, 7.06 ; N. 5.66.

EXAMPLE 11

Preparation of
N-(2(R),3(R)-dihydroxy-1(S)-indanyl)-5(S)-(1,1-dimethylethoxycarbonylamino)-4(S)-hydroxy-6-[(4-(2-(4-morpholinyl)ethoxy)phenyl-2(R)-(4-(2-morpholinyl)ethoxy)phenylmethyl) hexanamide hemihydrate

Employing the procedure substantially as described in Example 1, Steps J through L, but substituting for the N'-(1',1'-benzyloxyphenylmethyl)hexanoic acid and 1(S)-amino-2(R)-hydroxyindane in Step J, N'-(1',1'-dimethylethyl-1,1-dimethylsilyloxy)-6-(4-benzyloxyphenyl)-2(R)-(4-benzyloxyphenylmethyl)hexanoic acid and (1R,2S,3R)-3-amino-1,2-dihydroxy-indane, respectively, there was obtained in sequence the following (Steps A through C).

Step A through B :

N-(2(R),3(R)-dihydroxy-1(S)-indanyl-5(S)-(1,1-dimethylethoxycarbonylamino)-4(S)-hydroxy-6-(4-hydroxyphenyl)-2(R)-(4-hydroxyphenylmethyl) hexanamide, TLC (10% methanol/chloroform), mp 183-5°C dec. elemental analysis, Calc'd. for $C_{33}H_{40}N_2O_8$ : (592.695) :

C, 66.88 ; H, 6.80 ; N, 4.73 ;
Found : C, 66.63 ; H, 6.76 ; N, 4.84.

Step C :

N-(2(R),3(R)-dihydroxy-1(S)-indanyl)-5(S)-(1,1-dimethylethoxycarbonylamino)-4(S)-hydroxy-6-[(4-(2-(4-morpholinyl)ethoxy)phenyl-2(R)-(4-(2-morpholinyl)ethoxy)phenylmethyl) hexanamide hydrate, mp 112°C dec. (sinters at 100°C). elemental analysis.
Calc'd. for $C_{45}H_{62}H_4O_{10} \cdot C_4H_4O_4 \cdot 2.5\ H_2O$ : (980.13) :

C, 60.05 ; H, 7.30 ; N, 5.71 ;
Found : C, 60.05 ; H, 7.25 ; N, 5.56.

EXAMPLE 12

Preparation of
N-(cis-2(R)-hydroxy-1(S)-indanyl)-5(S)-[1-methylethoxycarbonylamino)-4(S)-hydroxy-6-phenyl)-2(R)-[(4-(2-(4-morpholinyl)ethoxy)phenyl]methyl]hexanamide

To a stirred solution of 73 mg of N-(cis-2(R)-hydroxy-1(S)-indanyl)-5(S)-[1,1-dimethylethoxycarbonylamino)-4(S)-hydroxy-6-phenyl-2(R)[(4-(2-(4-morpholinyl)ethoxy)phenyl]methyl]hexanamide in 2 mL of methylene chloride at 0°C was added 1 mL of trifluoroacetic acid. After warming to room temperature over 3 hours the solution was evaporated to dryness. The residue was dissolved in 10 mL of methylene chloride and allowed to react with 103 mg (5 equivalents) of diisopropyl dicarbonate (Kovalenko, V. I. ; Zhur. Obschei. Khim., 24, 1041 (1954) ; C.A. 47 ; 9510i) and 0.055 mL of triethylamine for 1 hour. The mixture was diluted with 50 mL of methylene chloride, washed with 50 mL of saturated sodium bicarbonate and dried over magnesium sulfate. After concentration to dryness the residue was purified by preparative TLC (2 mm 20 × 20 cm silica gel ;

2 : 8 : 90 $NH_4OH$ : MeOH : $CHCl_3$). The product was extracted off the silica gel with 10% MeOH in $CHCl_3$ and concentrated to dryness. Trituration with ether gave 60 mg of white solid, after drying under vacuum overnight: mp 199-200°C, elemental analysis, Calc'd. for $C_{38}H_{49}N_3O_7 \cdot 1.25\ H_2O$ (682.315) :

C, 66.89 ; H, 7.61 ; N, 6.16 ;
Found : C, 66.83 ; H, 7.38 ; N, 6.17.

maleate hydrate
mp 138-9°C. elemental analysis,
Calc'd. for $C_{40}H_{53}N_3O_6 \cdot C_4H_4O_4 \cdot H_2O$ : (866.025) :

C, 61.10 ; H, 6.87 ; N, 4.85 ;
Found : C, 60.88 ; H, 7.05 ; N, 5.02.

EXAMPLE 13

Preparation of
N-(cis-2(R)-hydroxy-1(S)-indanyl)-5(S)-(ethoxycarbonylamino)-4(S)-hydroxy-6-phenyl)-2(R)-[(4-(2-(4-morpholinyl)ethoxy)phenyl]methyl)hexanamide

To a stirred solution of 100 mg of N-(cis-2(R)-hydroxy-1(S)-indanyl-5(S)-[1,1-dimethylethoxycarbonylamino)-4(S)-hydroxy-6-phenyl-2(R)[(4-(2-(4-morpholinyl)ethoxy)phenyl]methyl]hexanamide in 2 mL of methylene chloride at 0°C was added 1 mL of trifluoroacetic acid. After warming to room temperature over 3 hours the solution was evaporated to dryness. The residue was dissolved in 10 mL of methylene chloride and allowed to react with 120 mg (5 equivalents) of diethyl pyrocarbonate and 0.103 mL of triethylamine for 1 hour. The mixture was diluted with 50 mL of methlene chloride, washed with 50 mL of sat'd sodium bicarbonate and dried over magnesium sulfate. After concentration to dryness the residue was purified by preparative TLC (2 mm 20 × 20 cm silica gel ; 2 :8 :90 $NH_4OH$ :MeOH :$CHCl_3$). The product was extracted off the silica gel with 10% MeOH in $CHCl_3$ and concentrated to dryness. Trituration with ether gave 60 mg of white solid, after drying under vacuum overnight : mp 169-70°C (sinters at 130°C) ; elemental analysis, Calc'd for $C_{37}H_{47}N_3O_7 \cdot H_2O$ (663.817) :

C, 66.95 ; H, 7.44 ; N, 6.33 ;
Found : C, 66.93 ; H, 7.11 ; N, 6.17.

EXAMPLE 14

Preparation of
N-(cis-2(R)-hydroxy-1(S)-indanyl)-5(S)-[1,1-dimethylethoxycarbonylamino)-4(S)-hydroxy-6-phenyl)-2(R)-[(4-(3-(4-morpholinyl)-1-propyloxy)-phenyl]methyl]hexanamide maleate

Employing the procedure substantially as described in Example 1, Step P. but substituting for the N-(2-chloroethyl)morpholine. N-(2-chloropropyl)-morpholine, free base, [Adams, R.R. and Whitmore, F.C., J. Am. Chem. Soc., 67, 735 (1945)] there was obtained 92% yield of a white solid that was converted to the maleate salt in the usual manner :mp 101-3°C. elemental analysis, Calc'd. for $C_{39}H_{51}N_3O_7 \cdot C_4H_4O_4 \cdot 0.5CHCl_3$

C, 61.19 ; H, 6.71 ; N, 4.87 ;
Found : C, 61.03 ; H, 6.70 ; N, 4.79.

EXAMPLE 15

Preparation of
N-(cis-2(R)-hydroxy-1(S)-indanyl)-5(S)-(1,1-dimethylethoxycarbonylamino)-4(S)-hydroxy-6-phenyl)-2(R)-[(4-(2-dimethylaminoethoxy)phenyl)-methyl]hexanamide maleate

Employing the procedure substantially as described in Example 1, Step P, but substituting for the N-(2-chloroethyl)morpholine, 2-dimethylamino-ethyl chloride, free base, there was obtained 98% yield of a white solid, mp 195-7°C (sintered at 185°C) mp 191-2°C. elemental analysis, Calc'd. for $C_{37}H_{49}N_3O_6 \cdot 0.35CHCl_3$ (673.583) :

C, 66.60 ; H, 7.38 ; N, 6.24 ;
Found : C, 66.77 ; H, 7.49 ; N, 6.07.
maleate :
mp 159-61°C. elemental analysis, Calc'd. for $C_{37}H_{49}N_3O_6.C_4H_4O_4.0.5SiO_2$ (777.932) :

C, 63.30 ; H, 6.87 ; N, 5.40 ;
Found : C, 63.09 ; H, 6.85 ; N, 5.23.

EXAMPLE 16

Preparation of
N-(cis-2(R)-hydroxy-1(S)-indanyl)-5(S)-(1,1-dimethylethoxycarbonylamino)-4(S)-hydroxy-6-phenyl-2(R)-[(4-(2 -(1-piperidinyl)ethoxy)phenyl)methyl]hexanamide.

Employing the procedure substantially as described in Example 1, Step P, but substituting for the N-(2-chloroethyl)morpholine, N-(2-chloroethyl)-piperidine, free base, there was obtained 97% yield of a white solid: mp 190.5-191.5°C. elemental analysis, Calc'd. for $C_{40}H_{53}N_3O_6.0.5H_2O$ (680.85) :

C, 70.56 ; H, 7.99 ; N, 6.17 ;
Found : C, 70.64 ; H, 7.97 ; N, 6.03.
maleate hydrate :
mp 95-97°C. dec. elemental analysis,
Calc'd. for $C_{40}H_{53}N_3O_6.C_4H_4O_4.SiO_2$ (866.025) :

C, 61.10 ; H, 6.87 ; N, 4.85 ;
Found : C, 60.88 ; H, 7.05 ; N, 5.02.

EXAMPLE 17

Preparation of
N-(cis-2(R)-hydroxy-1(S)-indanyl)-5(S)-(1,1-dimethylethoxycarbonylamino)-4(S)-hydroxy-6-phenyl)-2(R)-[(4-(2 -(1-pyrrolidinyl)ethoxy)phenyl)methyl]hexanamidemaleate dihydrate.

Employing the procedure substantially as described in Example 1, Step P, but substituting for the N-(2-chloroethyl)morpholine, 2-chloroethyl) pyrrolidine, free base, there was obtained 95% yield of a white solid : mp 102-104°C. elemental analysis, Calc'd. for $C_{39}H_{51}N_3O_6.C_4H_{404}.2H20$ (809.93) :

C, 63.76 ; H, 7.34 ; N, 5.19 ;
Found : C, 64.10 ; H, 7.03 ; N, 5.19.

EXAMPLE 18

Preparation of
N-(cis-2(R)-hydroxy-1(S)-indanyl)-5(S)-(1,1-dimethylethoxycarbonylamino)-4(S)-hydroxy-6-phenyl)-2(R)-[(4-(2 -(N,N-bis(2-methoxyethyl)amino)ethoxy)phenyl)methyl]hexanamide hemihydrate

Employing the procedure substantially as described in Example 1, Step P, but substituting for the N-(2-chloroethyl)morpholine,2-chloro-N,N-bis(2-methoxyethyl)ethylamine, free base, there was obtained 97% yield of a white solid : mp 176-77°C. elemental analysis, Calc'd. for $C_{41}H_{57}N_3O_8.0.5H20$ (728.933) :

C, 67.56 ; H, 8.02 ; N, 5.76 ;
Found : C, 67.81 ; H, 7.99 ; N, 5.71.
maleate hydrate :
mp 93-94°C. dec. elemental analysis,

Calc'd. for $C_{41}H_{57}N_3O_8.C_4H_4O_4.H_2O$ : (854.01) :

C, 63.28 ; H, 7.44 ; N, 4.92 ;
Found : C, 63.22 ; H, 7.15 ; N, 4.74.

EXAMPLE 19

Preparation of N-(cis-2(R)-hydroxy-1(S)-indanyl)-5(S)-(1,1-dimethylethoxycarbonylamino)-4(S)-hydroxy-6-phenyl-2(R)-[(4-(2-(4-thiomorpholinyl)ethoxy)phenyl]methyl]hexanamide hemihydrate.

Employing the procedure substantially as described in Example 1, Step P, but substituting for the N-(2-chloroethyl)morpholine, N-(2-chlorethyl) thiomorpholine, free base, [Leonard, F. and Horne, H., J. Am. Chem. Soc., 78, 1199 (1956)] there was obtained 95% a yield of a white solid : mp 195.5-197.5°C. elemental analysis, Calc'd. for $C_{39}H_{51}N_3O_6S{\cdot}0.25H_2O$ (694.385) :

C, 67.45 ; H, 7.48 ; N, 6.05 ;
Found : C, 67.43 ; H, 7.60 ; N, 6.03.

EXAMPLE 20

Preparation of N-(cis-2(s),3(R)-diacetoxy-1(S)-indanyl-5(S)-(1,1-dimethylethoxycarbonylamino)-4(S)-hydroxy-6-phenyl-2(R)-[(4-(2-(4-morpholinyl)ethoxy)phenyl]methyl]hexanamide maleate hydrate

To a stirred solution of (100 mg, 0.15 mmole) in 5 mL of methylene chloride and 2 mL of pyridine was added 0.14 mL of acetic anhydride. After stirring for 36 hrs at 25°C, the mixture was concentrated to dryness. The residue was purified by preparative thin layer chromatography on 2-2 mm 20 x 20 cm plates using double elution with 7% methanol in chloroform. The major uv-active band was collected and extracted with 200 mL of 10% methanol in chloroform and concentrated to dryness. Trituration of the residue with 25 mL of 1 :9 ethyl acetate: diethyl ether gave 71 mg of a white solid : mp 148-150°C. elemental analysis,.
Calc'd for $C_{43}H_{55}N_3O_{10}.C_4H_4O_4$. $H_2O$ (908.16) :

C, 62.17 ; H, 6.77 ; N, 4.62 ;
Found : C, 61.77 ; H, 6.52 ; N, 4.54.

EXAMPLE 21

Preparation of N-(3(S)-hydroxy-4(S)-benzopyranyl)-N'-(1,1-dimethylethoxycarbonyl)-5(S)-amino-4(S)-hydroxy-6-phenyl-2(R)-(4-(2-(4-morpholinyl)ethoxy)-phenyl)-methyl]-hexanamide.

Step A : Preparation of tetrahydro-2H-benzopyrano-(4.3)oxazole

A mixture of 0.138 g of silver cyanate and 0.228 g of iodine in 5 mL of ether were stirred at room temperature for 1 hour. The reaction mixture was then cooled (ice-water bath) and a solution of 0.116 g of 3-chromene in 1 mL of ether was added dropwise. The suspension was stirred vigorously at room temperature for about 4 hours and the precipitated silver salts filtered off. Methanol (5 mL) and a few drops of a solution of lithium methoxide in methanol, were added to the filtrate and the mixture stirred at room temperature for 12 hours. Solvents were then evaporated and the residue redissolved in a small volume of ether, washed with water, dried over anhydrous $Na_2SO_4$, and evaporated to furnish iodo-carbamate. The crude iodo-carbamate was dissolved in 2.5 mL of diglyme and heated to 160°C for 12 hours. After evaporation of solvent, the residue was chromatographed over silica gel (50% ethyl acetate-hexane as eluent) to give the Step A product.

Step B : Preparation of Cis-4-amino-chroman-3-ol, also know as (cis)3-hydroxy-4-amino-benzo-pyran

The oxazole from Step A was dissolved in 1 mL of methanol and 2 mL of 1 N methanolic potassium hydroxide was added. The resulting mixture was heated to 40°C for 12 hours. After this period, methanol was

evaporated, and the residue diluted with water, and extracted thoroughly with a mixture (3 :1) of chloroform-ether (3 x 10). The combined extracts were dried over anhydrous $Na_2SO_4$ and evaporated to afford the Step B amino-chromanol product which was used directly without further purification.

Step C : Preparation of N-((cis)-3-hydroxy-4-benzopyranyl))-2(S)-(1,1-dimethylethoxycarbonyl)amino-3-(phenyl)-propionamide.

To 11.13 g of BocPhenylalanine in 100 mL of DMF was added 8.05 g of 1-ethyl-3-(3-dimethyl aminopropyl)carbodiimide hydrochloride, 5.68 g of 1-hydroxybenzotriazole hydrate and 6.2 g of (cis)-3-hydroxy-4-amino-benzopyran. The pH of the solution was adjusted to 8.5 with 5.85 mL of triethylamine. After 18 h at 25°C, the reaction was concentrated to dryness, the residue dissolved in ethyl acetate and the organic layer washed with water, 10% citric acid solution, satd. $NaHCO_3$ solution, and brine. The organic layer was dried, filtered and concentrated to afford a yellow solid which was purified by flash chromatography ($SiO_2$, 98 :2 $CH_2Cl_2$/MeOH) to yield 8 g of product.

Step D : Preparation of N-((cis)-3(S)-hydroxy-4(S)-benzopyranyl))-2(S)-amino-3-(phenyl)-propionamide

Excess HCL gas was bubbled into a solution of 8 g of N-((cis)-3-hydroxy-4-benzopyranyl))-2(S)-(1,1-dimethylethoxycarbonyl)amino-3-(phenyl)-propion amide dissolved in 100 mL of ethyl acetate which was cooled to 0°C. After 30 min the mixture was concentrated to give a pale yellow solid. The solid was dissolved in water, made basic and was extracted with chloroform which was dried, filtered and concentrated to give a solid. The solid was chromatographed on a silica gel column (98 :2 ammonia satd. $CHCl_3$/MeOH) to afford 4.8 g of the wanted product as a white solid. Further elution with 10% MeOH/ammonia satd. $CHCL_3$ gave 3 g of the undesired enantiomer.

Step E : Preparation of N-(cis)-3(S)-hydroxy-4(S)-benzopyran

To 4.8 g of N-((cis)-3(S)-hydroxy-4(S)-benzopyranyl))-2(S)-amino-3-(phenyl)-propionamide in 100 mL of ethanol was added 50 mL of 20% sodium hydroxide solution and the solution was heated to reflux. After 18 h the mixture was cooled and concentrated to dryness. The residue was partitioned between methylene chloride and a minimum amount of water. The organic layer was drawn off, dried, filtered and concentrated to afford the crude product which was purified by chromatography ($SiO_2$, 97 :3 ammonia satd. chloroform/methanol) to give 2.1 g of white product.

Step F : Preparation of N-(3(S)-hydroxy-4(S)-benzopyranyl)-N'-(1,1-dimethylethoxycarbonyl)-5(S)-amino-4(S)-(1',1'-dimethylethyl-1,1-dimethylsilyloxy)-6-phenyl-2(R)-[(4-(benzyloxy)phenyl)methyl]-hexanamide.

N'-(1,1-dimethylethoxycarbonyl)-5(S)-amino-4-(S)-(1',1'-dimethylethyl-1,1-dimethylsilyloxy)-6-phenyl-2(R)-[(4-(benzyloxy)phenyl)methyl]-hexanoic acid of Example 1 step M.(0.17 g) was dissolved in 5 ml of DMF and to it was added 0.051 g of 1-ethyl-3-(3-dimethylaminopropyl)carbodiimide hydrochloride, 0.036 g of hydroxybenztriazole hydrate and 0.052 g of N-(cis)-3(S)-hydroxy-4(S)-benzopyran. Triethylamine was added to the stirring solution until the pH is 8.5. After stirring for 24 hours, the reaction was concentrated to dryness and diluted with 30 ml of 10% citric acid solution. The aqueous layer was washed with ethyl acetate which was dried, filtered and concentrated to afford a crude product which was purified by prep layer chromatography (2mm $SiO_2$, 40% EtOAc/Hexane) to give 75 mg of pure product.

Step G : Preparation of N-(3-(S)-hydroxy-4(S)-benzopyranyl)-N'-(1,1-dimethylethoxycarbonyl)-5(S)-amino-4(S)-hydroxy-6-phenyl-2(R)-[(4-hydroxy)phenyl)methyl]-hexanamide.

N'-(1,1-dimethylethoxycarbonyl)-5(S)-amino-4(S)-(1',1'-dimethylethyl-1,1-dimethylsilyloxy)-6-phenyl-2(R)-[(4-(benzyloxy)phenyl)methyl]-hexanamide (0.075 g) was dissolved in 3 ml of THF and 1 ml of a 1M solution of tetrabutylammonium fluoride in THF was added. After 24 hours at room temperature, the reaction was diluted with 20 ml of water and extracted with ethyl acetate which was dried, filtered and concentrated to afford a white solid. The crude product was dissolved in 10 ml of a 2 :1 mixture of EtOAc/MeOH, 60 mg of 10% Pd/C was added and the reaction was put under a blanket of hydrogen (1 atm). After 18 hours the reaction was flushed with nitrogen, filtered through celite which was washed with 1 :1 EtOH/EtOAc and evaporated to dryness to

afford an off-white solid. The solid was triturated with methylene chloride and filtered to give 30 mg of a white solid.

Step H : Preparation of N-(3(S)-hydroxy-4(S)-benzopyranyl)-N'-(1,1-dimethylethoxycarbonyl)-5(S)-amino-4(S)-hydroxy-6-phenyl-2(R) -[(4-(2-(4-morpholinyl)ethoxy)phenyl)-methyl]-hexanamide.

To 95 mg of N-(3-(S)-hydroxy-4(S)-benzopyranyl)-N'-(1,1-dimethylethoxycarbonyl)-5(S)-amino-4(S)-hydroxy-6-phenyl-2(R)-[(4-hydroxy)phenyl)-methyl]-hexanamide in 60 ml of dioxane was added 450 mg of cesium carbonate and 760 mg of 2-cloroethyl-morpholine. The reaction was heated to 95°C and monitered by TLC (10% MeOH/ammonia satd. chloroform). After 2 hours, the reaction was complete (product higher). The reaction was cooled, filtered through celite and the filtrate evaporated. The residue was triturated with ethyl acetate/methanol (10/1) and a white solid filtered to afford 55 mg of pure product.

EXAMPLE 22

Preparation of N-(cis-2(S)-hydroxy-1(R)-indanyl)-5(S)-(1,1-dimethylethoxycarbonyl)amino-4(S)-hydroxy-6-phenyl-2(R)-(4-(N-(4-morpholinyl)methyl)phenyl)-methyl hexanamide

Step A : Preparation of 4-(dimethoxymethyl)benzyl-bromide :

To a 500 ml round bottomed flask containing a stirring bar and an argon inlet was added 4-bromomethylbenzaldehyde (1.71 g, 8.59 mmol), methanol (80 ml), trimethylorthoformate (80 ml), and p-toluenesulfonic acid (32 mg, 0.17 mmol). This mixture was stirred at room temperature for 1 h. The reaction was quenched by addition of aqueous $NaHCO_3$ (10 ml) and the solvents were removed in vacuo. The residue was extracted with ethyl acetate and the organic solution was dried over $MgSO_4$. Filtration and removal of the solvent gave 1.79 g of 4-(dimethoxymethyl)benzyl bromide as an oil.

Step B : Preparation of (5S.1'S)-3-carboethoxy-3-(4-dimethoxymethyl)phenylmethyl-5-(1-((1,1-dimethylethoxycarbonyl)amino)-2-phenylethyl)dihydrofuran-2-(3H)-one :

To a 250 ml round bottomed flask with a stirring bar and an argon inlet was added (5S,1'S)-3-carboethoxy-5-(1-(1,1-dimethylethoxycarbonyl)amino)-2-phenylethyl)-dihydrofuran-2-(3H)-one (3.00 g, 7.95 mmol), absolute ethanol (50 ml), and 1 M sodium ethoxide in ethanol (7.59 ml, 7.59 mmol). This solution was stirred at room temperature for 10 min., then 4-(dimethoxymethyl)benzyl bromide (1.79 g, 7.59 mmol) was added in 10 ml of ethanol. The mixture was stirred at room temperature overnight.

The solvent was removed in vacuo and the residue was partitioned between ethyl acetate and 10% citric acid. The organic phase was separated and dried over $MgSO_4$. Filtration and removal of the solvent gave 4.00 g of the alkylation product which was used in the next step without further purification.

Step C : Preparation of (3R,5S,1'S)-3-(4-carbonyl) phenylmethyl-5-(1-((1,1-dimethylethoxycarbonyl)amino)-2-phenylethyl)dihydrofuran-2-(3H)-one :

The product from Step B, (4.0 g, 7.38 mmol) was dissolved in 20 ml of dimethoxyethane and to it was added 20 ml of water and lithium hydroxide (2.0g, 73.8 mmol). After stirring for 12 hours the solvents were removed in vacuo and the residue was suspended in 100 ml of 10% citric acid. This solution was extracted with two portions of ethyl acetate and the combined organic solutions were washed with brine and dried over $MgSO_4$. Filtration and removal of the solvent gave a residue which was redissolved in 250 ml of toluene. This solution was heated at reflux for 24 h under argon. The solvent was removed in vacuo and the residue was chromatographed on 100 g of silica gel using 17.5% ethyl acetate in hexane as eluant. There was obtained 1.02 g of the desired cis-lactone, the faster eluting product.

Step D : Preparation of N'-(1,1-dimethylethoxycarbonyl)-5(S)-amino-4(S)-(1',1'-dimethylethyl-1,1-dimethylsilyloxy)-6-phenyl-2(R)-(4-carbonylphenylmethyl)-hexanoic acid :

The product from Step C (1.02g, 2.41 mmol) was dissolved in 15 ml of dimethoxyethane and 15 ml of water. To this solution was added lithium hydroxide (0.60g, 24.1 mmol) and the mixture was stirred at room temperature for 20 h. The dimethoxyethane was removed in vacuo and the aqueous residue was made acidic with 10% citric acid. This solution was extracted with two portions of ethyl acetate. The combined organic extracts were dried ($MgSO_4$), filtered and concentrated in vacuo. The residue was dissolved in 10 ml of DMF and imidazole (1.64 g, 24.10 mmol) and tert-butyldimethylsilyl-chloride (1.82g, 12.05 mmol) were added. This mixture was stirred at room temperature for 24 h. The reaction mixture was poured into 50 ml of methanol and this solution was stirred for 3 h. The methanol was removed in vacuo and the residue was partitioned between 10% citric acid and ethyl acetate. The layers were separated and the organic phase was washed with water and brine. Drying ($MgSO_4$), filtration, and removal of the solvent in vacuo gave the crude silyloxy acid as a foam. This material was chromatographed on 50 g of silica gel using 50% ethyl acetate in chloroform as eluant. There was obtained 292 mg of the step D product as an oil.

Step E : Preparation of
N-(2(R)-hydroxy-1(S)-indanyl)-5(S)-1,1-dimethylethoxy)carbonyl)amino)-4(S)-(1',1'-dimethylethyl-1,1-dimethylsilyloxy)-6-phenyl-2(R)-(4-carbonylphenylmethyl)-hexanamide :

To a 25 ml round bottomed flask with a stirring bar and an argon inlet was added the product from Step D (292 mg, 0.52 mmol), ethyldimethyl-aminopropylcarbodiimide hydrochloride (300 mg, 1.57 mmol), N-hydroxybenztriazole (260 mg, 1.57 mmol), 1(S)-amino-2(R)-hydroxyindane (82 mg, 0.55 mmol), DMF (7.0 ml), and triethylamine (0.36 ml, 2.61 mmol). This mixture was stirred overnight at room temperature. The reaction mixture was partitioned between 10% citric acid and ethyl acetate. The layers were separated and the organic phase was washed with water and brine. Drying ($MgSO_4$), filtration, and removal of the solvent gave 370 mg of the coupling product as an oil.

Step F : Preparation of
N-(2(R)-hydroxy-1(S)-indanyl)-5(S)-1,1-dimethylethoxycarbonyl)amino)-4(S)-hydroxy-6-phenyl-2(R)-(4-carbonylphenylmethyl)hexanamide :

The title compound from Step E (370 mg, 0.51 mmol) was dissolved in 1M tetrabutylammonium fluoride in THF (10 ml, 10 mmol). This mixture was stirred overnight under argon. The reaction mixture was poured into 10% citric acid (50 ml) and this solution was extracted with two portions of ethyl acetate. The combined ethyl acetate extracts were washed with water and brine. Drying ($MgSO_4$), filtration, and removal of the solvent in vacuo gave an off white solid. This material was chromatographed on 20 g of silica gel using 3% methanol in chloroform as eluant to give 103 mg of the 4-hydroxy compound as a white solid.

Step G : Preparation of
N-(cis-2(S)-hydroxy-1(R)-indanyl)-5(S)-(1,1-dimethylethoxycarbonyl)amino-4(S)-hydroxy-6-phenyl-2(R)-4-(N-(4-morpholinyl)methyl)phenylmethylhexanamide :

To a 10 ml round bottomed flask with a stirring bar and an argon inlet was added the product from step F (15 mg, 0.026 mmol), methanol (3.0 ml), aluminum foil bits (70 mg, 2.60 mmol), morpholine (1.15 ml, 13.0 mmol) and mercuric chloride (2.1 mg). This mixture was stirred overnight at room temperature. The reaction mixture was filtered through a celite pad and the filtrate was concentrated in vacuo. The residue was chromatographed on 3 g of silica gel using 2% methanol in ammonia saturated chloroform as eluant. There was obtained 13 mg of the title compound free base. The maleate salt was prepared in methanol in the usual manner mp 119-121 C. Anal. Calc'd for : $C_{42}H_{53}N_3O_{10}$ :

C, 66.39 ; H, 7.03 ; N, 5.53.
Found : C, 66.40 ; H, 6.94 ; N, 5.41.

EXAMPLE 23

Preparation of
N-(2R)-hydroxy-1(S)-indanyl)-5(S)-(1,1-dimethylethyloxycarbonylamino)-4(S)-hydroxy-2(R)-(4-(2-(4-morpholino)ethoxy)phenyl)methyl-6-cyclohexylhexanamide

Step A : Preparation of

(5S,1'S)-5-(1'-((1,1-dimethylethoxy-carbonyl)amino)-2'-cyclohexylethyl)-4,5-dihydrofuran-2-(3H)-one :

A solution of the (5S,1'S)-5-(1'-((1,1-dimethylethoxy-carbonyl)amino)-2'-phenylethyl)-4,5-dihydrofuran-2-(3H)-one was dissolved in ethyl acetate and rhodium on alumina was added. This mixture was shaken under a hydrogen atmosphere (50 psi) at 50°C overnight. Filtration and evaporation of the solvent afforded the title compound as a viscous oil, which solidified as a hard glass.

Step B : Preparation of 4-Benzyloxybenzyl iodide :

A mixture of 25 g of commercial 4-benzyloxybenzyl chloride, 32 g of NaI and 107 mL of acetone was stirred in the dark for 5 hours. The mixture was diluted with 100 mL of acetone, filtered, and concentrated under reduced pressure to an orange solid. The solid was dissolved in 400 mL of ether, washed with 75 mL of water, 2 × 40 mL of 10% $NaHSO_3$, 75 mL of water and dried ($MgSO_4$). The solution was concentrated to 125 mL and 200 mL of hexane added with stirring and cooling (0°C). The product was collected by filtration and dried under vacuum.

Step C : Preparation of (3R,5S,1'S)-3-(4-benzyloxyphenylmethyl)-5-(1((1,1-dimethylethoxycarbonyl)amino)-2'-cyclohexylethyl)4,5-dihydrofuran-2-(3H)-one :

To a solution of 23 mL of diisopropylamine in 100 mL of tetrahydrofuran (THF) at 0°C was added 0.164 moles of n-butyllithium. After 15 minutes at 0-4°C the solution was cooled to -78°C and a solution of 25 g of the product of Step A in 125 mL of THF was added over 1 hour, keeping the temperature below -72°C. The reaction was aged at -54°C for 1 hour, then recooled to -78°C and a solution of 26 g of 4-benzyloxybenzyl iodide in 110 mL of THF was added over 80 minutes keeping the temperature below -74°C. The reaction aged at -78°C for 2.5 hours, then at -54°C for 1.5 hours and quenched at -50°C with 250 mL of 1M $NaHSO_4$. The mixture was extracted with 3 × 160 mL of ethyl acetate and the extracts washed with water, 10% $NaHSO_3$. satd. NaCl and dried ($MgSO_4$). Trituration with 15% ethyl acetate in hexanes gave 13.7 g of product as a tan solid. Chromatography of the mother liquors, eluting with 15% ethyl acetate hexanes gave an additional 8.1 g of product.

Step D : Preparation of N'-(1,1-dimethylethoxycarbonyl)-5(S)-amino-4(S)-(1',1'-dimethylethyl-1,1-dimethylsilyloxy)-6-cyclohexyl-2(R)-(-4-benzyloxyphenylmethyl)hexanoic acid :

The product of Step C, 11.8 g, was dissolved in 350 mL of dimethoxyethane and 350 mL of 0.5 N LiOH. After stirring 4 hours, the mixture was concentrated to 300 mL and acidified with 1 L of 5% citric acid. The white solid product was collected by filtration and dried at 60°C under vacuum 12 hours. The hydroxy-acid (11.6 g) was dissolved in a mixture of DMF (150 mL), imidazole (58.7 g) and tertbutyldimethylchlorosilane (58.7 g). After stirring at 25°C for 48 hours, 170 mL of methanol was added. The mixture was aged for 30 minutes at 25°C, then concentrated under reduced pressure to near dryness. The residue diluted with 250 mL of 10% citric acid and extracted with 4 × 600 mL of ethyl acetate. The extracts were washed with water, dried over $MgSO_4$ and concentrated to dryness. The product (14.9 g) was obtained as a clear resin.

Step E : Preparation of N-((2(R)-hydroxy-1(S)-indanyl)-5(S)-(1,1-dimethylethoxycarbonyl)-4(S)-(1',1'-dimethylethyl-1,1-dimethylsilyoxy)-6-cyclohexyl-2(R)-(-4-benzyloxyphenylmethyl)hexanamide :

The produce of Step E, 14.9 g, 1-hydroxybenzotriazole hydrate, 3.26 g, dimethyl-3-(3-dimethyl-aminopropyl)carbodiimide, 7.04 g, 4-methylmorpholine, 2.8 mL, and 250 mL of DMF were stirred at 25°C for 12 hours. The mixture was concentrated to dryness under reduced pressure dissolved in 300 mL of ethyl acetate, washed with 150 mL of 10% citric acid, water, sat'd $NaHCO_3$ and dried ($MgSO_4$). The residue was purified by chromatography on silica gel. Elution with 30% ethyl acetate in hexanes gave 10.1 g of a foamy resin.

Step F : Preparation of N-((2(R)-hydroxy-1-(S)-indanyl)-5(S)-(1,1-dimethylethoxycarbonyl)-4(S)-(1',1'-dimethylethyl-1,1-dimethylsilyloxy)-6-cyclohexyl-2(R)-(-4-hydroxyphenylmethyl)hexanamide :

The product of Step E, 10.1 g, and 6 g of 10% palladium on carbon in 600 mL of ethyl acetate was stirred under an atmosphere of hydrogen for 12 hours, filtered through celite and concentrated to dryness under reduced pressure. The product, 9.5 g, was obtained as a glass.

Step G : Preparation of N-(2R)-hydroxy-1(S)-indanyl)-5(S)-(1,1-dimethylethyloxycarbonylamino)-4(S)-hydroxy-2(R)-(4-(2-(4-morpholino) ethoxy)phenyl)methyl-6-cyclohexylhexanamide :

The product of Step E, 0.550 g, 30 mL of dioxane, 604 mg of 1-(2-chloroethyl)morpholine, and 1.31 g of cesium carbonate was heated at 80°C for 3.0 hours. After cooling, 100 mL of chloroform was added and the mixture was filtered and concentrated under reduced pressure. The residue, 650 mg, was dissolved in 5 mL of 1M tetrabutylammonium fluoride in THF. After stirring for 3 days, the solution was slowly poured into 100 mL of stirred ice-water. The solids were collected, washed with water, and dissolved in 100 mL of chloroform. After drying over $Na_2SO_4$, the organic solvents were removed at reduced pressure to give a solid which was crystallized from 15 mL of 2 :1 hexanes :ethyl acetate to afford 0.394 g (72%) of the title compound as a colorless solid :

mp = 150-151°C.

| | |
|---|---|
| Analysis : | calcd. for $C_{39}H_{57}N_3O_7$ |
| | C, 68.90 H, 8.45 N, 6.18 |
| Found : | C, 68.57 H, 8.29 N, 6.07. |

EXAMPLE 24

Preparation of 2-Amino-3-methyl cyclopentanol.

Step A : Preparation of 2-[N-(1′,2′-di-tert-butoxycarbonyl)-hydrazino]-3-methylcyclopentanone :

To a stirred suspension of CuCl, 0.297 g, in 10 ml anhydrous ether cooled to -60°C was added 0.74 ml of tri-n-butylphosphine. The yellow solution was cooled to -73°C, and 4.2 ml of 3M methylmagnesium chloride in THF was added at a rate so that the internal temperature did not exceed -60°C. The cuprate was stirred for 20 minutes at -78°C, forming a dark mustard colored solution. Cyclopentenone, 1.0 ml, was added dropwise keeping temperature below -70°C. After 1.5 hours at -78°C, 2.87 g of di-tert-butyl azodicarboxylate in 13 ml anhydrous THF added slowly over 45 minutes into the reaction mixture. The resulting dark brown mixture was stirred for 2 hours. The reaction was quenched by addition of 1 :9 concentrated $NH_4OH$ :sat'd $NH_4Cl$, allowed to warm to room temperature overnight, and diluted with ether. The ethereal layer was washed with sat'd $NH_4Cl$, sat'd brine, dried over $Na_2SO_4$ and concentrated under reduced pressure. Purification of the yellow-orange oily residue by chromatography using 20% ethyl acetate in hexane gave 0.78 g, of product as a white crystalline solid, mp 100-101.5°C.

Step B : Preparation of 2-[N-(1′,2′-di-tert-butoxycarbonyl)-hydrazine]-3-methylcyclopentanol :

To a stirred solution of the product of Step A, 1.1 g, in anhydrous THF cooled to -78°C was added 10 mL of a 1M solution of lithium tri-sec-butylborohydride in THF. After 4 hours at -78°C the reaction was quenched by dropwise addition of 10 mL of 1N NaOH and 10 mL of 30% hydrogen peroxide. The resulting mixture was allowed to warm to room temperature overnight, and extracted with 3 × 75 mL of ether. The combined organic layers were washed with brine, dried over $MgSO_4$ and concentrated under reduced pressure. Chromatography of the oily residue with 20% ethyl acetate in hexane gave, after recrystallization from ether/hexane 1.5 g of the product as a white solid, mp 146°C.

Step C : Preparation of 2-Amino-3-methyl cyclopentanol

To a stirred solution of the product of Step B, 1.494 g, in 5 mL of $CH_2Cl_2$ cooled to -20°C was added 10 mL of trifluoroacetic acid. The resulting brown mixture was allowed to warm to room temperature overnight and concentrated to dryness under reduced pressure. The brown residual oil, 1.7 g, was dissolved in 104 mL of ethanol and 26 mL of acetic acid containing 240 mg of platinum oxide and shaken under 55 psi of hydrogen for 2.5 hours. The catalyst was filtered off and the solution was concentrated under reduced pressure. Chromatography with 45/9/1 $CHCl_3$/MeOH/conc. $NH_4OH$ gave. after trituration with 1/5 $CHCl_3$/$Et_2O$, 0.75 g of

a white amorphous solid, m.p. 109-116°C.
$^1$H NMR (300 MHz, $CDCl_3$) δ 3.92-3.96 (m, 1H), 2.59-2.64 (m, 1H), 2.15 (brs, 3H), 1.92 (m, 2H), 1.65 (m, 2H), 1.12 (m, 1H), 1.04 (d, J = 6.6 Hz, 3H).

EXAMPLE 25

Preparation of (4,5-dihydro-3(2H)-furylidene)acetic acid

A mixture of 3.9 g of tetrahydrofuran-3-one and 22 g of (carbethoxymethyene)triphenylphosphorane in 40 mL of anhydrous acetonitrile was heated to reflux for 4 days. After concentration, the residue was purified by chromatography on silica gel. Elution with 25% ethyl acetate in hexanes gave, after evaporative distillation at 15 mm (oven temperature 140°C), 3.6 g of product as a fragrant liquid. The ester was saponified in 20 mL of ethanol with 20 mL of 1N LiOH for 24 hours. After acidification with 6N HCl to pH2, the acid was isolated by concentration to dryness and extraction with methylene chloride. Azeotropic drying with toluene gave 3.1 g of product as a resinous solid.

EXAMPLE 26

Preparation of 3-tetrahydrofuranylacetic acid

A mixture of 2 g of (4,5-dihydro-3(2H)-furylidene)acetic acid, 25 mL of ethyl acetate and 0.5 g of 10% palladium on carbon was shaken under 50 p.s.i. of hydrogen for 48 hours, filtered, and concentrated to dryness under reduced pressure. The product, 1.8 g, was obtained as a clear oily resin.

EXAMPLE 27

Preparation of 4-tetrahydropyranylacetic acid

To a stirred, cooled suspension of 0.9 g of sodium hydride (60% oil dispersion) in 5 mL of benzene was added 5.32 g of triethylphosphonoacetate in 3 mL of benzene, keeping the temperature below 30°C. After 1 hour at 25°C, a solution of 2 g of tetrahydro-4H-pyran-4-one in 3 mL of benezene was added slowly. After 1 hour at 25°C, 20 mL of water and 50 mL of ether were added. The organic layer was dried over $MgSO_4$ and concentrated to dryness. Evaporative distillation at 5 mm, oven temperature 130°C, gave 1.8 g of product as a fragrant liquid. Hydrogenation in 20 mL of ethanol with 0.5 g of 10% palladium on carbon under 1 atmosphere of hydrogen for 10 days gave 1.8 g of saturated product, after filtration and concentration. Saponification using 20 mL of ethanol, 10 mL of 10% NaOH for 4 hours followed acidification ($H_2SO_4$) and ether extraction gave 1.5 g of product. Evaporative distillation at 0.1 mm, oven temperature 160-8°C gave 1.1 g of product as a crystalline solid, mp 61-2°C.

| | |
|---|---|
| Anal. Calcd. for $C_7H_{12}O_3$ : | C, 58.32 H, 8.39 |
| Found : | C, 58.59 H, 8.40 |

EXAMPLE 28

Preparation of 2-tetrahydrofuranylacetic acid

To a stirred solution of 5 g of gamma-butyrolactone in 100 mL of toluene cooled to -78°C was added 50 mL of 1.5 M diisobutylaluminum hydride in toluene, keeping the temperature below -60°C. After 15 minutes, 25 mL of MeOH was added and the mixture allowed to warm to 25°C for 1 hour. To this solution of 20 g of (carbmethoxymethylene)triphenylphosphorane was added with 25 mL of MeOH. After 4 hours at 25°C, and 0.5 hours at 40°C, the mixture was cooled and quenched with 200 mL of sat'd $NH_4Cl$. The mixture was made homogeneous by addition of 6N HCl, and extracted 4 × 50 mL of ethyl acetate. The combined organics were dried over $MgSO_4$, concentrated to near dryness, triturated with ether (500 mL), filtered and again concentrated. Evaporative distillation at 0.1 mm, oven temperature 100-120°C gave 6 g of the methyl ester of trans-$\Delta^{2,3}$-6-hydroxy-hexenoic acid as a colorless oil. The oil was dissolved in 100 mL of 0.01N sodium methoxide in methanol, aged for 3 days, quenched with 0.1 mL of AcOH and concentrated under reduced pressure. Evaporative distillation at 8 mm, oven temperature 100-110°C gave 4.6 g of the methyl ester of the title compound as an oil. The ester was dissolved in 100 mL of MeOH and 40 mL of 1N LiOH for 3 hours, acidified with 20 mL of 6N HCl and concentrated to dryness. The crude product was triturated with ethyl acetate (100 mL), dried

($MgSO_4$), and concentrated. Evaporative distillation at 0.1 mm, oven temperature 120-30°C gave 3.0 g of product as a colorless liquid.

EXAMPLE 29

Preparation of (2-tetrahydrofuranyl)methyl succinimidyl carbonate

To a solution of 12.5% phogene in toluene (20 mL) coooled to -10°C was added a solution of 1.5 g of 2-tetrahydrofurfuryl alcohol in 5 mL of toluene over 15 minutes. After an additional 30 minutes at -10°C, the mixture was allowed to warm to 25°C for 1 hour. After removal of the excess phosgene with a stream of nitrogen, the mixture was concentrated to dryness under reduced pressure. The oily residue was dissolved in 30 mL of anhydrous acetonitrile and cooled to 0°C. After addition of 2 g of N-hydroxysuccinimide and 2.4 mL of triethylamine, the mixture was allowed to warm to 25°C and stir for 12 hours. The mixture was diluted with 200 mL of ethyl acetate, filtered and concentrated to dryness under reduced pressure. The residue was dissolved in 200 mL of ethyl acetate, washed with 2 × 20 mL of water, dried over $MgSO_4$, and concentrated to dryness. The product crystallized under vacuum overnight.

EXAMPLE 30

Preparation of 3-amino-4-methylcyclopentan-1-ol

Step A : Preparation of 1-oxa-2(N-carbobenzyloxy)-aza-[2.2.1]-bicyclohept-4-ene :

To a stirred solution of 25 g of N-hydroxybenzylcarbamate and 25 mL of freshly distilled cyclopentadiene in 250 mL of $CH_2Cl_2$ cooled to 0°C was added 44 g of tetraethylammonium periodate dissolved in 200 mL of $CH_2Cl_2$ at such a rate that internal temperaure did not exceed 5°C (40 minutes). The reaction was allowed to proceed for an additional hour then diluted with 200 mL of $CH_2Cl_2$ and washed successively with 200 mL of water, 2 × 200 mL of 10% $Na_2SO_3$, water, and 200 mL of sat'd NaCl. The organic layer was dried over $Na_2SO_4$, concentrated to a dark brown oil. Filtration through a pad of silica gel eluting with 50% ethyl acetate in hexanes to remove polar impurities gave a pale yellow oil.

Step B : Preparation of 5-carbenzyloxyamino-3-hydroxy-1-cyclonpentene :

To a stirred solution of the product of Step A, 1 g, in 40 mL of absolute ethanol cooled to 0°C was added 2.3 g of powdered $Na_2HPO_4$ and 14 g of 5% Na/Hg. After 8 hours at 0°C the mixture was diluted with 50 mL of ether, filtered, concentrated to dryness and purified by chromatography. Elution with 50% ethyl acetate in hexane gave, after recrystallyzation from the same solvents, 0.43 g of product, mp 99-101°C.

Step C : Preparation of 4-carbobenzyloxyamino-2-cyclopenten-1-one :

To a stirred solution of 7.3 mmole of oxalyl chloride in 8 mL of $CH_2Cl_2$ cooled to -78°C was added dropwise of 0.9 mL of anhydrous dimethylsulfoxide in 5 mL of $CH_2Cl_2$ containing 0.25 mL of dimethyl sulfoxide was added to a rate so that internal temperature did not exceed -70°C. After aging at -78°C for 10 minutes, the product of step B. 0.425 g, in 10 mL of $CH_2Cl_2$ containing 0.25 mL of dimethyl sulfoxide was added at a rate so that internal temperature did not exceed -70°C. After aging at -78°C for 1 hour, 4.9 mL of triethylamine was added and the contents of the flask allowed to slowly warm to 0°C. The reaction was stirred at this temperature for 25 minutes, then diluted with 50 mL of $CH_2Cl_2$ and water. The organic layer was washed with 50 mL each of 0.5N HCl, sat'd $NaHCO_3$, and sat'd NaCl. After drying ($Na_2SO_4$) the crude product was purified by chromatography. The oily product, 365 mg, solidified under vacuum.

Step D : Preparation of 3-carbobenzyloxyamino-4-methylcyclopentan-1-one :

To a stirred suspension of CuI, 1.17 g, in 6 ml anhydrous ether cooled to -5°C was added 21 mL of 0.6 M methyllithium in ether. After aging for 15 minutes at 0°C, the cuprate was cooled to -40°C and the product of Step C, 0.356 g, was added as a solution in 8 mL of ether. The reaction was aged at -40°C for 1 hour then -20°C for 1 hour and quenched with 9 mL of a solution of $NH_4Cl$ (sat'd) and 1 mL of conc. $NH_3OH$. The cooling bath was removed and the mixture aged for an additional 30 minutes. The layers were separated and the aqueous phase extracted with 50 mL of ether. The combined organics were washed with $NH_4Cl$ (sat'd), NaCl

(sat'd) and dried ($MgSO_4$). Chromatography of the residue (60% ethyl acetate in hexane) gave 0.28 g of a 4 :1 mixture of diastereomers that were used without separation in the next step.

Step E : Preparation of 3-carbobenzyloxyamino-4-methylcyclopentan-1-ol :

To a stirred solution of the product of Step D, 0.280 g, in 4 mL of THF cooled to -78°C was added 4 mL of a 1M solution of lithium tri-sec-butylboro-hydride in THF. The reaction was aged at -78°C for 2 hours, warmed to 0°C and carefully quenched with 5 mL of 1N NaOH and 1.2 mL of 30% $H_2O_2$. The reaction was allowed to warm to room temperature and aged for 30 minutes. The layers were separated and the aqueous layer extracted with 35 mL of ether. The combined organics were washed with water, NaCl (sat'd) and dried ($MgSO_4$). Chromatography of the residue (30% acetone in hexane) gave three products (in order of elution) : siomer A, 0.063 g, as an oil ; isomer B, 0.081 g, as a solid, mp 73-73.5°C, and isomer C, 0.075 g, as a solid, mp 111-112°C.

Step F : Preparation of 3-amino-4-methylcyclopentan-1-ol :

The product of Step E (isomer B), 0.182 g, was dissolved in 4 mL of THF and stirred under an atmosphere of hydrogen for with 0.075 g of 10% palladium on carbon for 1 hour. After filtration to remove the catalyst, the solution was concentrated to dryness under reduced pressure. There was obtained 0.076 g of product as a glassy solid.

EXAMPLE 31

N(2(R)-Hydroxy-1(S)indanyl)-5(S)-((1.1-dimethylethoxycarbonyl)amino)-4(S)-hydroxy-6-phenyl-2(R)-3-(4-(2-(morpholino)ethoxy)phenyl)prop-2-en-1-yl)hexanamide :

Step A : Preparation of 4-tert-butyldimethylsilyloxyphenylprop-2-en-1-yl bromide :

To a 1 L round bottomed flask with a stirring bar and an argon inlet was added 26.25 g (160 mmol) of p-hydroxycinnamic acid, 50.62 g (335 mmol) of tert-butyldimethylsilyl chloride, 32.68 g (480 mmol) of imidazole, and 250 mL of dry DMF. This mixture was stirred at room temperature for 24 hours. The DMF was removed in vacuo and the residue was partitioned between EtOAc and 10% aqeous citric acid. The layers were separated and the organic phase was washed with water (4x) and brine. Drying ($MgSO_4$), filtration and removal of the solvent in vacuo gave 62.3 g of the bissilylether-ester. This ester was placed in an oven dried 2 L round bottomed, 3 necked flask with a mechanical stirrer, argon inlet, and addition funnel. Ether (460 mL) was added and the solution was cooled in an ice bath to 0°C. To this solution was added a solution of Dibal-H 397 mL of a 1.0 M solution in hexanes), dropwise over 1 hour. The mixture was stirred for 1 hour and the reaction was then quenched by careful addition of 1 L of saturated aqueous sodium potassium tartrate solution. This viscous mixture was stirred for 20 hours at room temperature. The mixture was filtered through a celite pad, the filtrate layers were separated and the organic phase was extracted with 2 portions of EtOAc. The combined organic phases were washed with brine, dried ($MgSO_4$), filtered and concentrated in vacuo to give the crude alcohol. This material was chromatographed on 500 g of silica gel using 20% EtOAc in hexanes as eluant. There was obtained 34.9 g of 4-tert-butyldimethylsilyloxycinnamyl alcohol as a viscous oil. This material crystallized on cooling to -15°C. A portion of this alcohol (18.5 g, 69.96 mmol) was placed in a 300 mL round bottomed flask with a stirring bar and 125 mL of dry ether. This solution was cooled to 0°C and 20.83 g (76.95 mmol) of $PBr_3$ was added dropwise with a syringe over 5 minutes. This solution was maintained at 0°C for 20 minutes, diluted with hexanes (1L) and washed with aqueous $NaHCO_3$. This solution was washed with brine, dried ($MgSO_4$), filtered through a pad of silica gel, and concentrated in vacuo to give 17.53 g of 4-tert-butyldimethylsilyloxypenylprop-2-en-1-yl bromide as a colorless oil.

Step B : Preparation of 5(S)-(1,1-dimethylethoxycarbonyl)amino-4(S)-(1',1'-dimethylethyl-1,1-dimethylsilyloxy)-6-phenyl-2(R)-(4-(1',1'-dimethylsilyloxy)phenylprop-2-en-yl)hexanoic acid :

To a 500 mL, 3-necked, oven dried round bottomed flask with an argon inlet, stirring bar, low temperature thermometer, and a jacketed addition funnel was added 75 mL of dry THF and 15.39 mL (109.78 mmol) of diisopropylamine. This solution was cooled to -20°C and n-butyllithium (42.84 mL, 107.11 mmol of a 2.5M solution in hexanes) was added slowly. The resulting solution was cooled to -78°C and a solution of (5S,1'S)-5-((1,1-dimethylethoxycarbonyl)amino)-2-phenylethyl)dihydrofuran-2-(3H)-one (16.36 g, 53.55 mmol) in 75 mL

of dry THF was added at such a rate that the temperature of the solution did not rise above -70°C (ca. 40 minutes required for this additon). The resulting solution was aged at -78°C for 1 hour and the dropping funnel was charged with a solution of 4-tert-butyldimethyl-silyloxyphenylprop-2-en-1-yl bromide (17.53 g, 53.55 mmol) in 75 mL of dry THF. The bromide solution was cooled to -78°C and was then added to the enolate solution dropwise over 45 minutes, keeping the temperature below -70°C. When the addition was complete the solution was aged at -78°C for 1 hour, warmed to -50°C and quenched with a solution of $NaHSO_4$ (33 g) in 250 mL of water. The mixture was diluted with EtOAc and the layers were separated. The organic phase was washed with $NaHCO_3$ solution and brine. Drying ($MgSO_4$), filtration, and removal of the solvent in vacuo provided the crude alkylation product as an oil. This material was chromatographed on 1000 g of silica gel using 20% EtOAc in hexanes as eluant. There was obtained 23.6 g of pure (5S,3R,1'S) 3-(4-(1',1'-dimethylethyl-1,1-dimethysilyloxy)phenyl-prop-2-en-1-yl)-5-(1-((1,1-dimethylethoxycarbonyl)amino)-2-phenylethyl)dihydrofuran-2-(3H)-one as a colorless foam. This material was dissolved in 325 mL of DME and a solution of LiOH (6.99 g, 291.97 mmol) in $H_2O$ (325 mL) was added. This solution was stirred at room temperature for 24 hours. The DME was removed in vacuo and the aqueous residue was acidified with 10% aqueous citric acid. This milky suspension was extracted with several portions of EtOAc and the combined extracts were washed with water and brine, dried ($MgSO_4$), filtered, and concentrated in vacuo. To the crude α-hydroxy acid was added imidazole (56.79 g, 834 mmol), tert-butyl-dimethylsilyl chloride (62.87 g, 417 mmol) and dry DMF (325 mL). This mixture was stirred at room temperature for 24 hours. The mixture was then treated with methanol (325 mL) for 4 hours at room temperature. The solvents were removed in vacuo at 60°C, 20 torr. The residue was dissolved in 1L of EtOAc and washed with 10% aqueous citric acid, water and brine. Drying ($MgSO_4$), filtration and removal of the solvent gave 28.53 g of 5(S)-(1,1-dimethylethoxycarbonylamino)-4(S)-(1',1'-dimethylethyl-1,1-dimethylsilyloxy)-6-phenyl-2(R)-(4-(1',1'-dimethylethyl-1,1-dimethylsilyloxy)phenylprop-2-en-1-yl)hexanoic acid as a colorless foam.

Step C : Preparation of N-(2(R)-hydroxy-1(S)-indanyl)-5(S)-(1,1-dimethylethoxycarbonylamino)-4(S)-(1',1'-dimethylethyl-1,1-dimethylsilyloxy)-6-phenyl-2(R)-((4-(hydroxyphenyl)prop-2-en-1-yl)hexanamide :

To a 2L round bottomed flask with a stirring bar and an argon inlet was added 5(S)-(1,1-dimethylethylethoxycarbonylamino)-4(S)-(1',1'-dimethylethyl-1,1-dimethylsilyloxy)-6-phenyl-2(R)-(4(1',1'-dimethylethyl-1,1-dimethylsilyloxy)phenylprop-2-en-1-yl)hexanoic acid (28.53 g, 41.71 mmol) from Step B, 2(R)-hydroxy-1(S)-aminoindane (6.85 g 45.88 mmol), 3-(N,N-dimethylaminopropyl)ethylcarbodiimide hydrochloride (8.79 g, 45.8 mmol), 1-hydroxybenztriazole hydrate (6.20 g, 45.88 mmol), and dry DMF (300 mL). When all of the solids had dissolved, triethylamine (12.79 mL, 91.76 mmol) was added and the mixture was stirred at room temperature for 18 hours. The mixture was partitioned between EtOAc (1500 mL) and 10% aqueous citric acid (1500 mL). The layers were separated and the organic phase was washed with water (3 x 1000 mL), and brine. Drying ($MgSO_4$), filtration and removal of the solvent in vacuo gave 32 g of a yellow foam. This material was dissolved in 500 mL of methanol and LiOH (4.99 g, 208.55 mmol) was added. This solution was stirred at room temperature for 1 hour. The solution was acidified with aqueous citric acid and the methanol was removed in vacuo. The resulting aqueous residue was extracted with EtOAc (1L). The EtOAc extract was dried ($MgSO_4$), filtered, and concentrated in vacuo. This material was chromatographed on 1 Kg of silica gel using 8L of 40% EtOAc in hexanes as eluant. There was obtained 20.07 g of N-(2(R)-hydroxy-1(S)-indanyl)-5(S)-(1,1-dimethylethoxycarbonyl)amino-4(S)-(1',1'-dimethylethyl-1,1- dimethylsilyloxy)-6-phenyl-2(R)-((4-hydroxyphenyl)prop-2-en-1-yl)hexanamide as a colorless foam.

Step D : Preparation of N-(2(R)-hydroxy-1(S)-indanyl)-5(S)-(1,1-dimethylethoxycarbonylamino)-4(S)-hydroxy-6-phenyl-2(R)-(4-(2-(4-morpholino)-ethoxy)phenylprop-2-en-1-yl)hexanamide :

To a 1L round bottomed flask with a stirring bar and an argon inlet was added N-(2(R)-hydroxy-1(S)- indanyl)-5(S)-(1,1-dimethylethoxycarbonyl)-4(S)-(1',1'-dimethylethyl-1,1-dimethylsilyloxy)-6-phenyl-2(R)-(4-(1',1'-dimethylethyl-1,1-dimethylsilyloxy)phenyl-prop-2-en-1-yl)hexanamide (19.49 g, 28.1 mmol) 1,4-dioxane (400 mL), 4-(2-chloroethyl)morpholine (12.61 g, 84.3 mmol), and powdered cesium carbonate (27.5 g, 84.3 mmol). This mixture was heated at 80°C with vigorous stirring for 3 hours. The cooled reaction mixture was filtered through a celite pad and the 1,4-dioxane was removed in vacuo. To this residue was added a solution of tetrabutylammonium fluoride in THF (280 mL of a 1M solution, 280 mmol) and a stirring bar. This solution was stirred at room temperature under argon for 28 hours. The reaction mixture was poured into 3L of $H_2O$, with stirring. The white solid was collected by filtration and dried in vacuo overnight. The crude product was

chromatographed on silica gel using 5% MeOH in chloroform as eluant. The chromatographed material was recrystallized from boiling EtOAc-hexanes to give 13 g of analytically pure N-(2(R)-hydroxy-1(S)-indanyl)-5(S)-(1,1-dimethylethoxycarbonylamino)-4(S)-hydroxy-6-phenyl-2(R)-(4-(2-(4-morpholino)ethoxy)phenylprop-2-en-1-yl)hexanamide as a white solid, mp :178-179°C.

EXAMPLE 32

Preparation of N-(2(R)-hydroxy-1(S)-indanyl)-5(S)-amino-4(S)-hydroxy-6-phenyl-2(R)-(4-(2-(4-morpholinyl)ethoxy)phenyl)methyl hexanamide bis hydrochloride

To a stirred solution of 0.30 g of N-(2(R)-hydroxy-1(S)-indanyl)-5(S)-(1,1-dimethylethoxycarbonyl)amino-4(S)-hydroxy-6-phenyl-2(R)-(4-(2-(4-morpholinyl)ethoxy)phenyl)methyl hexanamide in 30 mL of $CH_2Cl_2$ was added 10 mL of 1M HC1 in anhydrous ether. After stirring for 3 hours, the mixture was diluted with 50 mL of ether, cooled in an ice bath and filtered. The white solid precipitate (bis HCL salt) weighed 300 mg after drying under vacuum.

EXAMPLE 33

Synthesis of primary alcohol urethanes

Step A : Preparation of (2-tetrahydrofuranyl)methyl succinimidyl carbonate :

To a solution of 12.5% phogene in toluene (20 mL) cooled to -10°C was added a solution of 1.5 g of 2-tetrahydrofurfuryl alcohol in 5 mL of toluene over 15 minutes. After an additional 30 minutes at -10°C, the mixture was allowed to warm to 25°C for 1 hour. After removal of the excess phosgene with a stream of nitrogen, the mixture was concentrated to dryness under reduced pressure. The oily residue was dissolved in 30 mL of anhydrous acetonitrile and cooled to 0°C. After addition of 2 g of N-hydroxy-succinimide and 2.4 mL of triethylamine, the mixture was allowed to warm to 25°C and stir for 12 hours. The mixture was diluted with 200 mL of ethyl acetate, filtered and concentrated to dryness under reduced pressure. The residue was dissolved in 200 mL of ethyl acetate, washed with 2 × 20 mL of water, dried over $MgSO_4$, and concentrated to dryness. The product crystallized under vacuum overnight.

Step B : Preparation of N-(2(R)-hydroxy-1(S)-indanyl)-5(S)-(2(R,S)-tetrahydrofuranyl)methoxycarbonylamino-4(S)-hydroxy-6-phenyl-2(R)-(4-(2-(4-morpholinyl)ethoxy)phenyl)methyl hexanamide :

To a stirred suspension of 70 mg of N-(2(R)hydroxy-1(S)-indanyl)-5(S)-amino-4(S)-hydroxy-6-phenyl-2(R)-(4-(2-(4-morpholinyl)ethoxy)phenyl)methyl hexanamide bis hydrochloride in 15 mL of methylene chloride was added 30 mg of (2-tetrahydrofuranyl)methyl succinimidyl carbonate and 0.040 mL of triethyl amine. After stirring for 12 hours, the mixture was diluted with 50 mL of chloroform, washed with 10 mL of sat'd. sodium bicarbonate, and concentrated to dryness. Chromatopraphy using 8% MeOH in $CHCl_3$ gave 53 mg of product as a white crystalline solid, mp 154-6°C ;
Calc'd for $C_{40}H_{51}N_3O_8xO{\cdot}25H_2O$

| | |
|---|---|
| | C, 68.02 H, 7.34 N, 5.95 |
| Found : | C, 67.92 H, 7.31 N, 5.84. |

EXAMPLE 34

Synthesis of secondary alcohol urethanes

Step A : Preparation of 3(S)-tetrahydrofuranyl succinimidyl carbonate :

A solution of 20 mL of 12.5% phosgene in toluene and 1.0 g of (S)-(+)-3-hydroxytetrahydrofuran was aged in a stoppered flask for 48 hours. The solvents were removed under reduced pressure and the residue dissolved in 30 mL of anhydrous acetonitrile, then cooled in an ice bath. To this cold solution was added 1.7 g of N-hydroxysuccinimide and 1.9 mL of triethylamine. The mixture was aged for 12 hours at 25°C, then concentrated

to dryness. The residue was dissolved in 200 mL of ethyl acetate, washed with 2 × 50 mL of water, dried over $MgSO_4$ and concentrated to dryness under reduced pressure. The oily residue was dissolved in 10 mL of ethyl acetate passed through a 300 mL of silica gel, eluting with ethyl acetate. Concentration of the eluate to dryness gave 2 g of product as a white crystalline solid.

Step B : Preparation of N-(2(R)-hydroxy-1(S)-indanyl)-5(S)-(3(S)-tetrahydrofuranyloxycarbonyl-amino)-4(S)-hydroxy-6-phenyl-2(R)-(4-(2-(4-morpholinyl)ethoxy)phenyl)methyl hexanamide :

To a stirred suspension of 100 mg of N-(2(R)-hydroxy-1(S)-indanyl)-5(S)-amino-4(S)-hydroxy-6-phenyl-2(R)-(4-(2-(4-morpholinyl)ethoxy)phenyl)methyl hexanamide bis hydrochloride in 15 mL of methylene chloride was added 40 mg of 3(S)-tetrahydrofuranyl succinimidyl carbonate and 0.060 mL of triethyl amine. After stirring for 3 hours ; the mixture was diluted with 50 mL of chloroform, washed with 10 mL of sat'd. sodium bicarbonate, and concentrated to dryness. Chromatography using 10% MeOH in $CHCl_3$ gave 69 mg of product as a white crystalline solid. mp 201-2°C ;

| | |
|---|---|
| Calc'd for $C_{39}H_{49}N_3O_8xH_2O$ | C, 66.36 H, 7.28 N, 5.95 |
| Found : | C, 66.51 H, 7.09 N, 5.91. |

EXAMPLE 35

Preparation of hexahydrofuro[2,3b]furan-3a-ol

To a stirred solution of 10 g of 2-n-butyloxy-3-allyl-tetrahydrofuran-3-ol (prepared as described by M. Jalali-Naini and J.Y. Lallemand, Tetrahedron Letters, pp 497-500, 1986) in 10 mL of methanol and 220 mL of methylene chloride cooled to -78°C was added a stream of ozone until a blue color persisted. The mixture was purged with nitrogen, warmed to 0°C and diluted with 100 mL of ethanol. To this mixture was added 5 g of $NaBH_4$. After aging at 25°C for 6 hours, the solvents were removed under reduced pressure and the residue partitioned between 50 mL of 10% citric acid and 3 × 100 mL of methylene chloride. The organic extracts were dried over $MgSO_4$ and concentrated to ca. 200 mL. To this stirred solution was added 0.10 g of p-toluenesulfonic acid monohydrate. The mixture was heated at reflux for 24 hours, then concentrated to dryness under reduced pressure. Evaporative distillation of the residue at 0.1 mm (110-130°C) gave 2 g of the title compound.

EXAMPLE 36

Preparation of tertiary alcohol urethanes

Step A : Preparation of hexahydrofuro[2,3b]furan-3a-yl succinimidyl carbonate :

To a stirred solution of 1 g of hexahydrofuro[2,3b]furan-3a-ol in 25 mL of 12.5% phosgene in toluene cooled to -10°C was added 1 mL of pyridine. The mixture was allowed to warm to 25°C and stir for 4 hours, then concentrated to dryness under reduced pressure. The oily residue after drying under vacuum (1.3 g) was dissolved in 30 mL of anhydrous acetonitrile, then cooled in an ice bath. To this cold solution was added 1.14 g of N-hydroxysuccinimide and 1.3 mL of triethylamine. The mixture was aged for 48 hours at 25°C, then concentrated to dryness. The residue was dissolved in 200 mL of ethyl acetate, washed with 2 × 50 mL of water, dried over $MgSO_4$ and concentrated to dryness under reduced pressure. Chromatography of the residue with 20% ethyl acetate in methylene chloride gave 0.49 g of product as a white crystalline solid.

Step B : Preparation of N-(2(R)-hydroxy-1(S)-indanyl)-5(S)-(hexahydrofuro[2,3b]-furanyl-3a-oxycarbonylamino)-4(S)-hydroxy-6-phenyl-2(R)-(4-(2-(4-morpholinyl)ethoxy)phenyl)methyl hexanamide :

To a stirred suspension of 100 mg of the bis HCl salt in 15 mL of methylene chloride was added 90 mg of hexahydrofuro[2,3b]furan-3a-yl succinimidyl carbonate and 0.060 mL of triethyl amine. After stirring for 12 hours, the mixture was diluted with 50 mL of chloroform, washed with 10 mL of sat'd. sodium bicarbonate, and concentrated to dryness. Chromatography using 8% MeOH in $CHCl_3$ gave 80 mg of product as a white crystalline solid. mp 194-6°C :

| | |
|---|---|
| Calc'd for $C_{41}H_{51}N_3O_9$ | C, 67.47 H, 7.07 N, 5.76 |
| Found : | C, 67.45 H, 6.90 N, 5.77. |

EXAMPLE 37

Preparation of 1-(R)-hydroxymethyl-2-(S)-methoxytetrhydrofuran

Step A : Preparation of methyl-5-(t-butyldimethyl-silyl)-2-deoxy-erythropentofuranoside :

A mixture of methyl 2-deoxy-erthropentoside (24 g), imidazole (64 g), dimethylformamide (170 mL), and t-butyldimethylsilylchloride (28.5 g) were stirred for five hours at 0°C. The solvents were removed under reduced pressure and the residue shaken with ice cold 5% citric acid and ether (250 mL). The aqueous layer was extracted with two additional 250 mL portions of ether and the combined ethereal extracts washed with 10% citric acid, $H_2O$, $NaHCO_3$ (sat'd), NaCl (sat'd) and dried over $MgSO_4$. Concentration under reduced pressure gave 38 g of product (pale yellow oil) as a mixture of anomers. The product was contaminated with disilylated material which could be removed in subsequent steps. $^1H$ NMR ($CDCl_3$, 300 MHz) δ 3.38 (3H, s, $OCH_3$) and 3.34 (3H, s, $OCH_3$).

Step B : Preparation of methyl 3-0-methyl-2-deoxyerythropentofuranoside :

The product from Step A (2.04 g) was dissolved in THF (10 mL) cooled to 0°C and treated with methyl iodide (1.5 mL) and NaH (0.448 g 60% oil dispersion). The reaction mixture was aged overnight then poured into ice cold $H_2O$ and extracted with $Et_2O$ (3 x 50 mL). The combined organics were washed with NaCl (sat'd) and dried ($MgSO_4$). A solution of the crude oily product in 2 mL of THF and 9 mL of 1M tetrabutylammoniumfluoride in THF was aged in 25°C for 2 days. After concentration under reduced pressure, the residue was diluted with 75 mL of ethyl acetate and washed with 10% citric acid (2 x 50 mL), NaCl (sat'd), and dried ($Na_2SO_4$). Concentration under reduced pressure gave a yellow oil that was sufficiently pure to be used without further purification.

Step C : Preparation of 1-(R)-hydroxymethyl-2-(S)-methoxytetrahydrofuran :

The product of Step B (570 mg) was dissolved in $CH_2Cl_2$ (5 mL) and cooled to -40°C. To this solution were added triethylsilane (1.4 mL) and trimethylsilyltrifluoromethanesulfonate (0.83 mL). After one hour at -40°C, the mixture was aged further at -20°C for one hour. The reaction was quenched by addition of 1.1 mL of triethylamine, then diluted with 50 mL of $CH_2Cl_2$ and ice cold saturated $NaHCO_3$. The organic layer was dried ($Na_2SO_4$) and concentrated under reduced pressure. The oily residue was dissolved in ethanol containing a drop of concentrated HC1 and aged for 15 minutes. After concentration under reduced pressure the residue was purified by low pressure chromatography. Elution with 25% ethyl acetate in hexanes gave 225 mg of the title compound as a colorless oil.
$^1H$ NMR ($CDCl_3$, 300 MHz) δ 3.30 (3H, s, $OCH_3$).

EXAMPLE 38

Preparation of cis-4-aminothiochroman-4-ol

Step A : Preparation of 3-hydroxy thiochroman-4-one dimethylacetal :

A solution of thiochromanone (3.3 g) in 100 mL of absolute methanol was added to a stirred solution of potassium hydroxide (3.4 g) in 60 mL of methanol over a period of 10 minutes at 0°C. The stirring was continued and iodobenzene diacetate (7.2 g) was added in portions during a 10 minute period. The resulting mixture was then stirred for an additional hour at 0°C and then overnight at room temperature. The reaction mixture was concentrated under reduced pressure and partitioned between 120 mL of saturated potassium carbonate and ether (3 x 100 mL). The combined ether extracts were dried over anhydrous sodium sulfate and concentrated under reduced pressure. The product was an oil (3.6 g).

Step B : Preparation of 3-hydroxy thiochroman-4-one :

To a stirred solution of the product of Step A (3.6 g) in mL of ethanol was added 30 mL of aqueous 3N hydrochloric acid. After stirring for 45 minutes, 50 mL of water was added and the mixture stirred for an additional 45 minutes. The product was isolated by concentrating the mixture to a minimum volume and extracting with ether (3 x 100 mL). The combined ether extracts were washed with aqueous sodium bicarbonate, dried over

anhydrous sodium sulfate and concentrated under reduced pressure. The product (2.7 g) was obtained as a foam.

Step C : Preparation of O-benzyl 3-hydroxy thiochroman-4-one oxime ether :

To a stirred solution of the product of Step B (840 mg) in mL of pyridine at 0°C was added 840 mg of benzyloxyamine hydrochloride. The resulting mixture was allowed to warm to room temperature for 12 hours. The solvents were removed under pressure and the residue partitioned between 10 mL of aqueous sodium bicarbonate and ethyl acetate (3 × 100 mL). The combined extracts were dried over anhydrous sodium sulfate and concentrated under reduced pressure. Chromatography of the residue gave a colorless oil.

Step D : Preparation of cis-4-aminothiochroman-3-ol :

To a stirred solution of 1.2 g of O-benzyl oxime in 10 mL dry tetrahydrofuran at 0°C was added 12 mL of a 1 M solution of borane in THF such a rate that the temperature did not exceed 5°C. The resulting mixture was allowed to warm to room temperature for 12 hours. The solvents were removed under reduced pressure and the residue diluted with 5 mL of 10% aqueous possium hydroxide. The mixture was stirred at reflux for 1 hour, cooled to room temperature, and extracted with ether (3 × 100 mL). The combined extracts were dried over anhydrous sodium sulfate and concentrated under reduced pressure. Chromatography of this crude product gave 615 mg of product as a pale yellow solid : mp 86-88°C ; cis/trans ratio 14 :1 by :
$^1$H NMR : (300 MHz) δ 7.1 (m, 4H), 4.12 (m, 1H), 3.9 (m, 1H), 3.1-2.85 (m, 2H), 2.3 (br s, 3H).

EXAMPLE 39

Preparation of 4-amino-isothiochromane

Step A : Preparation of O-methyl isothiochroman-4-one oxime :

To a stirred solution of 1.08 g (6.57 mmol) of 1H-2-benzothiopyran-4(3H)-one (isothiochroman-4-one) in 10 mL of dry pyridine was added 0.55 g (6.57 mmol) of methoxylamine hydrochloride. The solution was stirred under an argon atmosphere for 2 hours, after which the solvent was removed in vacuo and the residue partitioned between EtOAc and $H_2O$. The aqueous phase was extracted twice more with EtOAc and the combined EtOAc layers were washed with 3 × 15 mL of IN HCl, then brine solution. After drying over $Na_2SO_4$ the solvent was removed, leaving 1.13 g of a red-brown oil. Chromatography on silica gel (25% EtOAc in hexane) gave 0.880 g of the title compound as colorless needles.
$^1$H NMR ($CDCl_3$) δ 3.71 (s, 2H), 3.82 (s, 2H), 4.02 (s, 3H), 7.12 (m, 1H), 7.25 (m, 2H), 7.98 (m, 1H).

Step B : Preparation of 4-amino-isothiochromane :

38.9 (0.21 mmol) of the product of Step 4 was dissolved in 300 mL of freshly distilled THF and the solution was added dropwise at 0°C to 604 mL of a 1M borane-THF solution. After 30 minutes at 0°C, the reaction mixture was allowed to warm to 25°C and stirring was continued for 36 hours at ambient temperature, or until completed by TLC (25/75 EtOAc/hexane). The mixture was cooled to 0°C and quenched by the dropwise addition of abs. $CH_3OH$. After stirring for 1 hour the mixture was concentrated, then treated with 150 mL of 3N HC1 and stirring was continued for 3 hours. The mixture was extracted with ether to remove unreacted starting material. The aqueous phase was basified with 40% NaOH to pH 10 and then extracted with 3 × 200 mL EtOAc. The combined EtOAc layers were washed with $H_2O$, brine ; dried ($Na_2SO_4$). Removal of the drying agent and evaporation of the solvent left 28.9 g of pure amine as a deep red oil.
$^1$H NMR ($CDCl_3$) δ 1.86 (br s, 2H), 2.79 (ddd, J = 1.4, 5, 13, H3, 1H), 3.14 (dd, J = 3.6, 13, H3, 1H), 3.61 (d, J = 16, H3, 1H), 3.92 (d, J = 16, H3, 1H), 4.10 (t, J = 4.2, H3, 1H), 7.1-7.4 (m, 4H).

EXAMPLE 40

Assay for inhibition of Microbial Expressed Viral Protease

Inhibition studies of the reaction of the protease expressed in Eschericia coli with a peptide substrate [Val-Ser-Gln-Asn-(betanapthyl)Ala-Pro-Ile-Val, 0.5 mg/mL at the time the reaction is initiated] were in 50 mM Na acetate, pH 5.5, at 30°C for 1 hour. Various concentrations of inhibitor in 1.0 ul DMSO were added to 25 ul of

the peptide solution in water. The reaction is initiated by the addition of 15 ul of 0.33 nM protease (0.11 ng) in a solution of 0.133 M Na acetate pH 5.5 and 0.26% bovine serum albumin. The reaction was quenched with 160 ul of 5% phosphoric acid. Products of the reaction were separated by HPLC (VYDAC wide pore 5 cm C-18 reverse phase, acetonitrile gradient, 0.1% phosphoric acid). The extent of inhibition of the reaction was determined from the peak heights of the products. HPLC of the products, independently synthesized, proved quantitation standards and confirmation of the product composition. The products of synthesis in Examples 1-18 inclusive showed $IC_{50}$ values in the range of 0.1-10 nM.

While the foregoing specification teaches the principles of the present invention, with examples provided for the purpose of illustration, it will be understood that the practice of the invention emcompasses all of the usual variations, adaptations, or modifications, as come within the scope of the following claims and its equivalents.

## Claims

1. A compound of the formula
A-G-B-B-J I,
wherein A is :
1) hydrogen ;

2) $R^1-\overset{O}{\overset{\|}{C}}-$ wherein $R^1$ is hydrogen, or

=

a) $C_{1-6}$ alkyl or $C_{1-6}$ alkenyl, either unsubstituted or substituted with one or more of
i) $C_{1-4}$ alkyl ;
ii) hydroxy ;
iii) carboxy ;
iv) halo wherein halo is F, Cl, Br, or I :
v) amino ;
vi) $C_{1-3}$ alkoxycarbonyl ;
vii) $C_{1-3}$ alkoxy unsubstituted or substituted with tetrahydropyran ;
viii) —$CONR^2R^3$ wherein $R^2$ and $R^3$ are the same or different and are hydrogen, $C_{1-5}$ alkyl or $C_{1-5}$ alkoxyalkyl or joined together either directly to form a 5-7 membered heterocycle such as pyrrolidinyl or piperidyl, or through a heteroatom selected from N, O, and S, to form a 6-membered heterocycle with the nitrogen to which they are attached such as morpholinyl, piperazinyl, or N-$C_{1-3}$ alkylpiperazinyl ;
ix) —$NR^2R^3$ ;

x) $-\underset{R}{\underset{|}{N}}-D-R^4$ wherein,

R is hydrogen or $C_{1-4}$ alkyl,

D is $-\overset{O}{\overset{\|}{C}}-$, $-\overset{S}{\overset{\|}{C}}-$, $-\underset{O}{\underset{\|}{\overset{O}{\overset{\|}{S}}}}-$, $-\overset{NR}{\overset{|}{C}}-$, and

$R^4$ is N, $C_{1-3}$ alkyl, $C_{1-4}$ alkoxy, or $NR^2R^3$ ;
xi) $C_{3-7}$ cycloalkyl or $C_{6-10}$ aryl ;
xii) 5 or 6 membered heterocycle, saturated or unsaturated, such as furyl, morpholino, or a 7-10 membered bicyclic hetercyclic ring, saturated or unsaturated, the ring or rings being unsubstituted or substituted with
i) oxo,

ii) —OH,
iii) NH, or
iv) $C_{1-4}$ alkyl ; or

xiii) aryl or carbocyclic of 6-10 carbon atoms, either unsubstituted or substituted with one or more of

(a) halo,
(b) hydroxy,
(c) $C_{1-3}$ alkoxy,
(d) $C_{1-3}$ alkyl,
(e) —$NR_2$, wherein R is defined above,

(f) $-\overset{O}{\overset{\|}{C}}OR$,

(g) $-\overset{O}{\overset{\|}{C}}NR_2$,

(h) —$SO_2NR_2$,
(i) —$CH_2NR_2$,

(j) $-\underset{R}{\underset{|}{N}}-\overset{O}{\overset{\|}{C}}-R$, or

(k) $-\underset{R}{\underset{|}{N}}-SO_2R$;

xiv) —O-$[(CH_2)_mO]_n$-R, where m is 2-5 and n is 0-5 ;

b) aryl of 6-10 carbon atoms, either unsubstituted or substituted with one or more of

i) $C_{1-4}$ alkyl,
ii) $C_{1-3}$ alkoxy,
iii) hydroxy, or
iv) halo ;
v) —$NR_2$,

vi) $-\overset{O}{\overset{\|}{C}}OR$,

vii) $-\overset{O}{\overset{\|}{C}}NR_2$,

viii) —$SO_2NR_2$,
ix) —$CH_2NR_2$,
x) —NRCOR, or
xi) —$NRSO_2R$ ;

c) 5 or 6 membered heterocycle ;

3) $R^1$-$SO_2$—,

4) 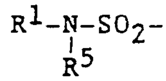 wherein

$R^5$ is H or $C_{1-5}$ alkyl or joined together with $R^1$ either directly to form 5-7 membered heterocycle such as pyrrolidinyl or piperidinyl, or through a heteroatom selected from N, O, and S, to form a 6-membered heterocycle with the nitrogen to which they are attached such as morpholinyl, piperazinyl, or N-$C_{1-3}$ alkyl- piperazinyl ;

5) 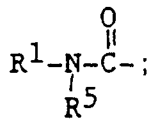

6) 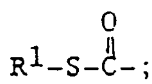

7) 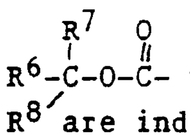 wherein $R^6$, $R^7$, and $R^8$ are independently

a) H,

b) $C_{1-8}$ alkyl unsubstituted or substituted with one or more of

i) halo,

ii) OH

iii) aryl $SO_2$—

iv) —O-[$(CH_2)_mO]_n$-R,

c) Aryl unsubstituted or substituted with one or more of

i) $C_{1-4}$ alkyl,

ii) $C_{1-3}$ alkoxy,

iii) halo,

iv) nitro,

v) acetoxy,

vi) dimethylaminocarbonyl,

vii) phenyl,

viii) $C_{1-3}$ alkoxycarbonyl ;

d) fluorenyl,

e) $R^6$, $R^7$, and $R^8$ may be independently joined to form a monocyclic, bicyclic, or tricyclic ring system which is $C_{3-10}$ cycloalkyl e.g. adamantyl, cyclopentyl, or cyclohexyl, and may be substituted with $C_{1-4}$ alkyl,

f) a 5-7 membered heterocyclic ring, saturated or unsaturated, such as pyridyl, furyl, 1,3-dioxolane, or tetrahydrofuryl ; or a 7-10 membered bicyclic heterocyclic ring, saturated or unsaturated, such as benzisoxazolyl, 4H-1,3-benzodioxin, the ring or rings being unsubstituted or substituted with $C_{1-4}$ alkyl ;

g) $R^6$, $R^7$ and $R^8$ may be independently joined to form a 5-7 membered heterocyclic ring, or 7-10 membered bicyclic heterocyclic ring, saturated or unsaturated, unsubstituted or substituted with one

or more of

i) acetyloxy,
i) $C_{1-4}$ alkyl,
iii) hydroxy,
iv) hydroxy $C_{1-4}$ alkyl ;
v) alkyloxy alkyl,
vi) phenyl alkoxy, or
vii) alkoxy ;

8) 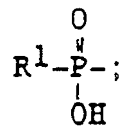

G is

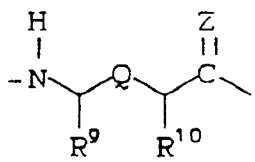 or 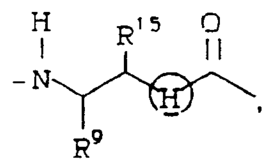,

wherein Z is O, S, or HH and

$R^9$ and $R^{10}$ are independently,

a) —$(CH_2)n$-$R^{11}$,
b) $C_{1-4}$alkenyl —$R^{11}$ ;

wherein n is 0-5 ; and either or both $R^9$ and $R^{10}$ must be substituted with $R^{12}$ ;

$R^{11}$ is

a) hydrogen ;
b) $C_{1-4}$ alkyl ;
c) $C_5$-$C_{10}$ cycloalkyl, optionally substituted with hydroxy ;
d) $C_6$-$C_{10}$ aryl, unsubstituted or substituted with $R^{12}$, and optionally substituted with one or more of
  i) halo,
  ii) $C_{1-4}$alkyl,
  iii) $C_{1-3}$ alkoxy
  iv) hydroxy ;
e) monocyclic or bicyclic heterocyle containing from 1 to 3 heteroatoms chosen from N, O, S and which is unsubstituted or substituted with $R^{12}$ and optionally with one or more of
  i) halo,
  ii) $C_{1-4}$ alkyl,
  iii) $C_{1-3}$ alkoxy ;

$R^{12}$ is

a) —X-$(CH_2)_m$--$NR^{13}R^{14}$ where X is —O—, —S—, or —NH—, m is 2-5, and $R^{13}$ and $R^{14}$ are independently hydrogen or
  i) $C_{1-6}$ alkyl,
  ii) $C_{1-6}$ alkyl substituted with one or more of
    (a) $C_{1-3}$ alkoxy,
    (b) —OH,
    (c) —$NR_2$ wherein R is hydrogen or $C_{1-4}$ alkyl ;
  iii) the same or different and joined together to form a 5-7 member heterocycle, such as morpholino, containing up to two additional heteroatoms selected from —NR,

$-O-$, $-S-$, $-\overset{O}{\overset{\|}{S}}-$, or $-SO_2-$, said heterocycle optionally substituted with C1-4 alkyl ;

iv) aromatic heterocycle unsubstituted or substituted with one or more of

(a) $C_{1-4}$ alkyl,

(b) $—NR_2$,

b) $—(CH_2)_q–NR^{13}R^{14}$ wherein q is 1-5, and $R^{13}$ and $R^{14}$ are defined above, except that $R^{13}$ or $R^{14}$ are not H or $C_{1-6}$ alkyl ;

c) benzofuryl, indolyl, azacycloalkyl, azabicyclo $C_{7-11}$ cycloalkyl, or benzopiperidinyl, substituted or unsubstituted with $C_{1-4}$ alkyl ;

$R^{15}$ is $-OH$ or $-NHR^{16}$, wherein $R^{16}$ is $-H$, $-\overset{O}{\overset{\|}{C}}H$, $-C_{1-4}$-alkyl, or $-COOR$; and

Ⓗ is

1) $C_{3-7}$ cycloalkyl either unsubstituted or substituted with one or more of

a) $C_{1-4}$ alkyl,

b) hydroxy,

c) $—NR_2$,

d) $—COOR$,

e) $—CONHR$,

f) $—NHSO_2R$,

g) $-NH\overset{O}{\overset{\|}{C}}R$.

h) aryl,

i) aryl substituted with $C_{1-4}$alkyl,

j) heterocycle, or

k) heterocycle substituted with $C_{1-4}$alkyl ;

Q is

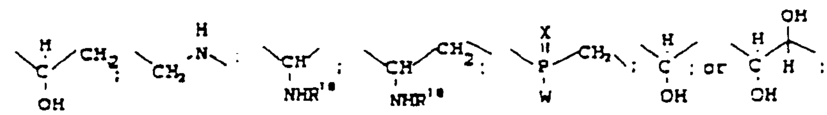

wherein $R^{16}$ is defined above ;

X is O, S or NH ; and

W is

1) OH,

2) $NH_2$,

3) OR, or

4) NHR ;

B is, independently, absent, or 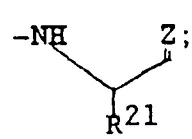

where $R^{21}$ is

a) $—CH(CH_3)_2$

b) $—CH(CH_3)(CH_2CH_3)$

c) —Phenyl ;

J is

1) $YR^{17}$ wherein :

Y is 0 or NH, and

$R^{17}$ is

a) H ;

b) $C_{1-6}$ alkyl, unsubstituted or substituted with one or more of

i) $—NR_2$,

ii) —OR,

iii) $—NHSO_2C_{1-4}$ alkyl,

iv) $—NHSO_2$ aryl, or $—NHSO_2$ (dialkyl-aminoaryl),

v) $—CH_2OR$,

vi) $—C_{1-4}$ alkyl,

vii) 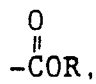

viii) 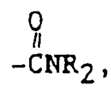

ix) 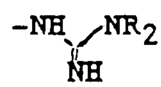 or 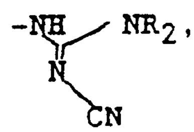

x) 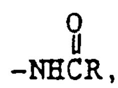

xi) 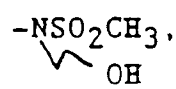

xii) 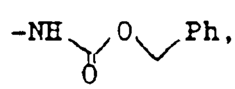

xiii) $—NR_3⊕ A⊖$ wherein $A⊖$ is a counterion,

xiv) $—NR^{18}R^{19}$ wherein $R^{18}$ and $R^{19}$ are the same or different and are $C_{1-5}$ alkyl joined together directly to form a 5-7 membered heterocycle containing up to one additional heteroatom selected from —O—, —S—, or —NR—,

xv) aryl,

xvi) —CHO,

xvii) $—OP(O)(OR_x)_2$ wherein $R_x$ is H or aryl, or

xvii) 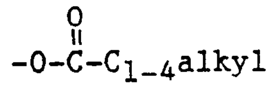 substituted with

one or more of amine or quaternary amine, or —O-[$(CH_2)_mO]_n$-R, or —$OP(O)(OR_x)_2$ ;

c) —$[(CH_2)_mO]_nCH_3$ or —$[(CH_2)_mO]_n$ H ;

2) $N(R^{17})_2$ ; or

3) —$NR^{18}R^{19}$ wherein $R^{18}$ and $R^{19}$ are defined above ;

4) 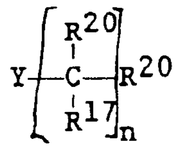

wherein :

Y, $R^{17}$ and n are defined above, and

$R^{20}$ is

a) hydrogen ;

b) aryl unsubstituted or substituted with one or more of

i) halo,

ii) —OR, wherein R is H or $C_{1-4}$ alkyl,

iii) 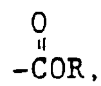

=

iv) 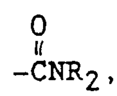

v) —$CH_2NR_2$,

vi) —$SO_2NR_2$,

vii) —$NR_2$,

viii) 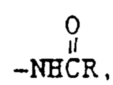

xi) $C_{1-4}$ alkyl,

x) phenyl

xi) —$CF_3$,

xii) 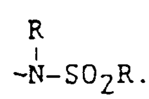

xiii) —$C_{1-4}$ alkyl —$NR_2$,

xiv) —$OP(O)(OR_x)_2$ wherein $R_x$ is H or aryl, or

xv) 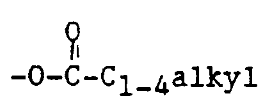 substituted with

one or more of amine or quaternary amine or $—OP(O)(OR_x)_2$ ;

c) A 5-7 membered heterocyclic ring or, 7-10 membered bicyclic heterocyclic ring which is saturated or unsaturated, such as isochroman, chroman, isothiochroman, thiochroman, benzimidazole, benzothiopyran, oxobenzothiopyran, benzopyran, benzothiopyranylsulfone, benzothiopyranylsulfoxide, the ring or rings being unsubstituted or substituted with one or more of

i) halo,

ii) —OR, wherein R is H, $C_{1-4}$alkyl, or $C_{1-4}$alkenyl,

iii) $-\overset{\overset{O}{\|}}{C}OR$,

iv) $-\overset{\overset{O}{\|}}{C}NR_2$,

v) $—CH_2NR_2$,

vi) $—SO_2NR_2$,

vii) $—NR_2$,

viii) $-NH\overset{\overset{O}{\|}}{C}R$,

xi) $C_{1-4}$ alkyl,

x) phenyl

xi) $—CF_3$,

xii) $-\overset{\overset{R}{|}}{N}-SO_2R$,

xiii) phenyl $C_{1-4}$ alkyl,

xiv) $—OP(O)(OR_x)_2$ wherein $R_x$ is H or aryl,

xv) $-O-\overset{\overset{O}{\|}}{C}-C_{1-4}$alkyl substituted with

one or more of amine or quaternary amine, or $—OP(O)(OR_x)_2$, or $—O[(CH_2)_mO]_n$-R, or

xvi) $-O-\overset{\overset{O}{\|}}{C}-O-[(CH_2)_mO]_n-R$;

d) A 5 to 7 membered carbocyclic or 7-10 membered bicyclic carbocyclic ring which is either saturated or unsaturated, such as cyclopentane, cyclohexane, indane, norbornane, or naphthalene, the carbocyclic ring being unsubstituted or substituted with one or more of

i) halo,

ii) —OR, or $—CH_2OR$, wherein R is H or $C_{1-4}$ alkyl,

iii) $-\overset{\overset{O}{\|}}{C}OR^{21}$, wherein $R^{21}$ is H,

$-(CH_2)_n-NR_2$, $C_{1-16}$alkyl, pyridine,

$$-(CH_2)_nNR-(CH_2)_n-NR_2,\quad -(CH_2)_n-\overset{O}{\overset{\|}{C}}-OR,$$

$-[(CH_2)_mO]_n-R$, quinuclidiniumyl substituted with R, piperazine-$C_{1-4}$alkyl-benzyl substituted once or more with R, or morpholinoalkyl-benzyl,

$$\text{iv)}\quad -\underset{\underset{O}{\|}}{C}NR_2,$$

v) $-CH_2NR_2$,
vi) $-SO_2NR_2$,
vii) $-NR_2$,

$$\text{viii)}\quad -NH\overset{O}{\overset{\|}{C}}R,$$

xi) $C_{1-4}$ alkyl,
x) phenyl,
xi) $-CF_3$,

$$\text{xii)}\quad -\overset{R}{\overset{\backslash}{N}}-SO_2R,$$

xiii) $-OP(O)(OR_x)_2$ wherein $R_x$ is H or aryl,

$$\text{xiv)}\quad -O-\overset{O}{\overset{\|}{C}}-C_{1-4}\text{alkyl substituted with}$$

one or more of amine or quaternary amine, $-OP(O)(OR_x)_2$, or $-O-[(CH_2)_mO]_n-R$, or

$$\text{xv)}\quad -O-\overset{O}{\overset{\|}{C}}-O-[(CH_2)_mO]_n-R;$$

or pharmaceutically acceptable salts thereof.

2. A compound of Claim 1, wherein

A is 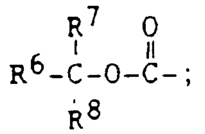 and G is 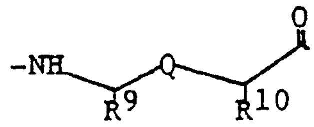

3. A compound of Claim 2, wherein B is absent or present once and Q is

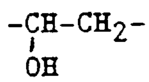

4. A compound of Claim 3, wherein B is absent and J is NH-$R^{20}$ wherein $R^{20}$ is a substituted 7 to 10 membered bicyclic carbocyclic or heterocyclic ring which is either saturated or unsaturated.

5. A compound, which is
N-(2-(R)-hydroxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2-(R)-hydroxy-1(S)-indanyl)-5(S)-((2-tetrahydrofuranyl)carbonylamino)-4(S)-hydroxy-6-phenyl-2(R)-((4-(2-(4-morpholinyl)ethoxy)phenyl)-methyl)hexanamide,
N-(2-(R)-hydroxy-1(S)-indanyl)-5(S)-(2-hydroxyethoxycarbonylamino)-4(S)-hydroxy-6-phenyl-2(R)-((4-(2-(4-morpholinyl)ethoxy)phenyl)methyl)hexanamide,
N-(2-(R)-hydroxy-1(S)-indanyl)-5(S)-(1-methylethoxycarbonylamino)-4(S)-hydroxy-6-phenyl-2(R)-((4-(2-(4-morpholinyl)ethoxy)phenyl)methyl)hexanamide,
N-(2-(R)-hydroxy-1(S)-indanyl)-5(S)-(ethoxycarbonylamino)-4(S)-hydroxy-6-phenyl-2(R)-((4-(2-(4-morpholinyl)ethoxy)phenyl)methyl)hexanamide.
N-(2-(R)-hydroxy-1(S)-indanyl)-5(S)-(1,1-dimethylethoxycarbonylamino)-4(S)-hydroxy-6-(4-(2-(4-morpholinyl)ethoxy)phenyl)-2(R)-(3′-phenylprop-2-′en-1-yl)-hexanamide,
N-(2-benzamidolylmethyl)-N-5(S)-(1,1-dimethylethoxycarbonylamino)-4(S)-hydroxy-6-(4-(2-(4-morpholinyl)ethoxy)phenyl)-2(R)-(3′-phenylprop-2-′en-1-yl)-hexanoyl isoleucyl amide,
N-(2-benzamidolylmethyl)-N′-5(S)-(1,1-dimethylethoxycarbonylamino)-4(S)-hydroxy-6-phenyl-2(R)-(4-(2-(4-morpholinyl)ethoxy)phenyl)-hexanoyl isoleucyl amide,
N-((pyrrolo[3,2-b]pyridin-2-yl)methyl)-N′-5(S)-(1,1-dimethylethoxycarbonylamino)-4(S)-hydroxy-6-phenyl-2(R)-(4-(2-(4-morpholinyl)ethoxy)phenyl)-hexanoyl isoleucyl amide,
N-((5-(2-morpholinylethyl)pyrrolo[3,2-b]pyridin-2-yl) methyl)-N′-5(S)-(1,1-dimethylethoxycarbonylamino)-4(S)-hydroxy-6-phenyl-2(R)-(4-(2-(4-morpholinyl)ethoxy)phenyl)-hexanoyl isoleucyl amide,
N-(2(R)-hydroxy-1(S)-indanyl)-5(S)-(1,1-dimethylethoxycarbonylamino)-4(S)-hydroxy-6-phenyl-2(R)-((3-(2-(4-morpholinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-(4-(2-(4-morpholinyl)ethoxy)phenyl)-2(R)-phenylmethylhexanamide,
N-(2(R)-hydroxy-1(S)-indanyl)-5(S)-(1,1-dimethylethoxycarbonylamino)-4(S)-hydroxy-6-(4-(2-(4-morpholino)ethoxy)phenyl)-2(R)-((4-(2-(4-morpholinyl)ethoxy)phenyl)methyl)hexanamide,
N-(2(S)-hydroxy-1(S)-benzopyranyl)-5(S)-[1,1-dimethylethoxycarbonylamino)-4(S)-hydroxy-6-(4-(2-(4-morpholino)ethoxy)phenyl)-2(R)-((4-(2-(4-morpholinyl)ethoxy)phenyl)methyl)hexanamide,
N-(2(S)-hydroxy-1(S)-benzopyranyl)-5(S)-[1,1-dimethylethoxycarbonylamino)-4(S)-hydroxy-6-(4-(2-(4-morpholinyl)ethoxy)phenyl)-2(R)-((4-hydroxyphenyl)-methyl)hexanamide,
N-(2(S),3-(R)-dihydroxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(S),3(R)-dihydroxy-1(S)-indanyl)-5(S)-(1,1-dimethylethoxycarbonylamino)-4(S)-hydroxy-6-phenyl-2(R)-((3-(2-(4-morpholinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-(4-(2-(4-morpholinyl)ethoxy)phenyl)-2(R)-((4-(2-(4-morpholinyl) ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(4-morpholinyl)propyloxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-dimethylaminoethoxy)phenyl)methyl)hexanamide,

N-(2(R)-hydroxy-1(S)-indanyl)-5(S)-(1,1-dimethylethoxycarbonylamino)-4(S)-hydroxy-6-phenyl-2(R)-((4-(2-(1-piperidinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(1-pyrrolidinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(N,N-bis(2-methoxyethyl)amino)ethoxy) phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(thiamorpholin-4-yl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-(4-(2-(4-(1-oxothiamorpholin-4-yl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-(4-(2-(4-(1,1-dioxythiamorpholin-4-yl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-(4-(2-(1-(4-methylpiperazin-1-yl)ethoxy)phenyl)methyl)hexanamide,
N-(3(S)-hydroxy-4(S)-benzopyranyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(S)-hydroxy-1(S)-benzothiopyranyl)-5(S)-(1,1-dimethylethoxycarbonylamino)-4(S)-hydroxy-6-ph enyl-2(R)-((4-(2-(4-morpholinyl)ethloxy)phenyl)methyl)hexanamide,
N-(2(R)-dimethylaminoacetoxy-1(S)-indanyl)-5(S)-(1,1-dimethylethoxycarbonylamino)-4(S)-hydroxy-6 -phenyl-2(R)-((4-(2-(4-morpholinyl)ethoxy)phenyl)methyl)hexanamide,
N-(2(R)-acetoxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(S),3(R)-diacetoxy-1(S)-indanyl)-5(S)-(1,1-dimethylethoxycarbonylamino)-4(S)-hydroxy-6-pheny l-2(R)-((4-(2-(4-morpholinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(4)-(2-methoxyethylamino)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-(3-(3-(2-(4-morpholinyl)ethoxy)phenyl)prop-2-en-1-yl)hexanamide,
N-(2(R)-hydroxy-1(S)-indanyl-5(S)-(1,1-dimethylethoxycarbonylamino)-4(S)-hydroxy-6-phenyl-2(R)-( 3-(4-(2-(4-morpholinyl)ethoxy)phenyl)prop-2-en-1-yl)hexanamide,
N-(5-(2-(4-morpholinyl)ethoxy)-2-benzimidazolyl-methyl))-5(S)-(1,1-dimethylethoxycarbonylamino)-4 (S)-hydroxy-6-phenyl-2(R)-((4-(2-(4-morpholinyl)ethoxy)phenyl)methyl)hexanoyl isoleucylamide.
N-((5-(2-(4-morpholinyl)ethoxy)benzimidazolylmethyl))-5(S)-(1,1-dimethylethoxycarbonylamino)-4(S) -hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(2-hydroxyethylamino)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(2-methylpropylamino)ethoxy)phenyl)methyl)hexanamide,
N-(3(R)-amino-2(R)-hydroxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(R)-((3-pyridinecarbonyloxy)-1(S)-indanyl))-5(S)-(1,1-dimethylethoxycarbonylamino)-4(S)-hydro xy-6-phenyl-2(R)-((4-(2-(4-morpholinyl)ethoxy)phenyl)methyl)hexanamide,
N-(2(R)-n-hexadecanoyloxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2-(R)-hydroxy-1(S)-indanyl)-5(S)-(1,1-dimethylethoxycarbonylamino)-4(S)-hydroxy-6-phenyl-2(R) -((4(2-(3,5-dimethylmorpholin-4-yl)ethoxy)phenyl)methyl)hexanamide,
N-(2-(R)-hydroxy-1(S)-indanyl)-5(S)-(1,1-dimethylethoxycarbonylamino)-4(S)-hydroxy-6-phenyl-2(R) -((4-(2-(2,6-dimethylmorpholin-4-yl)ethoxy)phenyl)methyl)hexanamide,
N-(2-(R)-hydroxy-1(S)-indanyl)-5(S)-(1,1-dimethylethoxycarbonylamino)-4(S)-hydroxy-6-phenyl-2(R) -((4-(2-(2,2,6,6-tetramethylpiperidin-4-yl)-amino-ethoxy)phenyl)methyl)hexanamide,
N-(2-(R)-hydroxy-1(S)-indanyl)-5(S)-(1,1-dimethylethoxycarbonylamino)-4(S)-hydroxy-6-phenyl-2(R) -((4-(3-(4-morpholinyl)propyl)phenyl)methyl)hexanamide,
N-(2-(R)-hydroxy-1(S)-indanyl)-5(S)-(1,1-dimethylethoxycarbonylamino)-4(S)-hydroxy-6-phenyl-2(R) -((4-(4-morpholinyl)methylphenyl)methyl hexanamide,
N-(2-(R)-hydroxy-1(S)-indanyl)-5(S)-(1,1-dimethylethoxycarbonylamino)-4(S)-hydroxy-6-phenyl-2(R) -((4-(3-(bis-(2-methoxyethyl)amino)propyl)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(3-(1-thiamorpholin-4-yl)propyl)phenyl)methyl)hexanamide,

N-(2(R)-hydroxy-1(S)-indanyl)-5(S)-(1,1-dimethylethoxycarbonylamino)-4(S)-hydroxy-6-phenyl-2(R)-((4-(2-(1-imidazolyl)ethoxy)phenyl)methyl)hexanamide,
N-(3(R)-hydroxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-((1,2-dimethyl-2-hydroxyethyl)phosphoroamidato-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(4-morpholinyl)-2-oxoethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(3-dimethylaminopropyl)phenyl)methyl)hexanamide,
N-(2(R)-hydroxy-1(S)-indanyl)-5(S)-(1-adamantyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(3-oxomorpholin-4-yl)ethoxy)phenyl)methyl)hexanamide,
N-(2(R)-hydroxy-1(S)-indanyl)-5(S)-((1,1-dimethylethoxycarbonyl)-Ala)-amino-4(S)-hydroxy-6-phenyl-2(R)-((4-(2-(4-morpholinyl)ethoxy)phenyl)methyl)hexanamide,
N-(2-(R)-hydroxy-1(S)-indanyl)-5(S)-(Ala-amino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(4-pyridinylamino)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(1-triazolyl)ethoxy)phenyl)methyl)hexanamide,
N-(2-(R)-(8-((2-dimethylaminoethyl)ethylamino)-8-oxooctanoyloxy)-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide.
N-(2-(R)-hydroxy-1(S)-indanyl-5(S)-(2,2-dimethyl-1-propyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1,2-trimethyl-1-propyloxycarbonylamino)-4(S)-hydroxy-6-phenyl-2(R)-((4-(2-(4-morpholinyl)ethoxy)phenyl)methyl)hexanamide,
N-(2-(R)-hydroxy-1(S)-indanyl)-5(S)-(1,1-dimethyl-1-propyloxycarbonylamino)-4(S)-hydroxy-6-phenyl-2(R)((4-(2-(4-morpholinyl)ethoxy)phenyl)methyl)hexanamide,
N-(2(R)-hydroxy-1(S)-indanyl)-5(S)-(cyclohexyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(cyclopentyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(cycloheptyloxycarbonylamino)-4(S)-hydroxy-6-phenyl-2(R)-((4-(2-(4-morpholinyl)ethoxy)phenyl)methyl)hexanamide,
N-(2-(R)-hydroxy-1(S)-indanyl)-5(S)-(1-methyl-1-propyloxycarbonylamino)-4(S)-hydroxy-6-phenyl-2(R)-((4-(2-(4-morpholinyl)ethoxy)phenyl)methyl)hexanamide,
N-(2-(R)-hydroxy-1(S)-indanyl)-5(S)-(1,1-dimethyl-2-hydroxyethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethyl-2-methoxyethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethyl-2-(2-methoxyethoxy)ethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethyl-2-(2-(2-methoxyethoxy)ethoxy)ethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(3-methyl-3-pentyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(3-ethyl-3-pentyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1-bicyclo[2,2,2]octyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1-bicyclo[2,2,1]heptyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(methoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-((2-tetrahydrofuranyl)methoxycarbonylamino)-4(S)-hydroxy-6-ph

enyl-2(R)-((4-(2-(4-morpholinyl)ethoxy)phenyl)methyl)hexanamide,
N-(2(R)-hydroxy-1(S)-indanyl)-5(S)-((3-tetrahydrofuranyl)methoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1-methyl-1-(2-tetrahydrofuranyl)ethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1-methyl-1-(3-tetrahydrofuranyl)methoxycarbonylamino)-4(S)-hydroxy-6 -phenyl-2(R)-((4-(2-(4-morpholinyl)ethoxy)phenyl)methyl)hexanamide,
N-(2(R)-hydroxy-1(S)-indanyl)-5(S)-(3-tetrahydrofuranyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(3-tetrahydropyranyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(4-tetrahydropyranyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-((2-tetrahydropyranyl)methoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(2-methoxyacetylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(2-(2-methoxyethoxy)acetylamino)-4(S)-hydroxy-6-phenyl-2(R)-(4-(2-(4-morpholinyl)ethoxy)phenyl)methyl)hexanamide,
N-(2(R)-hydroxy-1(S)-indanyl)-5(S)-(2-(2-(2-methoxyethoxy)ethoxy)acetylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-((2(R)-tetrahydrofuranyl)carbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-((2(S)-tetrahydrofuranyl)carbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-((3-tetrahydrofuranyl)carbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(2-methylpropyloxycarbonylamino)-4(S)-hydroxy-6-phenyl-2(R)-(4-(2-(4-morpholinyl)ethoxy)phenyl)methyl)hexanamide,
N-(2(R)-hydroxy-1(S)-indanyl)-5(S)-(2-methoxyethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(2-(2-methoxyethoxy)ethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(2-(2-(2-methoxyethoxy)ethoxy)ethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(2-oxomorpholin-4-yl)ethoxy)phenyl)methyl)hexanamide,
N-(2(R)-hydroxy-1(S)-indanyl)-5(S)-(1,1-dimethylethoxycarbonyl-N-methylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(4-morpholinyl)ethylthio)phenyl)methyl)hexanamide,
N-(2(R)-hydroxy-1(S)-indanyl)-5(S)-((carbobenzyloxy)amino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(4-pyridylmethyloxycarbonylamino)-4(S)-hydroxy-6-phenyl-2(R)-(4-(2-(4-morpholinyl)ethoxy)phenyl)methyl)hexanamide,
N-(2(R)-hydroxy-1(S)-indanyl)-5(S)-(n-propyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(n-butyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(n-pentyloxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethylethoxycarbonylamino)-3(S),4(R)-dihydroxy-6-phenyl-2(R)-((4-(2-(4-morpholinyl)ethoxy)phenyl)methyl)hexanamide,
N-(4(R)-hydroxy-2(R)-methyl-1(R)-cyclopentyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(4(R)-hydroxy-2(S)-methyl-1(R)-cyclopentyl)-5(S)-(1,1-dimethylethoxycarbonylamino)-4(S)-hydrox

y-6-phenyl-2(R)-((4-(2-(4-morpholinyl)ethoxy)phenyl)methyl)hexanamide,
N-(2(R)-hydroxy-5(R)-methyl-1(S)-cyclopentyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(R)-hydroxy-5(S)-methyl-1(S)-cyclopentyl)-5(S)-(1,1-dimethylethoxycarbonylamino)-4(S)-hydroxy-6-phenyl-2(R)-((4-(2-(4-morpholinyl)ethoxy)phenyl)methyl)hexanamide.
N-(2(S),3(R)-dihydroxy-5(R)-methyl-1(S)-cyclopentyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(S),3(R)-dihydroxy-5(S)-methyl-1(S)-cyclopentyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(3(S)-hydroxy-4(S)-thiochromanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(1(S)-oxo-4(R)-thiochromanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-bicyclo[4,3,0]nonyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(1(R,S)-oxo-4(S)-isothiochromanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethylethoxycarbonyl)amino-4(S)-hydroxy-6-phenyl-2(R)-[4-(2-(4-azabicyclo[2.2.2]octanyl-1-aminiumyl)ethoxy)phenylmethylhexanamide,
N-[(1-hydroxycyclohexyl)methyl]-5(S)-t-butoxycarbonylamino-4(S)-hydroxy-6-phenyl-2(R)-(4-[2-morpholinoethoxy]phenyl)methylhexanamide,
N-(3(S)-amino-2(R)-hydroxy-1(S)-indanyl)-5(S)-t-butoxycarbonylamino-4(S)-hydroxy-6-phenyl-2(R)-(4-[2-morpholinoethoxy]phenyl)methylhexanamide,
N-(2(R)-hydroxy-3A,4,5,6,7,7A-hexahydro-indan-1(S)-yl)-5(S)-(1,1-dimethylethoxycarbonylamino)-4(S)-hydroxy-6-phenyl-2(R)-(4-(2-(4-morpholinyl)ethoxy)phenyl)methylhexanamide,
N-(2(R)-((3-carboxypropanoyl)oxy)-1(S)-indanyl)-5(S)-(1,1-dimethylethoxycarbonylamino)-4(S)-hydroxy-6-phenyl-2(R)-((4-(2-(4-morpholinyl)ethoxy)phenyl)methylhexanamide,
N-(2(R)-hydroxy-1(S)-indanyl)-5(S)-succinoylamino-4(S)-hydroxy-6-phenyl-2(R)-[(4-(2-morpholinoethoxy)phenyl)methyl]hexanamide,
N-(2(R)-[(2-(2-(2-methoxyethoxy)ethoxy)ethoxy)carbonyl]oxy)-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(4-tetrahydropyranylcarbonylamino)-4(S)-hydroxy-6-phenyl-2(R)-((4-(2-(4-morpholinyl)ethoxy)phenyl)methylhexanamide,
N-(2(R)-hydroxy-1(S)-indanyl)-5(S)-((4-tetrahydropyranyl)acetamido)-4(S)-hydroxy-6-phenyl-2(R)-((4-(2-(4-morpholinyl)ethoxy)phenyl)methylhexanamide,
N-[(cis-2(R)-(1′-methyl-3′-(S)-quinuclidiniumylcarbonyl)oxy-(1(S)-indanyl)-5(S)-(1,1-dimethylethoxycarbonylamino)-4(S)-hydroxy-6-phenyl-2(R)-[4-(2-morpholinoethoxy)phenyl)methyl]hexanamide,
N-(2(R)-hydroxy-1(S)-indanyl)-5(S)-acetylamino-4(S)-hydroxy-6-phenyl-2(R)-(4-(2-(4-morpholinyl)ethoxy)phenyl)hexanamide,
N-(cis-2(R)-[4-(4-methyl-1-piperazinylmethyl)-benzoyl]oxy-1(S)-indanyl]-5(S)-(1,1-dimethylethoxycarbonylamino)-4(S)-hydroxy-6-phenyl-2(R)-[4-(2-morpholinoethoxy)phenyl]methylhexanamide,
N-(cis-2(R)-hydroxy-1(S)-indanyl)-5(S)-[2(S)-(1,1-dimethylethoxycarbonylamino)-4-hydroxybutyrylamino]-4(S)-hydroxy-6-phenyl-2(R)-[(4-(2-morpholinoethoxy)-phenyl)methyl]hexanamide,
N-(cis-(2R)-hydroxy-1(S)-indanyl)-5(S)-[(5-oxo-2(S)-tetrahydrofuranyl)carbonylamino]-4(S)-hydroxy-6-phenyl-2(R)-[4-(2-morpholinoethoxy)phenyl]methylhexanamide,
N-(cis-2(R)-hydroxy-1(S)-indanyl)-5(S)-[2(S)-(amino-4-hydroxybutyryl)amino]-4(S)-hydroxy-6-phenyl-2(R)-[(4-(2-morpholinoethoxy)phenyl)methyl]hexanamide.
N-(2(R)-[(4-(morpholinomethyl)benzoyl)oxy]-1(S)-indanyl)-5(S)-(1,1-dimethylethoxycarbonylamino)-4(S)-hydroxy-6-phenyl-2(R)-[(4-(2-morpholinoethoxy)phenyl)methyl]hexanamide,
N-(cis-2(R)-hydroxy-1(S)-indanyl)-5(S)-[1-trans-(2-hydroxyindanylidenyl)carbonyl]amino-4(S)-hydroxy-6-phenyl-2(R)-[(4-(2-morpholinoethoxy)phenyl)methyl]hexanamide,
N-(cis-2(R)-hydroxy-1(S)-indanyl)-5(S)-[1-cis-(2-hydroxyindanylidenyl)carbonyl]amino-4(S)-hydroxy-6-phenyl-2(R)-[(4-(2-morphoLinoethoxy)phenyl)methyl]hexanamide,
N-(cis-2(R)-hydroxy-1(S)-indanyl)-5(S)-[(2-hydroxy-1-indanylmethyl)carbonyl]amino-4(S)-hydroxy-6-phenyl-2(R)-[(4-(2-morpholinoethoxy)phenyl)methyl]hexanamide,
N-(cis-2(R)-hydroxy-1(S)-indanyl)-5(S)-[(4-morpholinyl)carbonyl]amino-4(S)-hydroxy-6-phenyl-2(R)-[(4-(2-morpholinoethoxy)phenyl)methyl]hexanamide,

N-(2(R)-hydroxy-1(S)-indanyl)-5(S)-(3(R)-tetrahydrofuranyloxycarbonylamino)-4(S)-hydroxy-6-pheny l-2(R)-(4-(2-(4-morpholinyl)ethoxy)phenyl)methylhexanamide,
N-(cis-2(S)-hydroxy-1(S)-benzopyranyl)-5(S)-(2-methoxyethoxycarbonylamino-4(S)-hydroxy-6-phenyl -2(R)-[(4-(2-morpholinoethoxy)phenyl)methyl]hexanamide,
N-(cis-2(S)-hydroxy-1(S)-benzopyranyl)-5(S)-(3,6,9,12-tetraoxatridecyloxycarbonylamino)-4(S)-hydr oxy-6-phenyl-2(R)-[(4-(2-morpholinoethoxy)phenyl)methyl]hexanamide,
N-(2(R)-hydroxy-1(S)-indanyl)-5(S)-(hexahydrofuro[2,3-b]furanyl-3A-oxycarbonylamino)-4(S)-hydrox y-6-phenyl-2(R)-(4-(2-(4-morpholinyl)ethoxy)phenyl)methylhexanamide,
N-(cis-2(R)-hydroxy-1(S)-indanyl)-5(S)-[(2,2-dimethyl-1,3-dioxolane-4-methyloxy)carbonyl]amino-4( S)-hydroxy-6-phenyl-2(R)-[(4-(2-morpholinoethoxy)phenyl)methyl]hexanamide,
N-(cis-2(R)-hydroxy-1(S)-indanyl)-5(S)-[(2-furanylmethyloxy)carbonyl]amino-4(S)-hydroxy-6-phenyl-2(R)-[(4-(2-morpholinoethoxy)phenyl)methyl]hexanamide,
N-[2(R)-hydroxy-1(S)-indanyl]-5(S)-(N'-acetylneuraminoyl]amino-4(S)-hydroxy-6-phenyl-2(R)-(4-(2-morpholino)ethoxy)phenyl]methylhexanamide,
N-(2(R)-hydroxy-1(S)-indanyl)-5(S)-[2-(1,3-bis-(methoxyethoxy))propoxycarbonyl]amino-4(S)-hydrox y-6-phenyl-2(R)-[4-(2-(4-morpholino)ethoxy)phenyl]methylhexanamide,
N-(2(R)-hydroxy-1(S)-indanyl)-5(S)-[5(R)-hydroxymethyl-3(S)-methoxytetrahydrofuranyloxyamino-4 (S)-hydroxy-6-phenyl-2(R)-(4-(2-(4-morpholinyl)ethoxy)phenyl)methyl]hexanamide,
N-(cis-2(R)-hydroxy-1(S)-indanyl)-5(S)-[(3-methoxy-1-butyloxy)carbonyl]amino-4(S)-hydroxy-6-phen yl-2(R)-[(4-(2-morpholinoethoxy)phenyl)methyl]hexanamide,
N-(cis-2(R)-hydroxy-1(S)-indanyl)-5(S)-[(1-methoxy-2-propyloxy)carbonyl]amino-4(S)-hydroxy-6-phe nyl-2(R)-[(4-(2-morpholinoethoxy)phenyl)methyl]hexanamide,
N-(2(R)-hydroxy-1(S)-indanyl]-5(S)-[2-(1,3-bis-(methoxy)propoxycarbonyl]amino-4(S)-hydroxy-6-phe nyl-2(R)-[4-(2-(4-morpholino)ethoxy)phenyl]methylhexanamide,
N-(cis-2(R)-hydroxy-1(S)-indanyl)-5(S)-[(2-(1,4-benzodioxanyl)methyl)oxycarbonyl]amino-4(S)-hydr oxy-6-phenyl-2(R)-[(4-(2-morpholinoethoxy)phenyl)methyl]hexanamide,
N-(2,2-dioxo-4(S)-isothiochromanyl)-5(S)-(1,1-dimethylethoxycarbonylamino)-4(S)-hydroxy-6-phenyl-2(R)-((4-(2-morpholinoethoxy)phenyl)methyl]hexanamide,
N-(2(R,S)-oxo-4(S)-isothiochromanyl)-5(S)-(1,1-dimethylethoxycarbonylamino)-4(S)-hydroxy-6-phenyl -2(R)-((4-(2-morpholinoethoxy)phenyl)methyl]hexanamide,
N-(cis-2(R)-hydroxy-1(S)-indanyl)-5(S)-[(2,3-bis-methoxyethyloxy)propyloxycarbonyl]amino-4(S)-hyd roxy-6-phenyl-2(R)-[4-(2-morpholinoethoxy)phenyl)methyl]hexanamide,
N-(cis-2(R)-hydroxy-1(S)-indanyl-5(S)-[4H-tetrahydro-4-pyranyloxymethylcarbonyl]amino-4(S)-hydro xy-6-phenyl-2(R)-[4-(2-morpholinoethoxy)phenyl)methyl]hexanamide,
N-(2(R)-hydroxy-1(S)-indanyl)-5(S)-(1,1-dimethylethoxycarbonylamino)-4(S)-hydroxy-6-cyclohexyl-2 (R)-[4-(2-(4-morpholino)ethoxy)phenylmethyl]hexanamide,
N-(cis-2(R)-hydroxy-1(S)-indanyl)-5(S)-[1,1-dimethylethoxycarbonyl-L-asparaginyl]amino-4(S)-hydro xy-6-phenyl-2(R)-[(4-(2-morpholinoethoxy)phenyl)methyl]hexanamide,
N-(4(S)-isothiochromanyl)-5(S)-(1,1-dimethylethoxycarbonylamino)-4(S)-hydroxy-6-phenyl-2(R)-((4-( 2-morpholinoethoxy)phenyl)methyl)hexanamide,
N-(cis-2(R)-hydroxy-1(S)-indanyl)-5(S)-[[N-2-quinolylcarbonyl-L-asparaginyl]amino-4(S)-hydroxy-6-p henyl-2(R)-[(4-(2-morpholinoethoxy)phenyl)methyl]hexanamide,
N-(2(R)-hydroxy-1(S)-indanyl)-5(S)-(2-furanylmethoxycarbonylamino)-4(S)-hydroxy-2(R)-(4-(2-(4-mo rpholino)ethoxy)phenyl)methyl-6-cyclohexylhexanamide,
N-(2(R)-hydroxy-1(S)-indanyl)-5(S)-((3(R,S)-tetrahydrofuranyl)acetamido)-4(S)-hydroxy-6-phenyl-2( R)-(4-(2-(4-morpholinyl)ethoxy)phenyl)methylhexanamide,
N-(2(R)-hydroxy-1(S)-indanyl)-5(S)-((2(R,S)-tetrahydrofuranyl)acetamido)-4(S)-hydroxy-6-phenyl-2( R)-(4-(2-(4-morpholinyl)ethoxy)phenyl)methylhexanamide,
N-(2(R)-hydroxy-1(S)-indanyl)-5(S)-((4,5-dihydro-3(2H)-furylidene)acetamido)-4(S)-hydroxy-6-pheny l-2(R)-(4-(2-(4-morpholinyl)ethoxy)phenyl)methylhexanamide,
N-(2(R)-hydroxy-1(S)-indanyl)-5(S)-(1,1-dimethylethoxycarbonylamino)-4(S)-hydroxy-6-cyclohexyl-2 (R)-(4-(2-(4-methylpiperazinyl)ethoxy)phenyl)methylhexanamide,
N-(2(R)-hydroxy-1(S)-indanyl)-5(S)-[(2-furanylmethyloxycarbonyl]amino-4(S)-hydroxy-6-phenyl-2(R)-[3-(4-(2-morpholinoethoxy)phenylprop-2-en-1-yl)hexanamide,
N-(2(R)-hydroxy-1(S)-indanyl)-5(S)-[(2-methoxyethoxy)carbonyl]amino-4(S)-hydroxy-6-phenyl-2(R)-[3-(4-(2-morpholinoethoxy)phenylprop-2-en-1-yl)hexanamide,
N-(2(R)-hydroxy-1(S)-indanyl)-5(S)-(1,1-dimethylethoxycarbonyl)amino-4(S)-hydroxy-6-cyclohexyl-2 (R)-(3-(4-(2-(4-morpholinyl)ethoxy)phenyl)prop-2-en-1-yl)hexanamide,

N-(2(R)-hydroxy-1(S)-indanyl)-5(S)-(1,1-dimethylethoxycarbonyl)amino-4(S)-hydroxy-6-cyclohexyl-2(R)-(4-(2-(diethylamino)ethoxy)phenyl)methylhexanamide,
N-(2(R)-hydroxy-1(S)-indanyl)-5(S)-(1,1-dimethylethoxycarbonylamino)-4(S)-hydroxy-6-cyclohexyl-2(R)-(4-(2-(diisopropylamino)ethoxy)phenyl)methylhexanamide,
N-(2(R)-hydroxy-1(S)-indanyl)-5(S)-(1,1-dimethylethoxycarbonylamino)-4(S)-hydroxy-6-(4-(2-dimethylaminoethoxy)phenyl)-2(R)-(3-phenyl-2-propen-1-yl)hexanamide,
N-(2(R)-hydroxy-1(S)-indanyl)-5(S)-(3(R,S)-tetrahydropyranyloxycarbonylamino)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-(5(R)-methoxymethyl-3(S)-tetrahydrofuranyloxycarbonylamino)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-(5-(2-acetoxy-1,4 :3,6-dianhydro-D-glucityl)carbonylamino)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-(3(S)-methyl-3-tetrahydrofuranyloxycarbonylamino)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-(4(S)-hydroxy-5(S)-(hydroxymethyl)-3(R)-tetrahydrofuranyloxycarbonylamino)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-(4(S)-benzyloxy-3(R)-tetrahydrofuranyloxycarbonylamino)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-(4(R)-benzyloxy-3(R)-tetrahydrofuranyloxycarbonylamino)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-(4(S)-methoxy-3(R)-tetrahydrofuranyloxycarbonylamino)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-(4(R)-methoxy-3(R)-tetrahydrofuranyloxycarbonylamino)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-(5(S)-methyl-3(S)-tetrahydrofuranyloxycarbonylamino)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-(5(R)-methyl-3(S)-tetrahydrofuranyloxycarbonylamino)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-(5(R)-methoxymethyl-3(S)-tetrahydrofuranyloxycarbonylamino)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-(5(S)-methoxymethyl-3(S)-tetrahydrofuranyloxycarbonylamino)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-(4(S)-methoxy-5(S)-methoxymethyl-3(R)-tetrahydrofuranyloxycarbonylamino)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-(3(S)-tetrahydrofuranyloxycarbonylamino)-4(S)-hydroxy-6-phenyl-2(R)-(trans-3-(4-(2-(4-morpholinyl)ethoxy)phenyl)prop-2-en-1-yl)hexanamide,
N-(2(R)-hydroxy-1(S)-indanyl)-5(S)-(3(S)-tetrahydropyranyloxycarbonylamino)-4(S)-hydroxy-6-phenyl-2(R)-(trans-3-(4-(2-(4-morpholinyl)ethoxy)phenyl)prop-2-en-1-yl)hexanamide,
N-(2(R)-hydroxy-1(S)-indanyl)-5(S)-(3(S)-tetrahydrofuranyloxycarbonylamino)-4(S)-hydroxy-6-cyclohexyl-2(R)-(trans-3-(4-(2-(4-morpholinyl)ethoxy)phenyl)-prop-2-en-1-yl)hexanamide,
N-(2(R)-hydroxy-1(S)-indanyl)-5(S)-(3(S)-tetrahydropyranyloxycarbonylamino)-4(S)-hydroxy-6-cyclohexyl-2(R)-(trans-3-(4-(2-(4-morpholinyl)ethoxy)phenyl)-prop-2-en-1-yl)hexanamide,
N-(2(R)-hydroxy-1(S)-indanyl)-5(S)-(3(S)-tetrahydrofuranyloxycarbonylamino)-4(S)-hydroxy-6-cyclohexyl-2(R)-(4-(2-(4-morpholinyl)ethoxy)phenyl)methylhexanamide,
N-(2(R)-hydroxy-1(S)-indanyl)-5(S)-(3(S)-tetrahydrofuranyloxycarbonylamino)-4(S)-hydroxy-6-cyclohexyl-2(R)-(4-(2-diethylaminoethoxy)phenyl)methyl)hexanamide,
N-(2(R)-hydroxy-1(S)-indanyl)-5(S)-(3(S)-tetrahydrofuranyloxycarbonylamino)-4(S)-hydroxy-6-cyclohexyl-2(R)-(4-(2-(N,N-di-2-propyl)aminoethoxy)phenyl)methylhexanamide,
N-(2(R)-hydroxy-1(S)-indanyl)-5(S)-(3(S)-tetrahydrofuranyloxycarbonylamino)-4(S)-hydroxy-6-cyclohexyl-2(R)-(4-(2-(4-methyl-1-piperazinyl)ethoxy)phenyl)methylhexanamide,
N-(2(R)-hydroxy-1(S)-indanyl)-5(S)-(3(S)-tetrahydropyranyloxycarbonylamino)-4(S)-hydroxy-6-cyclohexyl-2(R)-(4-(2-(4-methyl-1-piperazinyl)ethoxy)phenyl)methylhexanamide,
N-(2(R)-hydroxy-1(S)-indanyl)-5(S)-(3(S)-1,1-dioxotetrahydrothiofuranyloxycarbonylamino)-4(S)-hydroxy-6-cyclohexyl-2(R)-(4-(2-(4-morpholinyl)ethoxy)phenyl)methylhexanamide,
N-(2(R)-hydroxy-1(S)-indanyl)-5(S)-(3(S)-1,1-dioxotetrahydrothiofuranyloxycarbonylamino)-4(S)-hydroxy-6-phenyl-2(R)-(trans-3-(4-(2-(4-morpholinyl)ethoxy)phenyl)prop-2-en-1-yl)hexanamide.
N-(2(R)-hydroxy-1(S)-indanyl)-5(S)-(3(S)-1,1-dioxotetrahydrothiofuranyloxycarbonylamino)-4(S)-hydroxy-6-cyclohexyl-2(R)-(trans-3-(4-(2-(4-morpholinyl)ethoxy)phenyl)prop-2-en-1-yl)hexanamide,

N-(2(R)-hydroxy-1(S)-indanyl)-5(S)-(3(S)-1,1-dioxotetrahydrothiofuranyloxycarbonylamino)-4(S)-hydroxy-6-cyclohexyl-2(R)-(4-(2-(4-methyl-1-piperazinyl)ethoxy)phenyl)methylhexanamide,
N-(2(R)-hydroxy-1(S)-indanyl)-5(S)-(3(S)-1,1-dioxotetrahydrothiopyranyloxycarbonylamino)-4(S)-hydroxy-6-cyclohexyl-2(R)-(4-(2-(4-morpholinyl)ethoxy)phenyl)methylhexanamide,
N-(2(R)-hydroxy-1(S)-indanyl)-5(S)-(3(S)-1,1-dioxotetrahydrothiopyranyloxycarbonylamino)-4(S)-hydroxy-6-phenyl-2(R)-(trans-3-(4-(2-(4-morpholinyl)ethoxy)phenyl)prop-2-en-1-yl)hexanamide,
N-(2(R)-hydroxy-1(S)-indanyl)-5(S)-(3(S)-1,1-dioxotetrahydrothiopyranyloxycarbonylamino)-4(S)-hydroxy-6-cyclohexyl-2(R)-trans-3-(4-2-(4-morpholinyl)ethoxy)phenyl)prop-2-en-1-yl)hexanamide,
N-(2(R)-hydroxy-1(S)-indanyl)-5(S)-(3(S)-1,1-dioxotetrahydrothiopyranyloxycarbonylamino)-4(S)-hydroxy-6-cyclohexyl-2(R)-(4-(2-(4-methyl-1-piperazinyl)ethoxy)phenyl)methylhexanamide.
N-(2(R)-hydroxy-1(S)-indanyl)-5(S)-(4(S)-hydroxy-3(R)-tetrahydrofuranyloxycarbonylamino)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-(4(R)-hydroxy-3(R)-tetrahydrofuranyloxycarbonylamino)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-(4(S)-methyl-3(S)-tetrahydrofuranyloxycarbonylamino)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-(4(R)-methyl-3(S)-tetrahydrofuranyloxycarbonylamino)-4(S)-hydroxy-6-phenyl-2(R)-(4-(2-(4-morpholinyl)ethoxy)phenyl)methylhexanamide,
N-(2(R)-hydroxy-1(S)-indanyl)-5(S)-(2-methoxyethoxycarbonylamino)-4(S)-hydroxy-6-cyclohexyl-2(R)-(4-(2-(4-morpholinyl)ethoxy)phenyl)methylhexanamide,
N-(2(R)-hydroxy-1(S)-indanyl)-5(S)-(3(S)-tetrahydrofuranyloxycarbonylamino)-4(S)-hydroxy-6-phenyl-2(R)-(4-(2-(4-morpholinyl)ethoxy)phenyl)methylhexanamide,
or pharmaceutically acceptable salts thereof.

6. A compound, which is
N-(2(R)-hydroxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(N,N-bis(2-methoxyethyl)amino)ethoxy)phenyl)methyl)hexanamide,
N-(2(R)-hydroxy-1(S)-indanyl)-5(S)-(1-methylethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-ethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-cyclohexyl-2(R)-(4-(2-(4-morpholinyl)ethoxy)phenyl)methyl hexanamide,
N-(2(R)-hydroxy-1(S)-indanyl)-5(S)-(3(S)-tetrahydrofuranyloxycarbonylamino)-4(S)-hydroxy-6-cyclohexyl-2(R)-(4-(2-(4-morpholinyl)ethoxy)phenyl)methyl hexanamide,
N-(2(R)-hydroxy-1(S)-indanyl)-5(S)-(1,1-dimethylethoxycarbonylamino)-4(S)-hydroxy-6-cyclohexyl-2(R)-(4-(2-(4-methyl-1-piperazinyl)ethoxy)phenyl)methyl hexanamide,
N-(2(R)-hydroxy-1(S)-indanyl)-5(S)-(3(S)-tetrahydrofuranyloxycarbonylamino)-4(S)-hydroxy-6-cyclohexyl-2(R)-(4-(2-(4-methyl-1-piperazinyl)ethoxy)phenyl)methyl hexanamide,
N-(2(R)-hydroxy-1(S)-indanyl)-5(S)-(1,1-dimethylethoxycarbonylamino)-4(S)-hydroxy-6-cyclohexyl-2(R)-(4-(2-(N,N-di-2-propyl)aminoethoxy)phenyl)methyl hexanamide,
N-(2(R)-hydroxy-1(S)-indanyl)-5(S)-(3(S)-tetrahydrofuranyloxycarbonylamino)-4(S)-hydroxy-6-cyclohexyl-2(R)-(4-(2-(N,N-di-2-propyl)aminoethoxy)phenyl)methyl hexanamide,
N-(2(R)-hydroxy-1(S)-indanyl)-5(S)-(1,1-dimethylethoxycarbonylamino)-4(S)-hydroxy-6-cyclohexyl-2(R)-(trans-3-(4-(2-(4-morpholinyl)ethoxy)phenyl)prop-2-en-1-yl)hexanamide,
N-(2(R)-hydroxy-1(S)-indanyl)-5(S)-(3(S)-tetrahydrofuranyloxycarbonylamino)-4(S)-hydroxy-6-cyclohexyl-2(R)-(trans-3-(4-(2-(4-morpholinyl)ethoxy)phenyl)prop-2-en-1-yl) hexanamide,
N-(2(R)-hydroxy-1(S)-indanyl)-5(S)-(1,1-dimethylethoxycarbonylamino)-4(S)-hydroxy-6-phenyl-2(R)-(trans-3-(4-(2-(4-morpholinyl)ethoxy)phenyl)prop-2-en-1-yl) hexanamide,
N-(2(R)-hydroxy-1(S)-indanyl)-5(S)-(3(S)-tetrahydrofuranyloxycarbonylamino)-4(S)-hydroxy-6-phenyl-2(R)-(trans-3-(4-(2-(4-morpholinyl)ethoxy)phenyl)prop-2-en-1-yl) hexanamide, or
N-(2(R)-hydroxy-1(S)-indanyl)-5(S)-(2-methoxyethyloxycarbonylamino)-4(S)-hydroxy-6-phenyl-2(R)-(trans-3-(4-(2-(4-morpholinyl)ethoxy)phenyl)prop-2-en-1-yl) hexanamide,
or pharmaceutically acceptable salts thereof.

7. A compound which is

N-(2(R)-hydroxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide,
N-(2(R)-hydroxy-1(S)-indanyl)-5(S)-(1,1-dimethylethoxycarbonylamino)-4(S)-hydroxy-6-phenyl-2(R)-((4-(2-(N,N-bis(2-methoxyethyl)amino)-ethoxy)phenyl)methyl)hexanamide,
N-(2(R)-hydroxy-1(S)-indanyl)-5(S)-(1-methylethoxycarbonylamino)-4(S)-hydroxy-6-phenyl-2(R)-((4-(2-(4-morpholinyl)ethoxy)phenyl)methyl)hexanamide, or
N-(2(R)-hydroxy-1(S)-indanyl)-5(S)-(ethoxycarbonylamino)-4(S)-hydroxy-6-phenyl-2(R)-((4-(2-(4-mor pholinyl)ethoxy)phenyl)methyl)hexanamide,
or pharmaceutically acceptable salts thereof.

8. The compound of Claim 7, which is N-(2(R)-hydroxy-1(S)-indanyl)-5(S)-(1,1-dimethylethoxycarbonylamino)-4(S)-hydroxy-6-phenyl-2(R)-((4-(2-(4-morpholinyl)ethoxy)phenyl)methyl)hexanamide, or pharmaceutically acceptable salt thereof.

9. The compound of Claim 7, which is N-(2(R)-hydroxy-1(S)-indanyl)-5(S)-(1,1-dimethylethoxycarbonylamino)-4(S)-hydroxy-6-phenyl-2(R)-((4-(2-(N,N-bis(2-methoxyethyl)amino)ethoxy)phenyl)methyl)hexanamide, or pharmaceutically acceptable salt thereof.

10. The compound of Claim 7, which is N-(2(R)-hydroxy-1(S)-indanyl)-5(S)-(1-methylethoxycarbonylamino)-4(S)-hydroxy-6-phenyl-2(R)-((4-(2-(4-morpholinyl)ethoxy)phenyl)methyl)hexanamide, or pharmaceutically acceptable salt thereof.

11. The compound of claim 7, which is N-(2(R)-hydroxy-1(S)-indanyl)- 5(S)-(ethoxycarbonylamino)-4(S)-hydroxy-6-phenyl-2(R)-((4-(2-(4-morpholinyl)ethoxy)phenyl)methyl)hexanamide, or pharmaceutically acceptable salt thereof.

12. The compounds of Claims 1-11 in combination with any of the antivirals, immunomodulators, antibiotics or vaccines of the Table.

13. A pharmaceutical composition comprising the compounds of Claims 1-11 and a pharmaceutically acceptable carrier.

14. A pharmaceutical composition comprising the compound in combination according to Claim 12 and a pharmaceutically acceptable carrier.

15. The pharmaceutical composition of Claim 13, for use in the treatment of AIDS, in the prevention of infection by HIV, in the treatment of infection by HIV, or in the inhibition of HIV protease.

16. The pharmaceutical composition of Claim 14 for use in the treatment of AIDS, in the prevention of infection by HIV, in the treatment of infection by HIV, or in the inhibition of HIV protease.

17. The use of a compound as claimed in any one of Claims 1-11, for the preparation of a medicament useful for treating AIDS.

18. The use of a combination according to Claim 12, for the preparation of a medicament useful for treating AIDS.

19. The use of the compound of any one of Claims 1-11, for the preparation of a medicament useful for preventing infection by HIV.

20. The use of the combination according to Claim 12, for the preparation of a medicament useful for preventing infection by HIV.

21. The use of the compound, as claimed in any one of Claims 1-11, for the preparation of a medicament useful for treating infection by HIV.

22. The use of the combination according to Claim 12, for the preparation of a medicament useful for treating

infection by HIV.

23. The use of a compound as claimed in any one of Claims 1-11, for the preparation of a medicament useful for inhibiting HIV protease.

24. The use of the combination according to Claim 12, for the preparation of a medicament useful for inhibiting HIV protease.